# EUROPEAN PATENT APPLICATION

(11) **EP 4 116 306 A1**
(43) Date of publication of application: **11.01.2023**
(21) Application number: 21765050.6
(22) Date of filing: 04.03.2021
(51) Int. Cl.: C07D 513/04, C07F 5/00, C07K 16/18, A61K 51/00

(54) **COMPOUND AND RADIOACTIVE LABELING COMPOUND**

(30) Priority: 04.03.2020 JP 2020037150
(71) Applicant: Nihon Medi-Physics Co., Ltd, Koto-ku Tokyo, 136-0075 (JP); Kyoto University, Kyoto-shi, Kyoto 606-8501 (JP)
(72) Inventor: MAYA, Yoshifumi, Tokyo 136-0075 (JP); ONO, Masahiro, Kyoto-shi, Kyoto 606-8501 (JP); IIKUNI, Shimpei, Kyoto-shi, Kyoto 606-8501 (JP)
(74) Representative: Westphal, Petra
(86) International application number: PCT/JP2021/008333
(87) International publication number: WO 2021/177390

(57) **Abstract**

A compound according to the present invention has a chemical structure which, when viewed macroscopically, is arranged such that a chelating portion is positioned in the center of the structure, an atomic group containing an albumin binding site is bonded to one side of the chelating portion, and an atomic group containing a target molecule binding site is bonded to the other side of the chelating portion. The chelating portion is preferably DOTA or a derivative thereof. The target molecule binding site preferably has a structure which binds to a target molecule expressed in cancer tissue. In addition, the present invention provides a radioactive labeling compound in which the aforementioned compound is coordinated with a radioactive metal ion.

SEQUENCE LISTING

<110>  Nihon Medi-Physics Co.,Ltd.
       Kyoto University

<120>  Compound and Radiolabeled compound

<130>  P1912

<160>  13

<170>  PatentIn version 3.5

<210>  1
<211>  10
<212>  PRT
<213>  Unknown

<220>
<223>  capable of binding to serum albumin

<220>

<221> DOMAIN

<222> (1)..(10)

<223> capable of binding to serum albumin

<400> 1

Leu Cys Leu Arg Asp Trp Gly Cys Leu Trp

1               5                   10

<210> 2

<211> 12

<212> PRT

<213> Unknown

<220>

<223> capable of binding to serum albumin

<220>

<221> DOMAIN

<222> (1)..(12)

<223> capable of binding to serum albumin

<400> 2

Asp Ile Cys Leu Pro Arg Trp Gly Cys Leu Trp Trp
1               5                   10

<210>  3
<211>  15
<212>  PRT
<213>  Unknown

<220>
<223>  capable of binding to serum albumin

<220>
<221>  DOMAIN
<222>  (1)..(15)
<223>  capable of binding to serum albumin

<400>  3

Met Glu Asp Ile Cys Leu Pro Arg Trp Gly Cys Leu Trp Gly Asp
1               5                   10                  15

<210>  4

<211>  20

<212>  PRT

<213>  Unknown

<220>

<223>  capable of binding to serum albumin

<220>

<221>  DOMAIN

<222>  (1)..(20)

<223>  capable of binding to serum albumin

<400>  4

Gln Arg Leu Met Glu Asp Ile Cys Leu Pro Arg Trp Gly Cys Leu Trp

1               5                   10                  15

Glu Asp Asp Glu

            20

<210>  5

<211>  20

<212>  PRT

<213>  Unknown

<220>

<223>  capable of binding to serum albumin

<220>

<221>  DOMAIN

<222>  (1)..(20)

<223>  capable of binding to serum albumin

<400>  5

Gln Gly Leu Ile Gly Asp Ile Cys Leu Pro Arg Trp Gly Cys Leu Trp

1               5                   10                  15

Gly Arg Ser Val

            20

<210>  6

<211>  21

<212>  PRT

<213>  Unknown

<220>

<223> capable of binding to serum albumin

<220>

<221> DOMAIN

<222> (1)..(21)

<223> capable of binding to serum albumin

<400> 6

Gln Gly Leu Ile Gly Asp Ile Cys Leu Pro Arg Trp Gly Cys Leu Trp

1               5                   10                  15

Gly Arg Ser Val Lys

            20

<210> 7

<211> 15

<212> PRT

<213> Unknown

<220>

<223> capable of binding to serum albumin

<220>

<221>  DOMAIN

<222>  (1)..(15)

<223>  capable of binding to serum albumin

<400>  7

Glu Asp Ile Cys Leu Pro Arg Trp Gly Cys Leu Trp Glu Asp Asp

1               5                   10                  15

<210>  8

<211>  18

<212>  PRT

<213>  Unknown

<220>

<223>  capable of binding to serum albumin

<220>

<221>  DOMAIN

<222>  (1)..(18)

<223>  capable of binding to serum albumin

<400>  8

Arg Leu Met Glu Asp Ile Cys Leu Pro Arg Trp Gly Cys Leu Trp Glu
1               5                   10                  15

Asp Asp

<210>  9
<211>  16
<212>  PRT
<213>  Unknown

<220>
<223>  capable of binding to serum albumin

<220>
<221>  DOMAIN
<222>  (1)..(16)
<223>  capable of binding to serum albumin

<400>  9

Met Glu Asp Ile Cys Leu Pro Arg Trp Gly Cys Leu Trp Glu Asp Asp
1               5                   10                  15

<210>  10
<211>  15
<212>  PRT
<213>  Unknown

<220>
<223>  capable of binding to serum albumin

<220>
<221>  DOMAIN
<222>  (1)..(15)
<223>  capable of binding to serum albumin

<400>  10

Met Glu Asp Ile Cys Leu Pro Arg Trp Gly Cys Leu Trp Glu Asp
1               5                   10                  15

<210>  11

<211>  18

<212>  PRT

<213>  Unknown

<220>

<223>  capable of binding to serum albumin

<220>

<221>  DOMAIN

<222>  (1)..(18)

<223>  capable of binding to serum albumin

<400>  11

Arg Leu Met Glu Asp Ile Cys Leu Ala Arg Trp Gly Cys Leu Trp Glu

1               5                   10                  15

Asp Asp

<210>  12

<211>  20

<212>  PRT

<213>  Unknown

<220>

<223>  capable of binding to serum albumin

<220>

<221>  DOMAIN

<222>  (1)..(20)

<223>  capable of binding to serum albumin

<400>  12

Glu Val Arg Ser Phe Cys Thr Arg Trp Pro Ala Glu Lys Ser Cys Lys

1               5                   10                  15

Pro Leu Arg Gly

            20

<210>  13

<211>  20

<212>  PRT

<213>  Unknown

<220>

<223>  capable of binding to serum albumin

<220>

<221>  DOMAIN

<222>  (1)..(20)

<223>  capable of binding to serum albumin

<400>  13

Arg Ala Pro Glu Ser Phe Val Cys Tyr Trp Glu Thr Ile Cys Phe Glu

1               5                   10                  15

Arg Ser Glu Gln

            20

## Description

### Technical Field

The present invention relates to a compound and a radioactive labeled compound.

### Background Art

A radioactive labeled compound including a radioactive nuclide in the structure thereof is used as a reagent for detecting a target molecule, a diagnostic agent, or a medicine for treating a disease. For the purpose of further improving lesion detection performance and a lesion therapeutic effect, studies on improvement of specific accumulation in a target tissue and site and reduction of accumulation in a non-target tissue and site are in progress.

Patent Literature 1 describes a derivative (HTK01169) to which an iodophenylbutyryl group as an albumin binding site is added so as to be branched from the structure of PSMA-617. Patent Literature 1 also describes that this derivative binds to a prostate-specific membrane antigen (PSMA) as a target and can be used for the purpose of detecting and treating prostate cancer.

Patent Literature 2 describes a derivative having a structure derived from Evans blue as an albumin binding site. Patent Literature 2 describes that this derivative has a chelating moiety for chelating a radioactive metal, a peptide for binding to a target molecule, and the like in the structure thereof, and can be used as a radiation therapeutic agent and an imaging agent.

Patent Literature 3 describes a PSMA inhibitor capable of binding to albumin. Patent Literature 3 also describes that this inhibitor also binds to PSMA as a target and can be used for the purpose of detecting and treating prostate cancer like Patent Literature 1.

### Citation List

### Patent Literature

Patent Literature 1: WO 2019/075583 A
Patent Literature 2: WO 2017/196806 A
Patent Literature 3: WO 2018/098390 A

### Summary of Invention

In order to achieve improvement of specific accumulation in a target tissue and site and reduction of accumulation in a non-target tissue and site, control of in vivo kinetics such as a retention property in blood is important, and optimization of a chemical structure is desired as one of methods for effectively controlling in vivo kinetics. In general, a compound having a small molecular weight is poor in retention property in blood, and as a result, may cause insufficient accumulation in a target tissue or unintended accumulation in a normal tissue. In addition, the compounds described in Patent Literatures 1 to 3 often cause non-specific accumulation in the kidney, and there is room for improvement in this respect.

Therefore, the present invention relates to a compound and a radioactive labeled compound, capable of achieving both improvement of accumulation in a target tissue and reduction of accumulation in a non-target tissue, particularly reduction of accumulation in the kidney.

The present invention provides a compound represented by the following formula (1).

C-A-B (1)

In the formula (1), A represents a chelating moiety capable of being coordinated to a radioactive metal, B represents an atomic group containing an albumin binding moiety, and C represents an atomic group containing a target molecule binding moiety,
B binds to a site of A, and
C binds to A at a site different from the site where B binds to A.

The present invention also provides a radioactive labeled compound in which an ion of a radioactive metal is coordinated to the compound.

### Brief Description of Drawings

Fig. 1 illustrates graphs illustrating results of an in vivo radioactivity distribution assay in Examples and Comparative Examples.
Fig. 2 illustrates SPECT/CT images in Evaluation 5.
Fig. 3 illustrates SPECT/CT images in Evaluation 6.
Fig. 4 illustrates graphs illustrating results of a cell binding assay in Example 5-1 ([¹¹¹In]In-PSMA-DA1) and Comparative Example 2-1 ([¹¹¹In]In-PSMA-DB).
Fig. 5 illustrates graphs illustrating results of an albumin binding assay in [¹¹¹In]In-PSMA-DA1 and [¹¹¹In]In-PSMA-DB.
Fig. 6 illustrates graphs illustrating results of an in vivo radioactivity distribution assay in [¹¹¹In]In-PSMA-DA1 and [¹¹¹In]In-PSMA-DB.
Fig. 7 illustrates SPECT/CT images in [¹¹¹In]In-PSMA-DA1 and [¹¹¹In]In-PSMA-DB.
Fig. 8 illustrates graphs illustrating changes in tumor volume and mouse body weight in Example 5-2 ([⁹⁰Y]Y-PSMA-DA1) and Comparative Example 2-2 ([⁹⁰Y]Y-PSMA-DB).
Fig. 9 illustrates graphs illustrating changes in tumor volume and mouse body weight in Example 5-3 ([²²⁵Ac]Ac-PSMA-DA1) and Comparative Example 2-3 ([²²⁵Ac]Ac-PSMA-DB).
Fig. 10 illustrates graphs illustrating results of a cell binding assay in E4DA1 and E4D.
Fig. 11 is a graph illustrating results of an in vivo radioactivity distribution assay in E4DA1 and E4D.
Fig. 12 illustrates SPECT/CT images in Example 6 ([¹¹¹In]In-E4DA1) and Comparative Example 3 ([¹¹¹In]IIn-E4D).
Fig. 13 is an HPLC chart illustrating results of stability in plasma in Example 7 ([¹¹¹In]In-PtDA).
Fig. 14 is a graph illustrating results of a cell binding assay in [¹¹¹In]In-PtDA.
Fig. 15 is a graph illustrating results of an albumin binding assay in [¹¹¹In]In-PtDA.
Fig. 16 illustrates SPECT/CT images in [¹¹¹In]In-PtDA.

### Description of Embodiments

Hereinafter, a compound of the present invention and a radioactive labeled compound using the compound will be described based on preferred embodiments thereof. In the following description, "T to U [V]" (T and U represent arbitrary numbers, and [V] represents a unit) means "T [V] or more and U [V] or less" unless otherwise specified. In addition, when one or more asymmetric carbon atoms are present in a structure, they may each independently have an S configuration or an R configuration, unless stated otherwise.

The compound of the present invention includes, in the structure thereof, a chelating moiety capable of being coordinated to an ion of a radioactive metal, an atomic group containing an albumin binding moiety having a chemical structure capable of binding to albumin, and an atomic group containing a target molecule binding moiety having a chemical structure capable of binding to a target molecule. By having such a chemical structure, a radioactive labeled compound in which a radioactive metal ion is coordinated to the compound of the present invention achieves both improvement of accumulation in a target tissue and reduction of accumulation in a non-target tissue, particularly reduction of accumulation in the kidney. The compound of the present invention is a precursor compound used for labeling with a radioisotope such as a radioactive metal, that is, preferably a compound used as a labeling precursor. The radioactive metal will be described later.

The compound of the present invention is preferably represented by the following formula (1).

That is, in the compound, in a macroscopic view of a chemical structure thereof, preferably, a chelating moiety (represented by symbol A in formula (1)) is located at the center of the structure, and an atomic group (represented by symbol B in formula (1)) containing an albumin binding moiety and an atomic group (represented by symbol C in formula (1)) containing a target molecule binding moiety are disposed via the chelating moiety.

More preferably, the atomic group containing an albumin binding moiety binds to one side of the chelating moiety, and the atomic group containing a target molecule binding moiety binds to the other side of the chelating moiety. Still more preferably, the atomic group containing an albumin binding moiety, the chelating moiety, and the atomic group containing a target molecule binding moiety are substantially linearly disposed. "One side" and "the other side" of the chelating moiety refer to one side and the other side of a molecular structure when the molecular structure is virtually divided by a molecular symmetry plane in the chelating moiety. When the atomic group containing an albumin binding moiety and the target molecule binding moiety are present at plane-symmetric positions, the atomic group containing an albumin binding moiety, the chelating moiety, and the atomic group containing a target molecule binding moiety are "substantially linearly disposed".

C-A-B (1)

In formula (1), A represents a chelating moiety capable of being coordinated to an ion of a radioactive metal, B represents an atomic group containing an albumin binding moiety, and C represents an atomic group containing a target molecule binding moiety.

In formula (1), B preferably binds to a site of A.

In formula (1), C preferably binds to A at a site different from the site where B binds to A.

In the above formula (1), preferably, A has a cyclic structure, the cyclic structure has two or more nitrogen atoms, and the nitrogen atoms are connected to each other across two or more adjacent carbon atoms, or A has a chain structure, the chain structure has two or more nitrogen atoms, and the nitrogen atoms are connected to each other across two or more adjacent carbon atoms from a viewpoint of achieving both improvement of accumulation in a target tissue and reduction of accumulation in a non-target tissue, particularly reduction of accumulation in the kidney at a high level when the compound of the present invention is formed into a radioactive labeled compound.

When A has a cyclic structure, the skeleton of the cyclic structure may be constituted only by a nitrogen atom and a carbon atom, or may be constituted by a nitrogen atom, a carbon atom, and an oxygen atom. Binding between carbon atoms in the cyclic structure may be a chain or may form a ring structure.

When A has a chain structure, binding between carbon atoms in the chain structure may be divided by a nitrogen atom. Binding between carbon atoms in the chain structure may be a chain or may form a ring structure.

When A has a cyclic structure or a chain structure, A preferably has a nitrogen binding atomic group directly binding to the nitrogen atom constituting the cyclic structure or the chain structure. Specific preferable examples of the nitrogen binding atomic group include an atomic group containing one or more of a carboxy group, a phosphate group, an amide group, a benzene ring, and a pyridine ring, and the atomic group is more preferably a chain.

Furthermore, in the case that A has a cyclic structure or a chain structure, when B binds to the above-described nitrogen binding atomic group under a condition that B binds to a site of A, and C binds to A at a site different from the site where B binds to A, C preferably binds to a site other than the nitrogen binding atomic group to which B binds.

Specifically, in the above formula (1), the chelating moiety capable of being coordinated to a radioactive metal, represented by symbol A preferably has a structure derived from a compound represented by any one of the following formulas (A1) to (A9), and more preferably has a structure derived from a compound represented by the following formula (A1). That is, the compound of the present invention is preferably a derivative of a compound represented by any one of the following formulas (A1) to (A9), and more preferably a derivative of a compound represented by the following formula (A1). These structures can be appropriately selected depending on the type of radioactive metal described later. The chelating moiety having any of the structures achieves both improvement of accumulation in a target tissue and reduction of accumulation in a non-target tissue, particularly reduction of accumulation in the kidney.

In the above formula (1), examples of the chelating moiety represented by symbol A include structures derived from the following compounds, but are not limited thereto, and other structures can be applied.
- 1,4,7,10-tetraazacyclododecane-1,4,7,10-tetraacetic acid (DOTA)
- 1,4,7,10-tetraazacyclododecane-1,4,7,10-tetrapropionic acid (DOTPA)
- 1,4,7,10-tetraazacyclododecane-1,4,7,10-tetramethylene phosphoric acid (DOTMP)
- Hydroxypropyltetraazacyclododecane triacetic acid (HP-DO3A)
- (1R,4R,7R,10R)-α,α',α",α‴-tetramethyl-1,4,7,10-tetraazacyclododecane-1,4,7,10-tetraacetic acid (DOTAMA)
- 1,4,7,10-tetrakis(carbamoylmethyl)-1,4,7,10-tetraazacyclododecane (DOTAA)
- 1,4,7,10-tetraazacyclododecane-1,4,7,10-tetrakis(acetamidomethylene) phosphonic acid (DOTA-A-AMP)
- tetraazacyclododecane dimethane phosphonic acid (DO2P)
- α-(2-carboxyethyl)-1,4,7,10-tetraazacyclododecane-1,4,7,10-tetratetraacetic acid (DOTAGA)
- 1,4,8,11-tetraazacyclotetradecane-1,4,8,11-tetraacetic acid (TETA)
- 1,4,8,11-tetraazacyclotetradecane-1,4,8,11-tetrapropionic acid (TETPA)
- N,N',N",N‴-tetra(1,2-dihydro-1-hydroxy-2-oxopyridine-6-carbonyl)-1,5,10,14-tetraazatetradodecane (1,2-HOPO)
- 1,4,7,10,13-pentaazacyclopentadodecane-N,N',N",N‴,N""-pentaacetic acid (PEPA)
- Ethylenediaminetetraacetic acid (EDTA)
- 6,6'-((ethane-1,2-diylbis((carboxymethyl)azanediyl)) bis(methylene)) dipicolinic acid (H₄octapa)
- 6,6'-({9-hydroxy-1,5-bis(methoxycarbonyl)-2,4-di(pyridin-2-yl)-3,7-diazabicyclo [3.3.1] nonane-3,7-diyl} bis(-methylene)) dipicolinic acid (H₂bispa2)
- 1,2-[{6-(carboxy)-pyridin-2-yl}-methylamino] ethane (H₂dedpa)
- 6-(1,4,10,13-tetraoxa-7,16-diazacyclooctadecane-N,N'-dimethy) picolinic acid (H₂macropa)
- N,N"-bis(6-carboxy-2-pyridylmethyl)-diethylenetriamine-N,N',N"-triacetic acid (Hsdecapa)
- N,N'-(methylenephosphonate)-N,N'-[6-(methoxycarbonyl)pyridin-2-yl]-methyl-1,2-diaminoethane (H₆phospa)
- 6,6'-(((((4-isothiocyanate phenethyl)azanediyl)bis(ethane-2,1-diyl)) bis((carboxymethyl)azanediyl)) bis(methylene)) dipicolinic acid (p-SCN-Bn-H₄neunpa)
- 6,6'-(((((4-nitrophenethyl)azanediyl)bis(ethane-2,1-diyl)) bis((carboxymethyl)azanediyl)) bis(methylene)) dipicolinic acid (p-NO₂-Bn-H₄neunpa)
- 6,6'-(((azanediylbis(ethane-2,1-diyl)) bis((carboxymethyl)azanediyl)bis(methylene)) dipicolinic acid (Hsneunpa)
- 2-[4,7-bis(carboxymethyl)-1,4,7-triazonan-1-yl] acetic acid (NOTA)

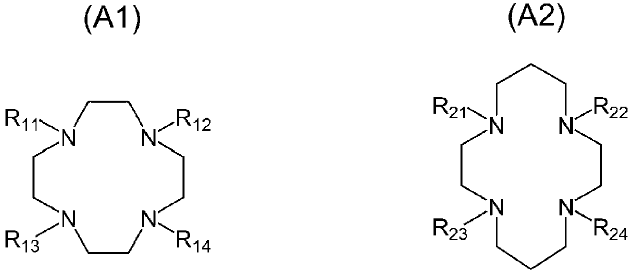

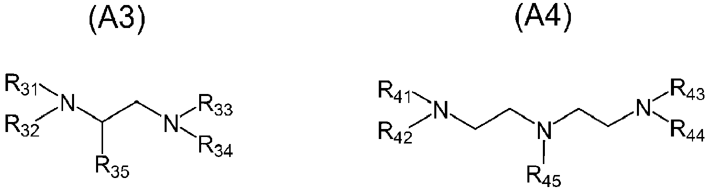

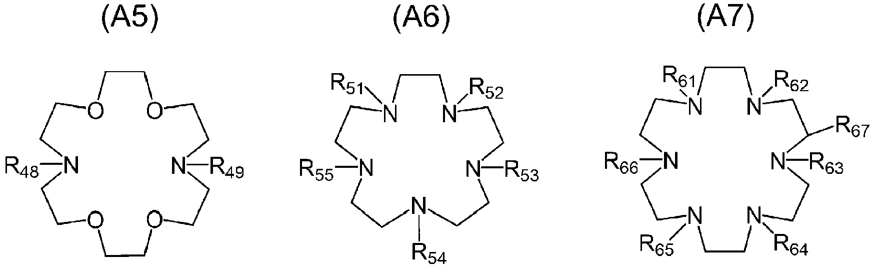

In formula (A1), R₁₁, R₁₂, R₁₃, and R₁₄ each independently represent any one of groups consisting of -(CH₂)ₚCOOH, -(CH₂)ₚC₅H₅N, -(CH₂)ₚPO₃H₂, -(CH₂)ₚCONH₂, - (CHCOOH)(CH₂)ₚCOOH, and p represents an integer of 0 or more and 3 or less.

In formula (A2), R₂₁, R₂₂, R₂₃, and R₂₄ each independently represent a carboxy group or a carboxyalkyl group having 2 or 3 carbon atoms.

In formula (A3), R₃₁, R₃₂, R₃₃, and R₃₄ each independently represent an atomic group having a hydrogen atom and 2 or more and 10 or less carbon atoms and optionally containing a nitrogen atom or an oxygen atom, and R₃₅ represents a hydrogen atom, a carboxy group, or a carboxyalkyl group having 2 or 3 carbon atoms.

In formula (A4), R₄₁, R₄₂, R₄₃ and R₄₄ each independently represent an atomic group having a hydrogen atom and 2 or more and 10 or less carbon atoms and optionally containing a nitrogen atom or an oxygen atom, and R₄₅ represents a hydrogen atom, a carboxy group, or a carboxyalkyl group having 2 or 3 carbon atoms.

In formula (A5), R₄₈ and R₄₉ each independently represent an atomic group having a hydrogen atom and 2 or more and 10 or less carbon atoms and optionally containing a nitrogen atom or an oxygen atom.

In formula (A6), R₅₁, R₅₂, R₅₃, R₅₄, and R₅₅ each independently represent an atomic group having a hydrogen atom and 2 or more and 10 or less carbon atoms and optionally containing a nitrogen atom or an oxygen atom.

In formula (A7), R₆₁, R₆₂, R₆₃, R₆₄, R₆₅, and R₆₆ each independently represent an atomic group having a hydrogen atom and 2 or more and 10 or less carbon atoms and optionally containing a nitrogen atom or an oxygen atom, and R₆₇ represents a hydrogen atom, a carboxy group, or a carboxyalkyl group having 2 or 3 carbon atoms.

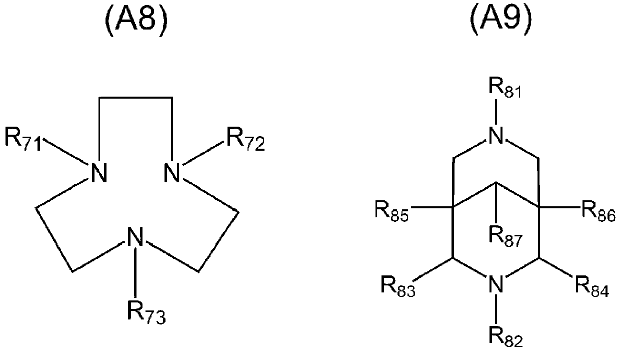

In formula (A8), R₇₁, R₇₂, and R₇₃ each independently represent an atomic group having a hydrogen atom and 2 or more and 10 or less carbon atoms and optionally containing a nitrogen atom or an oxygen atom.

In formula (A9), R₈₁ and R₈₂ each independently represent an alkyl group having 1 or more and 5 or less carbon atoms, a terminal of the alkyl group may be replaced with a pyridyl group having 1 or more carboxy groups as substituents, R₈₇ represents a hydroxy group or a carbonyl group, R₈₃ and R₈₄ each represent a pyridinyl group with or without a substituent, R₈₅ and R₈₆ each independently represent -COO-Rₐ, and Rₐ represents an alkyl group having 1 or more and 5 or less carbon atoms.

Examples of a specific structure represented by formula (A1) include structures represented by the following formulas (A1-1) to (A1-7).

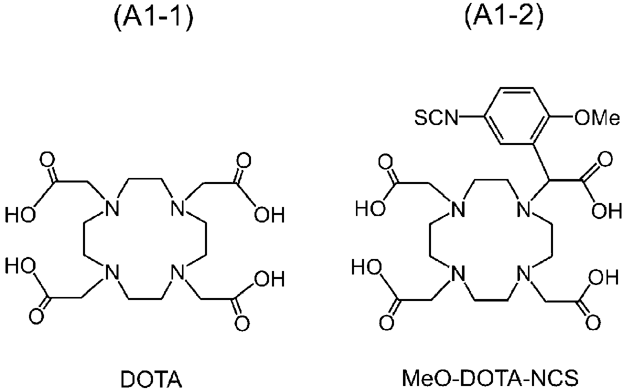

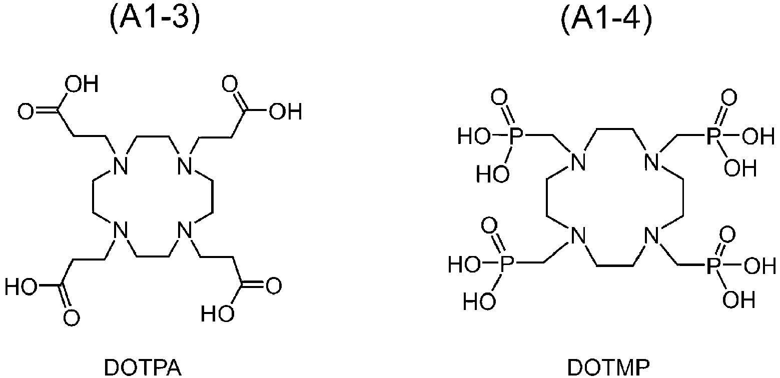

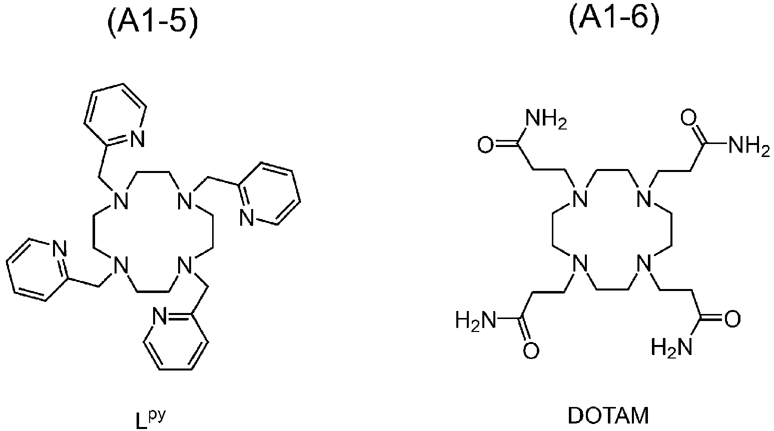

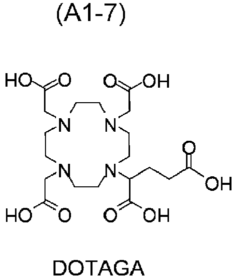

Examples of a specific structure represented by formula (A2) include structures represented by the following formulas (A2-1) and (A2-2).

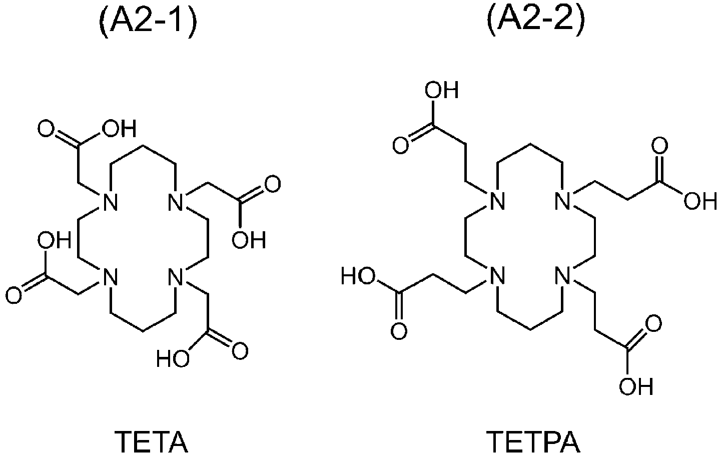

Examples of a specific structure represented by formula (A3) include structures represented by the following formulas (A3-1) to (A3-7).

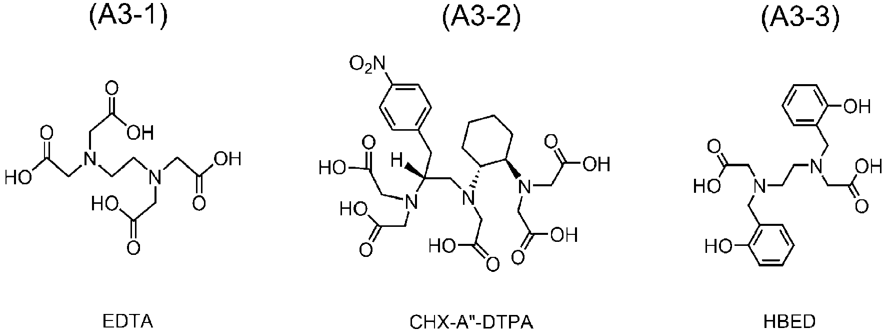

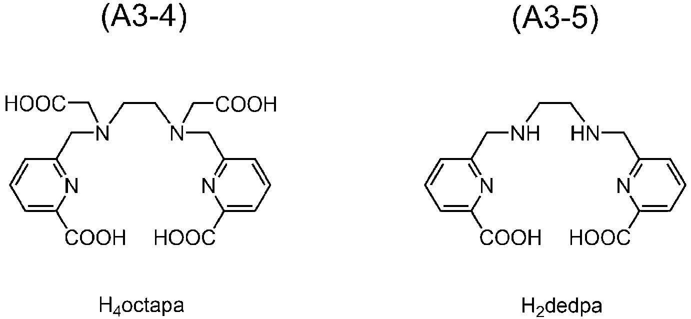

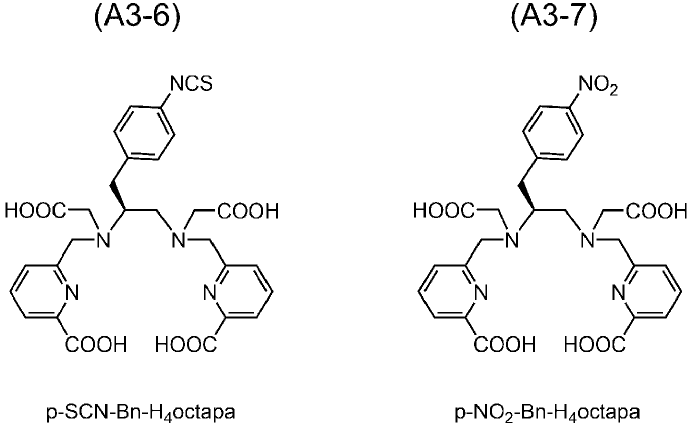

Examples of a specific structure represented by formula (A4) include structures represented by the following formulas (A4-1) and (A4-2).

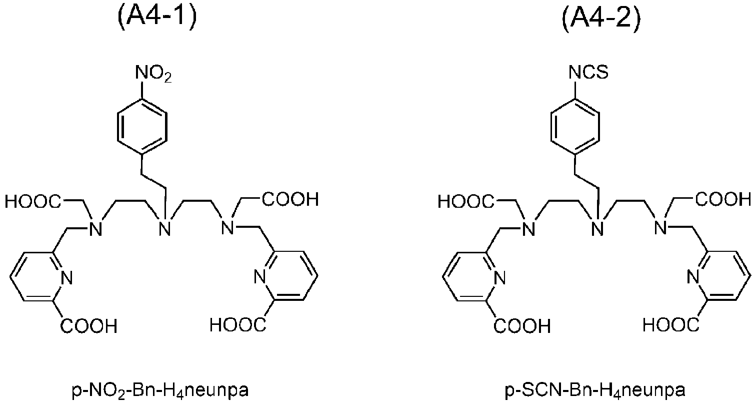

Examples of a specific structure represented by formula (A5) include structures represented by the following formulas (A5-1) to (A5-3).

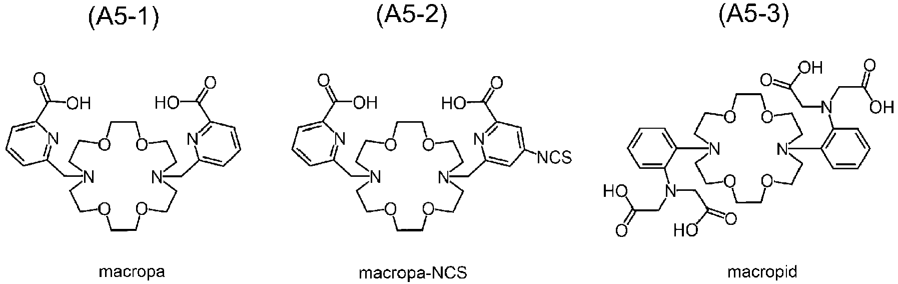

Examples of a specific structure represented by formula (A6) include a structure represented by the following formula (A6-1).

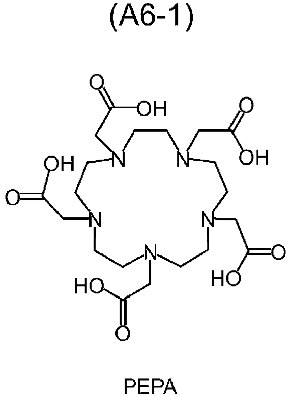

Examples of a specific structure represented by formula (A7) include structures represented by the following formulas (A7-1) and (A7-2).

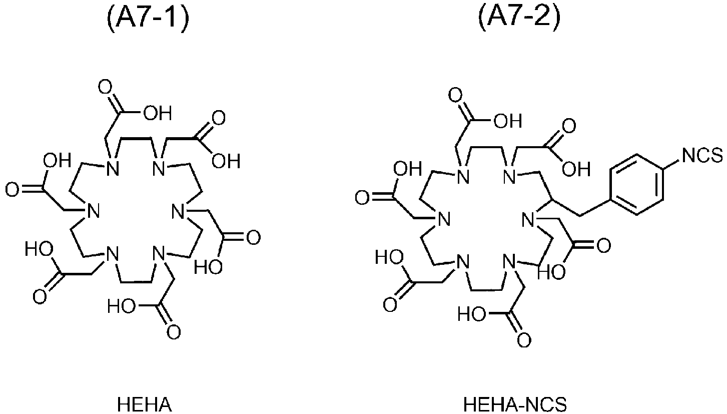

Examples of a specific structure represented by formula (A8) include structures represented by the following formulas (A8-1) to (A8-3).

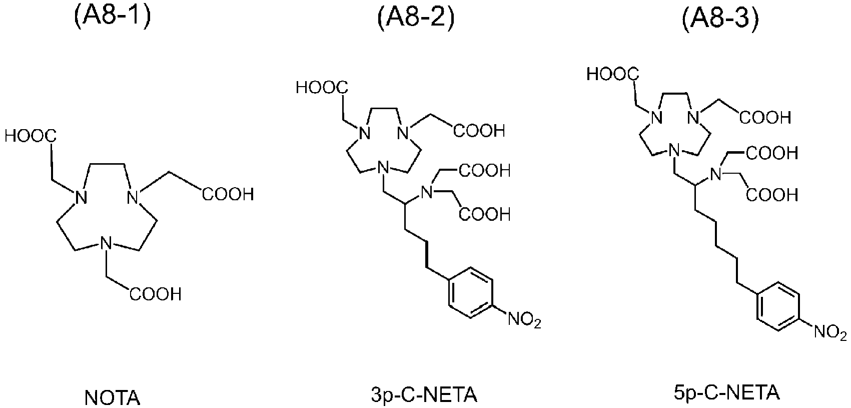

Examples of a specific structure represented by formula (A9) include structures represented by the following formulas (A9-1) to (A9-4).

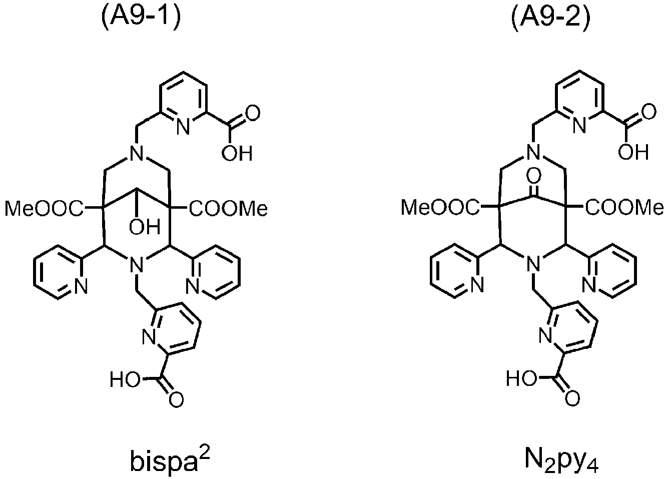

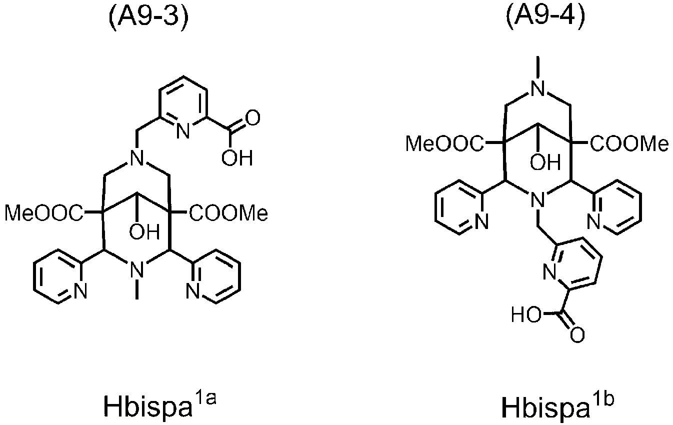

In formula (1), the site represented by symbol B is an atomic group having an affinity for albumin, preferably serum albumin, more preferably human serum albumin, and containing an albumin binding moiety having a chemical structure capable of reversibly binding to the albumin. By inclusion of such a structure in the compound of the present invention, when the compound is labeled with a radioactive metal and administered to a living body, accumulation in the kidney can be reduced while increasing a retention property in blood.

Specifically, while the compound having an albumin binding moiety in the molecule thereof is likely to bind to albumin in blood, the compound binding to albumin does not undergo glomerular filtration in the kidney. Therefore, migration and excretion into the kidney and urine are reduced, and a retention property in blood is improved. As a result, accumulation in the kidney which is a normal tissue is decreased, and transferability of the compound to a target tissue such as a tumor tissue can be further enhanced.

When a compound in which, in a macroscopic view of the chemical structure thereof, an atomic group containing an albumin binding moiety and an atomic group containing a target molecule binding moiety are disposed via a chelating moiety is used, a distance between the albumin binding moiety and the target molecule binding moiety can be appropriately ensured. Therefore, the compound can have both affinity for albumin and affinity for a target molecule. In particular, the albumin binding moiety is disposed at one structural terminal of the compound of the present invention, the target molecule binding moiety is disposed at the other structural terminal of the compound of the present invention, and a distance between the albumin binding moiety and the target molecule binding moiety can be thereby sufficiently secured, which is more advantageous in that both affinity for albumin and affinity for a target molecule can be achieved at a high level.

Examples of the structure of the albumin binding moiety in the above formula (1) include structures derived from one or more of γ-glutamic acid, a phenylbutyric acid with or without a substituent, lipid, hematin, bilirubin, clofibric acid, clofibrate, carotenoid, a compound having a steroid skeleton, a compound having an ibuprofen skeleton, a linear or branched and saturated or unsaturated hydrocarbon having 13 or more and 20 or less carbon atoms, a cyanine dye, a dye having a sulfonate group, a diazo dye, a pentamethine cyanine dye, blue dextran, bromocresol green, Evans blue, and derivatives thereof, and structures described in WO 2005/117984 A, WO 2010/127336 A, and WO 2010/172844 A. In addition to or in place of these, an antibody or a peptide capable of binding to albumin (for example, the peptide described in WO 2007/106120 A) can also be used as the albumin binding moiety.

Among these, it is preferable to use one or more of a phenylbutyric acid with or without a substituent, Evans blue, and derivatives thereof, or an antibody or a peptide capable of binding to albumin as the structure of the albumin binding moiety from a viewpoint of obtaining a compound applicable to a living body and reducing accumulation in an unintended normal organ such as the kidney.

Examples of the phenylbutyric acid with or without a substituent, applicable as the albumin binding moiety include a structure represented by the following formula (B1).

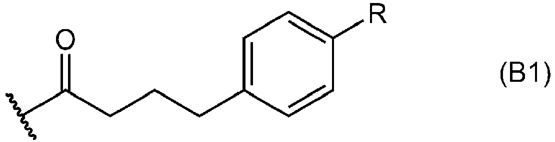

In formula (B 1), R represents a hydrogen atom, a halogen atom, or an alkyl group having 1 or more and 5 or less carbon atoms, and a portion indicated by a wavy line is a binding moiety to another structure.

In formula (B1), R is preferably a hydrogen atom, an iodine atom, a bromine atom, or a methyl group.

Examples of Evans blue and derivatives thereof applicable as the albumin binding moiety include a structure represented by the following formula (B2).

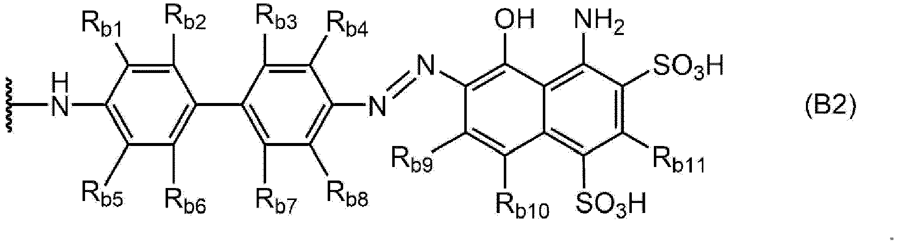

In formula (B2), R_{b1} to R_{b11} each independently represent a hydrogen atom, a halogen atom, a hydroxy group, a cyano group, an alkyl group having 1 or more and 6 or less carbon atoms with or without a substituent, or an alkoxy group having 1 or more and 6 or less carbon atoms with or without a substituent, and a portion indicated by a wavy line represents a binding moiety to another structure.

In formula (B2), preferably, both R_{b1} and R_{b4} are methyl groups, and R_{b2}, R_{b3}, and R_{b5} to R_{b11} are all hydrogen atoms.

As the antibody capable of binding to albumin, an immunoglobulin having a class of IgG, IgA, IgM, IgD, or IgE may be used, or an antibody fragment (for example, a Fab fragment) may be used as long as the antibody has affinity for albumin. When the antibody capable of binding to albumin is used as the albumin binding moiety, it is preferable to use a Fab fragment having a small molecular weight from a viewpoint of reducing accumulation in an unintended tissue such as the liver.

Examples of the peptide capable of binding to albumin include peptides containing the sequences described in WO 2007/106120 A, and specifically include peptides containing the following peptide sequences, but are not limited to these sequences.

In the following peptide sequences, an amino acid is represented by one letter, the left side of the paper indicates an N-terminal, and the right side of the paper indicates a C-terminal.
- LCLRDWGCLW (SEQ ID NO: 1)
- DICLPRWGCLWW (SEQ ID NO: 2)
- MEDICLPRWGCLWGD (SEQ ID NO: 3)
- QRLMEDICLPRWGCLWEDDE (SEQ ID NO: 4)
- QGLIGDICLPRWGCLWGRSV (SEQ ID NO: 5)
- QGLIGDICLPRWGCLWGRSVK (SEQ ID NO: 6)
- EDICLPRWGCLWEDD (SEQ ID NO: 7)
- RLMEDICLPRWGCLWEDD (SEQ ID NO: 8)
- MEDICLPRWGCLWEDD (SEQ ID NO: 9)
- MEDICLPRWGCLWED (SEQ ID NO: 10)
- RLMEDICLARWGCLWEDD (SEQ ID NO: 11)
- EVRSFCTRWPAEKSCKPLRG (SEQ ID NO: 12)
- RAPESFVCYWETICFERSEQ (SEQ ID NO: 13)

In formula (1), the site represented by symbol C is an atomic group having affinity for a target molecule expressed in a tissue causing a disease such as cancer and containing a target molecule binding moiety having a chemical structure capable of reversibly binding to the target molecule. By inclusion of such a structure in the compound of the present invention, when the compound is labeled with a radioactive metal and administered to a living body, the compound can be efficiently accumulated in a tissue to be treated or diagnosed, and efficiency of treatment or diagnosis can be increased.

Examples of the cancer include: a solid cancer such as brain tumor, a breast cancer, a prostate cancer, a pancreatic cancer, a stomach cancer, a lung cancer, a colon cancer, a rectum cancer, a large intestine cancer, a small intestine cancer, an esophagus cancer, a duodenum cancer, a tongue cancer, a pharynx cancer, a salivary gland cancer, glioma, a liver cancer, a kidney cancer, a bile duct cancer, a uterine body cancer, a cervical cancer, an ovarian cancer, a bladder cancer, a skin cancer, hemangioma, malignant melanoma, a thyroid cancer, a parathyroid cancer, a nasal cavity cancer, a sinus cancer, bone tumor, angiofibroma, retinal sarcoma, a penile cancer, a testicular cancer, or a pediatric solid cancer; a hematological cancer such as malignant lymphoma, leukemia, or myeloma, sarcoma, and multiple glioblastoma. These cancers may be primary or metastatic.

The chemical structure in the target molecule binding moiety can be appropriately selected according to a target tissue and the amount of a target molecule expressed in the tissue. Specifically, as the target molecule binding moiety, it is possible to adopt a structure targeting a molecule that is not much expressed in a normal tissue but is abundantly expressed in a tissue that causes a disease, such as a cancer tissue, as a target molecule, and having affinity for the molecule. Examples of the structure having affinity for the target molecule include one or more of a low molecular weight compound, a peptide, an antibody, and an antibody fragment such as a Fab fragment.

Examples of the target molecule include a protein, DNA, and RNA. The target molecule is preferably a protein present on a cell membrane surface or present penetrating the cell membrane.

Specific examples of the target molecule include: a tyrosine kinase such as HER2, HER3, EPHA2, or KIT; a protein expressed in hematopoietic lineage cells, such as CD19, CD19A, CD20, CD22, CD27L, CD30, CD33A, CD38, CD56, CD70, CD74, CD79b, CD138, or SLAMF7; a folate receptor such as FOLR1; a tumor-associated Ca signal transducer such as TROP2; a carcinoembryonic antigen-related cell adhesion molecule such as CEACAM5; an ectonucleotide pyrophosphatase/phosphodiesterase such as ENPP3; a metalloreductase such as STEAP1, an epidermal growth factor receptor such as EGFR or EGFRvIII; a cell adhesion molecule such as Nectin-4; a fibroblast growth factor such as FGFR2; a phosphate transporter such as NaPi2b; an endothelin receptor such as ETB1 or ETB2; a zinc transporter such as LIV1A; a carbonic anhydrase such as CA-IX; a glucagon-like peptide receptor such as a GLP-1 receptor; a transmembrane glycoprotein NMB (gpNMB); a prostate-specific membrane antigen (PSMA); mesothelin; guanylate cyclase C; and integrin.

When the compound of the present invention is used as a precursor of a compound used for treatment or diagnosis of cancer, examples of the structure of the target molecule binding moiety in the above formula (1) include a structure to bind to any one target molecule of CA-IX, PSMA, and a GLP-1 receptor.

CA-IX is a membrane-binding protein whose expression is enhanced when cells become hypoxic. For example, expression of CA-IX is high in a tissue having a hypoxic region in a solid cancer tissue, but is low in a normal tissue. Therefore, CA-IX is one of useful target molecules in the present invention, and is particularly useful as a target for treatment and diagnosis of a solid cancer.

PSMAis a membrane-binding protein whose expression is enhanced in a prostate cancer. An expression level of PSMA in a normal tissue including prostate is low, but the expression level of PSMA is enhanced as the degree of malignancy of the prostate cancer increases. Therefore, PSMA is one of useful target molecules in the present invention, and is particularly useful as a target for diagnosis and treatment of the prostate cancer.

The GLP-1 receptor is a receptor for a glucagon-like peptide known as a type of incretin hormone secreted in a living body. The GLP-1 receptor is known to be specifically expressed higher in a pancreatic cancer (in particular, an insulinoma) than in other normal tissues. Therefore, the GLP-1 receptor is also one of useful target molecules in the present invention, and is particularly useful as a target for diagnosis and treatment of the pancreatic cancer.

In the above formula (1), the target molecule binding moiety is preferably an antibody or a peptide having a structure represented by any one of the following formulas (C1) to (C3) or capable of binding to a target molecule. By inclusion of such a structure in the compound of the present invention, when the compound is labeled with a radioactive metal and administered, the compound can be efficiently accumulated in a tissue to be treated or diagnosed, and efficiency of treatment or diagnosis can be increased.

Specifically, each of the following formulas (C1) and (C2) is one form of the structure of a target molecule binding moiety preferably adopted when CA-IX is a target molecule.

In addition, the following formula (C3) is one form of the structure of a target molecule binding moiety preferably adopted when PSMA is a target molecule.

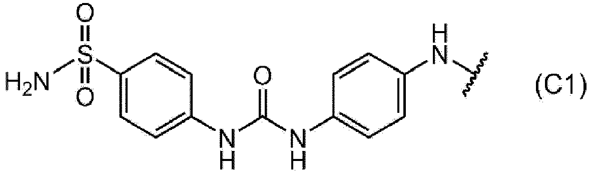

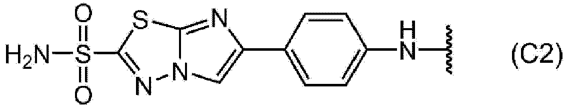

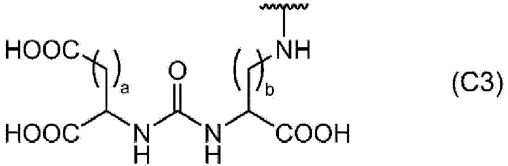

In formula (C3), a and b each independently represent an integer of 1 or more and 7 or less. In the formulas (C1) to (C3), each of portions indicated by wavy lines is a binding moiety to another structure.

As the antibody capable of binding to a target molecule, an immunoglobulin having a class of IgG, IgA, IgM, IgD, or IgE may be used, or an antibody fragment (for example, a Fab fragment) may be used as long as the antibody has affinity for the target molecule. When the antibody capable of binding to a target molecule is used as the target molecule binding moiety, it is preferable to use a Fab fragment having a small molecular weight from a viewpoint of reducing accumulation in an unintended tissue such as the liver.

Examples of the peptide capable of binding to a target molecule include, but are not limited to, a peptide having affinity for the GLP-1 receptor such as exendin-4, and a cyclic peptide having affinity for αvβ3 integrin and αvβ5 integrin, such as a cyclic RGD peptide (cRGD peptide).

The compound of the present invention more preferably has a structure represented by the following formula (2).

By having such a structure, the compound sufficiently has a hetero atom capable of being coordinated to a radioactive metal in the structure. Therefore, complex forming efficiency can be enhanced when the radioactive metal is coordinated to the compound of the present invention. In addition, since the degree of freedom of movement of the molecule in the albumin binding moiety and the target molecule binding moiety is increased, both the affinity for albumin and the affinity for a target molecule can be achieved at a high level. Furthermore, unintended accumulation in a normal tissue such as the liver or the kidney is reduced.

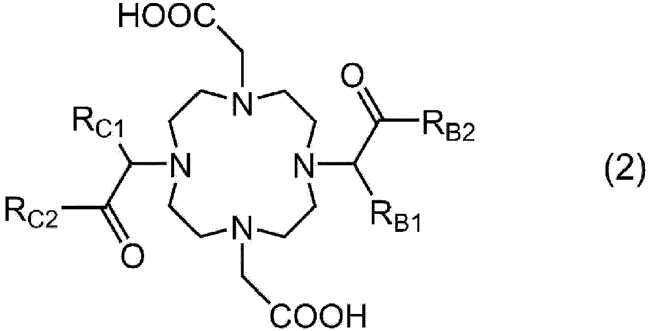

In formula (2), one of R_{B1} and R_{B2} represents an atomic group containing an albumin binding moiety, the other represents a hydrogen atom, a hydroxy group, or a carboxy group, one of Rci and R_{C2} represents an atomic group containing a target molecule binding moiety, and the other represents a hydrogen atom, a hydroxy group, or a carboxy group.

Among these, in formula (2), preferably, R_{B1} represents an atomic group containing an albumin binding moiety, Rci represents an atomic group containing a target molecule binding moiety, and R_{B2} and R_{C2} both represent a hydroxy group.

Alternatively, in formula (2), preferably, R_{B2} represents an atomic group containing an albumin binding moiety, R_{C2} represents an atomic group containing a target molecule binding moiety, and R_{B1} and R_{C1} both represent a hydrogen atom or each independently represent a carboxyalkyl group having 1 to 5 carbon atoms.

In any of the forms described above, the albumin binding moiety is disposed at one structural terminal of the compound, and the target molecule binding moiety is disposed at the other structural terminal of the compound, whereby the chelating moiety, the albumin binding moiety, and the target molecule binding moiety are preferably disposed substantially linearly in a macroscopic view of the chemical structure of the compound of the present invention.

In particular, in formula (2), the atomic group containing an albumin binding moiety is preferably an atomic group containing the structure represented by formula (B1) or formula (B2) described above as the albumin binding moiety. Specific examples of such a chemical structure are indicated by the following formulas (2-1) to (2-4).

In the following formulas (2-1) and (2-2), Li's each independently represent an alkyl group having 1 or more and 8 or less carbon atoms and having a carboxy group.

In the following formulas (2-3) and (2-4), g's each independently represent an integer of 1 or more and 5 or less, and h's each independently represent 0 or 1.

In addition, in the following formulas (2-1) to (2-4), the above-described descriptions regarding formulas (B1), (B2) and (2) are each independently applied to descriptions regarding R, R_{b1} to R_{b11}, R_{C1}, and R_{C2} appropriately.

That is, R represents a hydrogen atom, a halogen atom, or an alkyl group having 1 or more and 5 or less carbon atoms, and preferably represents a hydrogen atom, an iodine atom, a bromine atom, or a methyl group.

R_{b1} to R_{b11} each independently represent a hydrogen atom, a halogen atom, a hydroxy group, a cyano group, an alkyl group having 1 or more and 6 or less carbon atoms with or without a substituent, or an alkoxy group having 1 or more and 6 or less carbon atoms with or without a substituent. Preferably, R_{b1} and R_{b4} each represent a methyl group, and R_{b2}, R_{b3}, and R_{b5} to R_{b11} each represent a hydrogen atom.

One of Rci and R_{C2} represents an atomic group containing a target molecule binding moiety, and the other represents a hydrogen atom, a hydroxy group, or a carboxyalkyl group having 1 to 5 carbon atoms.

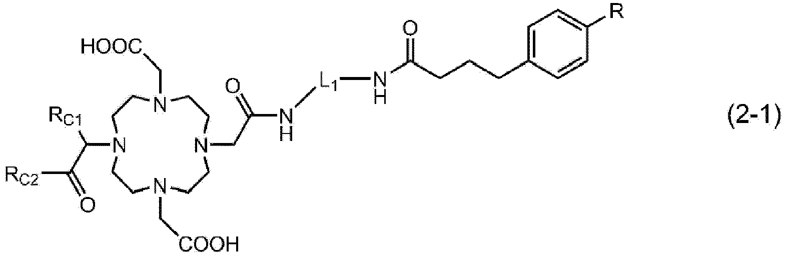

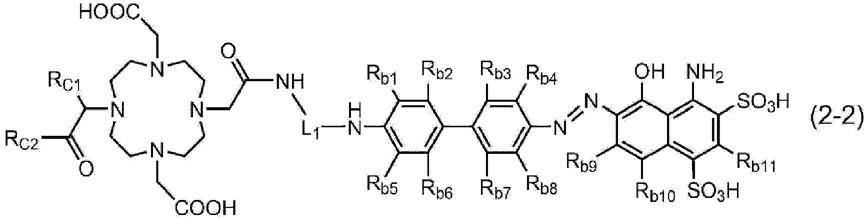

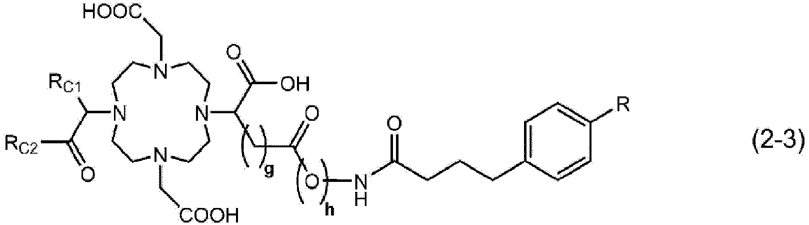

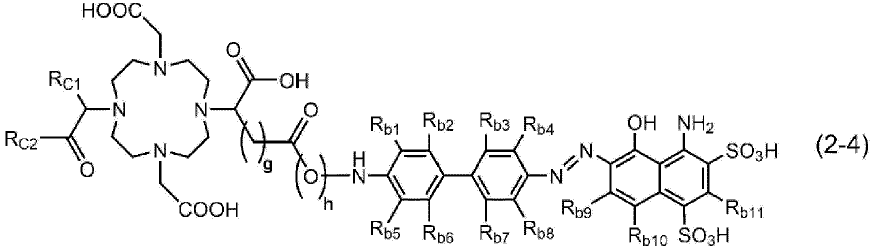

Specific examples of the chemical structure of a compound containing the structure of formula (B1) as the albumin binding moiety in formula (2) and targeting CA-IX as a target molecule are indicated by the following formulas (2a) to (2d).

In formulas (2a) to (2d), R's each independently represent a hydrogen atom, a halogen atom, or an alkyl group having 1 or more and 5 or less carbon atoms, and preferably each independently represent a hydrogen atom, an iodine atom, a bromine atom, or a methyl group.

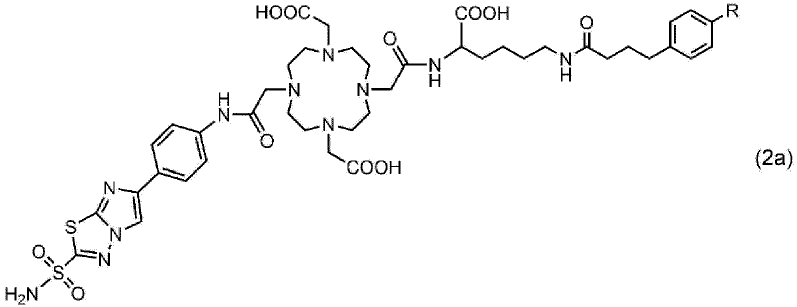

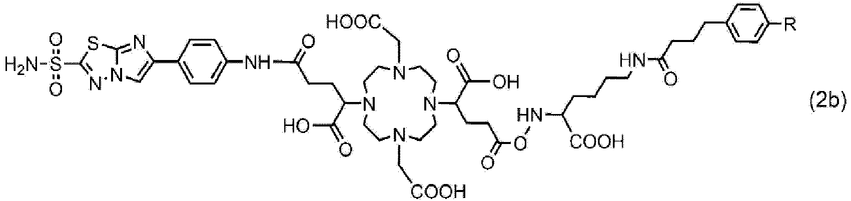

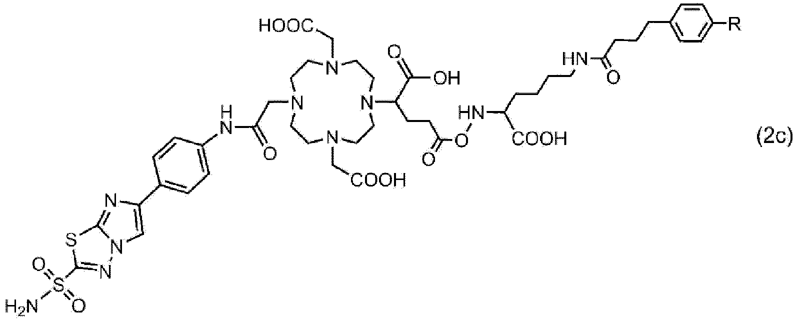

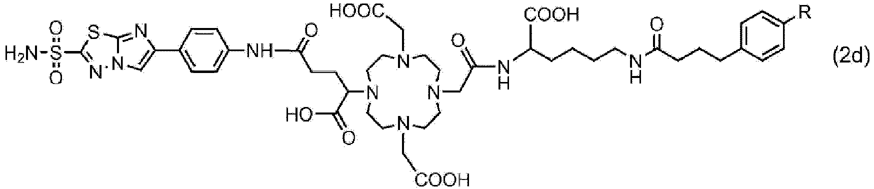

Specific examples of the chemical structure of a compound containing the structure of formula (B1) as the albumin binding moiety in formula (2) and targeting a GLP-1 receptor as a target molecule are indicated by the following formulas (3a) to (3d).

In formulas (3a) to (3d), R's each independently represent a hydrogen atom, a halogen atom, or an alkyl group having 1 or more and 5 or less carbon atoms, and preferably each independently represent a hydrogen atom, an iodine atom, a bromine atom, or a methyl group.

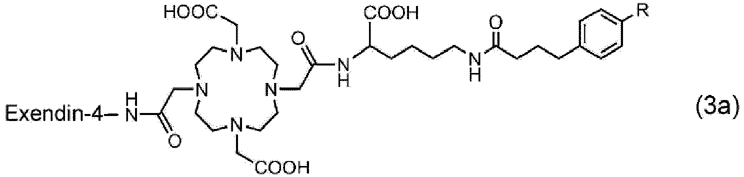

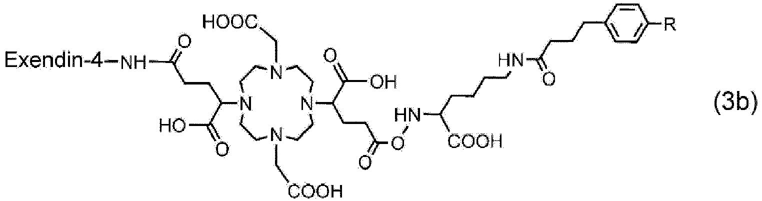

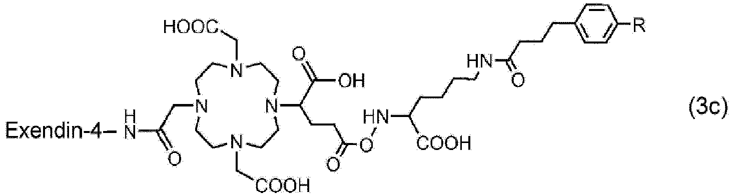

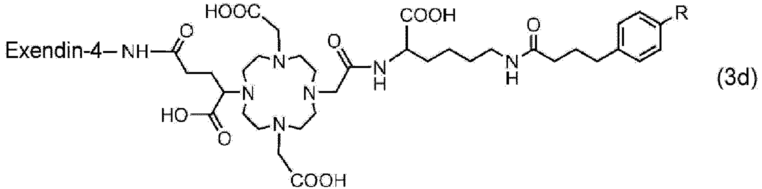

The compound of the present invention having each of the above structures can be manufactured, for example, by a method described in the following reaction path (I) or (II) or a method described in Examples described later.

In reaction paths (I) and (II), R represents a hydrogen atom, a halogen atom, or an alkyl group having 1 or more and 5 or less carbon atoms, and preferably represents a hydrogen atom, an iodine atom, a bromine atom, or a methyl group.

In reaction path (II), "ALB" represents an albumin binding moiety, and "Ligand" represents a target molecule binding moiety.

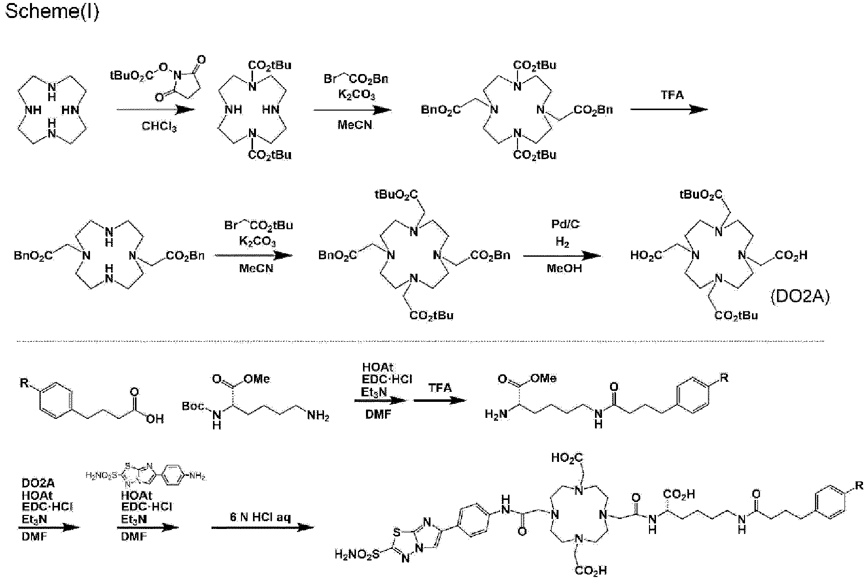

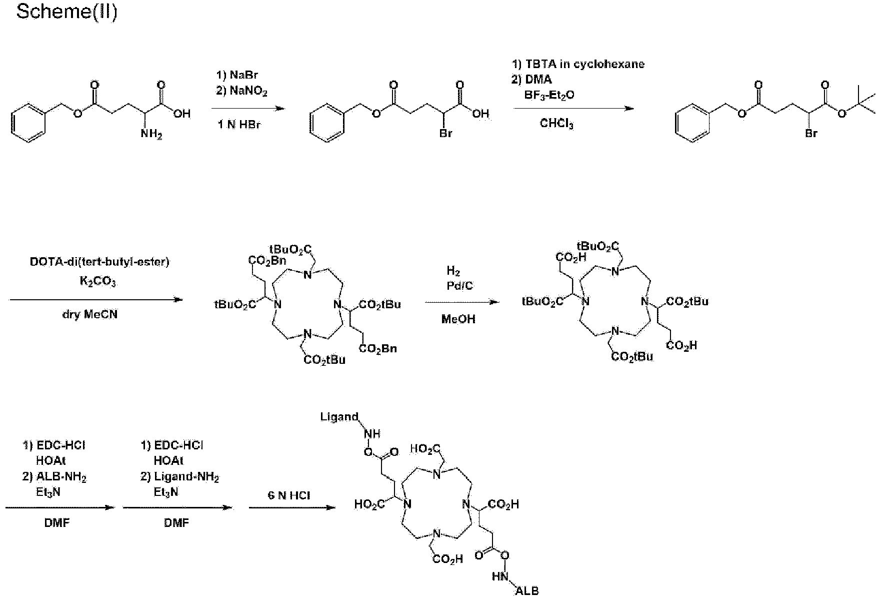

The compound of the present invention can be reacted with a radioactive metal preferably in a state of being dissolved in an aqueous liquid such as a solvent or a buffer to obtain a radioactive labeled compound which is a radioactive metal complex. In this radioactive labeled compound, the chelating moiety in the compound is coordinated to an ion of a radioactive metal.

The radioactive metal to be reacted with the compound is preferably used in a form of an ionizable radioactive metal compound, and more preferably used in a form of a radioactive metal ion (hereinafter, these forms are also collectively referred to as "radioactive metal source") from a viewpoint of enhancing the complex forming efficiency. As the radioactive metal source, for example, a radioactive metal ion-containing liquid in which a radioactive metal ion is dissolved or dispersed in a solvent mainly containing water can be used.

The compound and the radioactive metal are preferably heated and reacted with each other in complex formation from a viewpoint of enhancing the complex forming efficiency with the radioactive metal without depending on a combination of the chelating moiety and the radioactive metal in the compound. By performing the reaction under such reaction conditions, even when low-energy radiation that is difficult to detect or a radioactive metal nuclide that emits an α ray is used, complex formation can proceed well, and therefore a target radioactive labeled compound can be obtained at a high yield.

In obtaining the radioactive labeled compound, the order of adding the compound and the radioactive metal source is not limited as long as the compound and the radioactive metal ion can form a complex. For example, one of the compound and the radioactive metal source may be added to a reaction vessel containing a solvent, and then the other may be added thereto to cause a reaction, or one of the compound and the radioactive metal source may be dissolved in a solvent to obtain a solution, and then the other may be added to the solution to cause a reaction. Alternatively, the compound and the radioactive metal source may be simultaneously added to a reaction vessel containing a solvent to cause a reaction.

Reaction conditions for obtaining the radioactive labeled compound can be, for example, the following conditions. Examples of the solvent used in this step include water, saline, and a buffer such as a sodium acetate buffer, an ammonium acetate buffer, a phosphate buffer, phosphate buffer saline, a Tris buffer, a HEPES buffer, or a tetramethylammonium acetate buffer. Reaction temperature may be, for example, room temperature (25°C), or the reaction may be performed under heating conditions.

As the radioactive metal source, for example, a solution in which a radioactive metal ion is dispersed in a solvent mainly containing water can be used.

The amount of the reaction liquid in this step is not particularly limited, but is practically 0.01 mL to 100 mL at the time of start of this step from a viewpoint of practicality in the manufacturing process. In addition, the concentrations of the compound and the radioactive metal ion in the reaction liquid are preferably each independently 1 µmol/L to 100 µmol/L at the time of start of this step from a viewpoint of an yield of the target radioactive labeled compound.

The obtained radioactive labeled compound may be used as it is, or may be purified using a filtration filter, a membrane filter, a column packed with various fillers, chromatography, or the like.

If necessary, in the subsequent steps, a solvent mainly containing water and other pharmaceutically acceptable components may be added to the radioactive labeled compound to form a radioactive pharmaceutical composition. The radioactive pharmaceutical composition can be manufactured, for example, by dissolving a radioactive labeled compound manufactured by the above-described method in a solvent that mainly contains water and is substantially isotonic with a living body. The radioactive pharmaceutical composition is administered to a living body orally or parenterally such as intravenously, subcutaneously, intraperitoneally, or intramuscularly, and is used for treatment of a disease, diagnosis of a disease, detection of a lesion, or the like.

As the radioactive metal coordinated to the radioactive labeled compound in an ionic state, a metal nuclide that emits radiation of an α ray, a β ray, a γ ray, or a combination thereof can be used. Examples of such a radioactive metal nuclide include a radioactive isotope of an alkali metal, an alkaline earth metal, a lanthanoid, an actinoid, a transition metal, or a metal other than these metals.

Among these nuclides, ⁴⁴Sc, ⁵¹Cr, ⁵⁷Co, ⁵⁸Co, ⁶⁰Co, ⁵⁹Fe, ⁶⁷Ga, ⁶⁸Ga, ⁶⁴Cu, ⁶⁷Cu, ⁸⁹Sr, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, ²²⁷Th, or ²²⁵Ac is preferably used as the radioactive metal nuclide from a viewpoint of being commercially available and improving a complex forming property. These radioactive metals can be manufactured by a conventional method. These radioactive nuclides are preferably obtained as a solution containing a radioactive metal in an ionized state.

When the radioactive labeled compound is used for the purpose of treating a disease, an α ray-emitting nuclide or a β⁻ ray-emitting nuclide is preferably used as the radioactive metal from a viewpoint of enhancing a therapeutic effect. The α ray-emitting nuclide may be any nuclide that emits an α ray in a decay process of the radioactive metal. Specifically, ²¹²Bi, ²¹³Bi, ²²⁷Th, ²²⁵Ac, or the like is preferably used, ²²⁷Th or ²²⁵Ac is more preferably used, and ²²⁵Ac is still more preferably used.

The β⁻ ray-emitting nuclide may be any nuclide that emits a β ray in a decay process of the radioactive metal. Specifically,⁶⁰Co, ⁵⁹Fe, ⁶⁴Cu, ⁶⁷Cu, ⁸⁹Sr, ⁹⁰Y, ^{99m}Tc, ¹⁰³Ru, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰³Hg, ²¹²Bi, ²¹³Bi, ²¹²Pb, or the like is preferably used, ⁶⁴Cu, ⁶⁷Cu, ⁸⁹Sr, or ⁹⁰Y is more preferably used, and ⁹⁰Y is still more preferably used.

In addition, when the radioactive labeled compound is used for the purpose of diagnosis of a disease or detection of a lesion, a β⁺ ray-emitting nuclide, an electron-capturing decay nuclide, or a γ ray-emitting nuclide is preferably used as the radioactive metal from a viewpoint of enhancing diagnostic performance. The β⁺ ray-emitting nuclide may be any nuclide that emits a positron in a decay process of the radioactive metal. ⁴⁴Sc, ⁵⁸Co, ⁶⁸Ga, ⁶⁴Cu, ⁸⁹Zr, or the like is preferably used, and ⁶⁴Cu or ⁸⁹Zr is more preferably used.

The electron-capturing decay nuclide may be any nuclide that emits an Auger electron or a characteristic X ray in a decay process of the radioactive metal. ⁵¹Cr, ⁵⁷Co, ⁵⁸Co, ⁶⁷Ga, ⁶⁸Ga, ⁶⁴Cu, ⁸⁹Zr, ¹¹¹In, ¹⁸⁶Re, ²⁰¹Tl, ¹⁹⁷Hg, or the like is preferably used.

The γ ray-emitting nuclide may be any nuclide that emits a γ ray by γ decay. As the nuclide that emits a γ ray by γ decay, ^{99m}Tc, ⁶⁸Ga, or ²⁰¹Tl is preferably used.

When the radioactive metal coordinated to the radioactive metal complex in an ionic state is selected based on an ionic radius, examples of a radioactive metal having an ionic radius of about 70 to 130 pm include ⁶⁷Ga, ⁶⁸Ga, ⁶⁴Cu, ⁶⁷Cu, ⁸⁹Zr, ⁹⁰Y, ^{99m}Tc, ¹⁰³Ru, ¹¹¹In ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, and ²²⁵Ac. These can each form a complex of the compound of the present invention having a chelating moiety having the structure represented by any one of the above formulas (A1) to (A9) and the radioactive metal ion.

For example, when the radioactive labeled compound is used for the purpose of treatment of a disease and ²²⁵Ac is used as the radioactive metal, a compound having a chelating moiety having the structure represented by any one of the above formulas (A1), (A3) to (A5), and (A7) is preferably used, and a compound having a chelating moiety having the structure represented by the above formula (A1) is more preferably used as the compound of the present invention. When ⁹⁰Y is used as the radioactive metal, a compound having a chelating moiety having the structure represented by any one of the above formulas (A1) to (A3) and (A8) is preferably used, and a compound having a chelating moiety having the structure represented by the above formula (A1) is more preferably used as the compound of the present invention.

When the radioactive labeled compound is used for the purpose of diagnosis of a disease or detection of a lesion and ⁸⁹Zr is used as the radioactive metal, a compound having a chelating moiety having the structure represented by any one of the above formulas (A1), (A3), and (A4) is preferably used, and a compound having a chelating moiety having the structure represented by the above formula (A1) is more preferably used as the compound of the present invention. When ⁶⁸Ga or ¹¹¹In is used as the radioactive metal, a compound having a chelating moiety having the structure represented by any one of the above formulas (A1) to (A4) and (A9) is preferably used, and a compound having a chelating moiety having the structure represented by the above formula (A1) is more preferably used as the compound of the present invention.

Regarding binding between the chelating moiety and the atomic group containing an albumin binding moiety, the chelating moiety and the albumin binding moiety may directly bind to each other without interposing a linker structure described later therebetween, or the chelating moiety and the atomic group containing an albumin binding moiety may indirectly bind to each other via the linker structure described later.

Similarly, regarding binding between the chelating moiety and the atomic group containing a target molecule binding moiety, the chelating moiety and the target molecule binding moiety may directly bind to each other without interposing the linker structure described later therebetween, or the chelating moiety and the atomic group containing an albumin binding moiety may indirectly bind to each other via the linker structure described later.

In either of the above-described forms "directly" and "indirectly", binding via an amide bond is preferable from a viewpoint of achieving both easy synthesis and a stable chemical structure.

The linker structure is preferably a structure derived from a compound capable of forming an amide bond. Specific examples thereof include structures derived from an L-form or D-form amino acid such as an acidic amino acid including glutamic acid and aspartic acid or a basic amino acid including lysine, a dicarboxylic acid such as oxalic acid or malonic acid, and a diamine such as ethylenediamine.

When a structure derived from an amino acid or the like is included as the above-described linker structure, for example, for the purpose of controlling in vivo kinetics, peptide linkers described in WO 2017/150549 A, WO 2019/065774 A, WO 2019/221269 A, WO 2020/075746 A, WO 2020/145227 A, and WO 2020/145228 A can be used.

When a structure derived from ethylene glycol is included as the above-described linker structure, preferably, binding is indirectly made by a linker structure represented by the following formula (P). The structure is derived from ethylene glycol, and in formula (P), n represents preferably an integer of 2 or more and 10 or less, more preferably an integer of 2 or more and 8 or less, and still more preferably an integer of 2 or more and 5 or less.

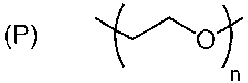

These linker structures may be constituted by one type of linker structure. Alternatively, one type of linker structure may repeatedly bind to each other in a linear or branched manner, or a plurality of types of linker structures may bind to each other in combination in a linear or branched manner.

As another form of the above-described case where the chelating moiety and the albumin binding moiety "indirectly" bind to each other or in the above-described case where the chelating moiety and the target molecule binding moiety "indirectly" bind to each other, these moieties may be linked to each other by a known coupling method, and for example, a click reaction can be used. Hereinafter, a case where the click reaction is used for binding between the chelating moiety and the target molecule binding moiety will be described as an example. In this case, the chelating moiety and the target molecule binding moiety each have an atomic group capable of undergoing the click reaction, and these atomic groups react with each other such that the chelating moiety and the target molecule binding moiety can bind to each other. That is, the reaction is performed between a first atomic group included in the chelating moiety and a second atomic group included in the target molecule binding moiety.

In the present invention, as a combination of atomic groups capable of undergoing the click reaction, an appropriate combination is selected according to the type of click reaction, and examples thereof include a combination of an alkyne and an azide and a combination of 1,2,4,5-tetrazine and an alkene. In these atomic groups, the first atomic group only needs to have one of the above atomic groups, and the second atomic group only needs to have an atomic group to be combined with the first atomic group. Preferably, the first atomic group is an alkyne and the second atomic group is an azide, or the first atomic group is 1,2,4,5-tetrazine and the second atomic group is an alkene from a viewpoint of achieving both stability of the chelating moiety and the target molecule binding moiety and improvement of binding efficiency thereof. Specific examples of the click reaction by such a combination of atomic groups include a Huisgen cycloaddition reaction and a reverse electron request type Diels-Alder reaction.

Specific examples of the combination of atomic groups capable of undergoing the click reaction include a combination of an atomic group (formula (11a)) containing dibenzylcyclooctyne (DBCO) as an alkyne of the first atomic group and an atomic group (formula (12a)) containing an azide group as an azide of the second atomic group, and a combination of an atomic group (formula (11b)) containing 1,2,4,5-tetrazine in the first atomic group and an atomic group (formula (12b)) containing trans-cyclooctene (TCO) as an alkene of the second atomic group, as illustrated in the following formulas.

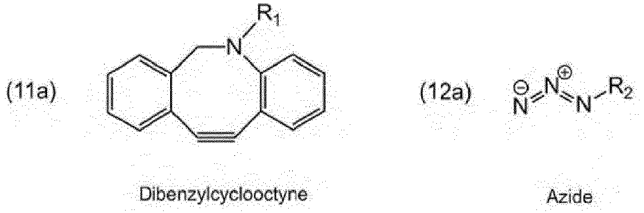

In formula (11a), R₁ represents a binding site to the chelating moiety, and in formula (12a), R₂ represents a binding site to the target molecule binding moiety.

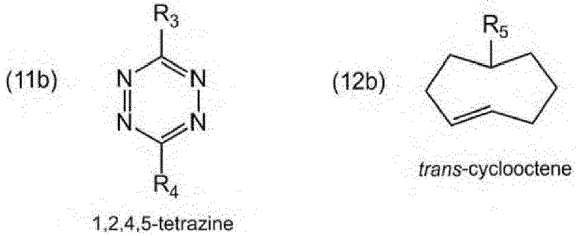

In formula (11b), one of R₃ and R₄ represents a binding site to the chelating moiety or the target molecule binding moiety, and the other represents a hydrogen atom, a methyl group, a phenyl group, or a pyridyl group, and in formula (12b), R₅ represents a binding site to the chelating moiety or the target molecule binding moiety.

When the chelating moiety and the target molecule binding moiety bind to each other by the click reaction in the present invention, the order of adding these is not limited as long as the click reaction can proceed. For example, one of the chelating moiety and the target molecule binding moiety may be added to a reaction vessel containing a solvent, and then the other may be added to cause a reaction, or one of the chelating moiety and the target molecule binding moiety may be dispersed in a solvent to obtain a dispersion, and then the other may be added to the dispersion to cause a reaction. Alternatively, the chelating moiety and the target molecule binding moiety may be simultaneously added to a reaction vessel containing a solvent to cause a reaction.

In each of the above-described embodiments, examples of a substituent that can be used for substitution in the chemical structure of each of A, B, C, the compound, and the radioactive labeled compound include a halogen atom, a saturated or unsaturated alkyl group, a hydroxy group, an aldehyde group, a carboxy group, an acyl group, an amino group, a nitro group, an ester group, an isothiocyanate group, a thioxy group, a cyano group, an amide group, an imide group, a phosphate group, a phenyl group, a benzyl group, and a pyridyl group. These substituents may be used singly or in combination of two or more thereof.

Although the present invention has been described above based on the preferred embodiments thereof, the present invention is not limited to the above-described embodiments. For example, in each of the above-described embodiments, the compound having one chelating moiety, one albumin binding moiety, and one target molecule binding moiety has been described. However, a plurality of the albumin binding moieties and/or a plurality of the target molecule binding moieties may be present in one chemical structure as long as the present invention is exerted.

### Examples

Hereinafter, the present invention will be described in more detail with reference to Examples. However, the scope of the present invention is not limited to such Examples.

In the following Examples, unless otherwise specified, NMR was performed with a JNM-AL400 FT-NMR apparatus manufactured by JEOL Ltd. using tetramethylsilane (TMS) as an internal standard substance, and TMS resonance was set to 0.00 ppm. All chemical shifts were indicated using ppm on the delta scale (δ), and signal multiplicity was indicated using abbreviations (s: singlet, d: doublet, t: triplet, m: multiplet, br: broad).

In mass spectrometry, LCMS 2020 (manufactured by Shimadzu Corporation) was used when MS was performed, and LCMS-IT-TOF (manufactured by Shimadzu Corporation) was used when HRMS was performed.

### Examples 1 to 4

In the present Examples, as illustrated in formula (2a), a compound in which CA-IX as a target molecule was targeted, and an iodine atom (Example 1), a methyl group (Example 2), a bromine atom (Example 3), or a hydrogen atom (Example 4) was used as R was synthesized, and subsequently a radioactive labeled compound in which an ¹¹¹In ion as a radioactive metal was coordinated to each compound was obtained. Hereinafter, an outline of a reaction path in each of the Examples is illustrated as a reaction path (III).

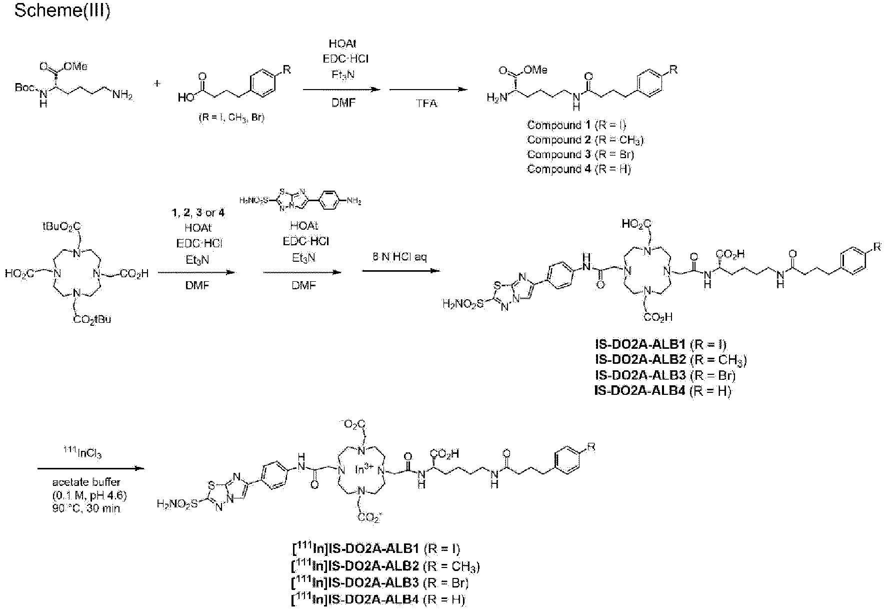

### <Example 1>

### (1) Synthesis of compound

First, methyl N⁶-(4-(4-iodophenyl)butanoyl)-L-lysinate (compound 1)) was synthesized by the following method.

Boc-Lys(OMe)-OH (118 mg, 0.40 mmol) was dissolved in 20 mL of anhydrous N,N-dimethylformamide (DMF), and 4-(4-iodophenyl) butyric acid (116 mg, 0.40 mmol), 1-ethyl-3-(3-dimethylaminopropyl)carbodiimide hydrochloride (EDC•HCl) (153 mg, 0.80 mmol), 1-hydroxybenzotriazole (HOAt) (109 mg, 0.80 mmol), and triethylamine (150 µL, 1.1 mmol) were added thereto to obtain a mixed liquid.

This mixed liquid was stirred overnight at room temperature under an argon atmosphere, and then the mixed liquid was freeze-dried to obtain a residue. A mixed liquid obtained by adding 2 mL of trifluoroacetic acid (TFA) to this residue was stirred at room temperature for three hours and then concentrated, and the residue was purified by medium-pressure silica gel chromatography (chloroform: methanol) to obtain 172 mg (yield: 100%) of a target product as a pale yellow oily substance.

¹H-NMR of compound 1 (400 MHz, CDCl₃) δ 7.57 (d, J = 8.4 Hz, 2H), 6.91 (d, J = 8.0 Hz, 2H), 4.03 (s, 1H), 3.77 (s, 3H), 3.20 (s, 2H), 2.54 (t, J = 7.6 Hz, 2H), 2.18 (t, J = 7.4 Hz, 2H), 1.98 (s, 2H), 1.87 (t, J = 7.2 Hz, 2H), 1.50-1.40 (m, 4H).

¹³C-NMR of compound 1 (100 MHz, CDCl₃) δ 173.9, 170.0, 141.1, 137.2 (2C), 130.5 (2C), 90.9, 53.1, 52.8, 38.7, 35.4, 34.5, 29.6, 28.4, 27.0, 21.7.

MS (ESI) m/z of compound 1 C₁₇H₂₆IN₂O₃, calculated value: 433.1; measured value: 433.1 ([M+H]⁺).

Subsequently, as the compound, IS-DO2A-ALB1 ((S)-2,2'-(-(2-((1-carboxy-5-(4-(4-iodophenyl)butanamide)pentyl)amino)-2-oxoethyl)-10-(2-oxo-2-((4-(2-sulfamoylimidazo[2,1-b][1,3,4]thiadiazol-6-yl)phenyl)amino)ethyl)-1,4,7,10-tetraazacyclododecane-1,7-diyl) diacetic acid) was synthesized by the following method.

The above-described compound 1 (86 mg, 0.20 mmol) was dissolved in anhydrous DMF (5 mL), and subsequently 2,2'-(4,10-bis(2-(tert-butoxy)-2-oxoethyl)-1,4,7,10-tetraazacyclododecane-1,7-diyl) diacetic acid (103 mg, 0.20 mmol), EDC•HCl (38 mg, 0.20 mmol), HOAt (27 mg, 0.20 mmol), and triethylamine (27 µL, 0.20 mmol) were added thereto to obtain a mixed liquid. This mixed liquid was stirred at room temperature under an argon atmosphere for 24 hours.

Thereafter, 6-(4-aminophenyl)imidazo[2,1-b][1,3,4]thiadiazole-2-sulfonamide (59 mg, 0.20 mmol), EDC•HCl (38 mg, 0.20 mmol), HOAt (27 mg, 0.20 mmol), and triethylamine (27 µL, 0.20 mmol) were further added to the mixed liquid, and the mixture was further stirred at room temperature under an argon atmosphere for 24 hours.

After completion of the reaction, 6 N hydrochloric acid was slowly added to a residue obtained by freeze-drying the mixed liquid in an ice bath, and the mixture was stirred at room temperature for 12 hours. Thereafter, the reaction liquid was concentrated and purified by reverse phase high performance liquid chromatography (HPLC) under the following conditions to obtain a desired compound (IS-DO2A-ALB1). An obtained amount was 2.5 mg (yield: 1.2%).

HRMS (ESI) m/z of IS-DO2A-ALB1 C₄₂H₅₆IN₁₁NaO₁₁S₂, calculated value: 1104.2539; measured value: 1104.2458 ([M+Na]⁺).

HPLC purification conditions (first time): Cosmosil 5C₁₈-AR-II column (20 x 250 mm), mobile phase: MeCN/H₂O/TFA [gradient from 15/85/0.1 (vol%, 0 min) to 45/55/0.1 (vol%, 60 min)], flow rate: 5 mL/min.

HPLC purification conditions (second time): Cosmosil 5C₁₈-AR-II column (4.6 x 150 mm), mobile phase: MeCN/H₂O/TFA [gradient from 15/85/0.1 (vol%, 0 min) to 45/55/0.1 (vol%, 120 min)], flow rate: 1 mL/min.

### (2) Synthesis of radioactive labeled compound

Finally, as the target radioactive labeled compound, [¹¹¹In]IS-DO2A-ALB1([¹¹¹In]indium (III)(S)-2,2'-4-(2-((1-carboxy-5-(4-(4-iodophenyl)butanamide)pentyl)amino)-2-oxoethyl)-10-(2-oxo-2-((4-(2-sulfamoylimidazo[2,1-b][1,3,4]thiadiazol-6-yl)phenyl)amino)ethyl)-1,4,7,10-tetraazacyclododecane-1,7-diyl) diacetic acid) was obtained.

¹¹¹In was used as the radioactive metal to be coordinated. A sodium acetate buffer (0.1 M, pH 4.6, 200 µL) and [¹¹¹In] indium chloride (100 µL) as a radioactive metal source were allowed to stand in a protein low adsorption tube (1.5 mL) at room temperature for 10 minutes. Thereafter, a DMSO solution (1 mM, 10 µL) of IS-DO2A-ALB1 was added to the buffer, mixed, and reacted at 90°C for 30 minutes. Thereafter, the reaction liquid was purified by reverse phase HPLC under similar conditions to those described above to obtain [¹¹¹In]IS-DO2A-ALB1 as a radioactive labeled compound in which a radioactive metal ion was coordinated to the compound. A radiochemical yield was 31%, and a radiochemical purity was higher than 99%.

### <Example 2>

### (1) Synthesis of compound

Methyl N⁶-(4-4-tolyl)butanoyl)-L-lysinate (compound 2) was synthesized by the following method.

A reaction similar to that in Example 1 was performed except that 4-(4-tolyl) butyric acid (71 mg, 0.40 mmol) was used in place of 4-(4-iodophenyl) butyric acid in Example 1 to obtain 128 mg (yield: 100%) of compound 2 as a pale yellow oily substance.

¹H-NMR of compound 2 (400 MHz, CDCl₃) δ 8.58 (s, 2H), 7.06 (d, J = 8.4 Hz,2H), 7.02 (d, J = 8.4 Hz, 2H), 6.43 (s, 1H), 4.02 (t, J = 6.0 Hz, 1H), 3.73 (s, 1H), 3.17 (d, J = 5.2 Hz, 2H), 2.54 (t, J = 7.6 Hz, 2H), 2.29 (s, 3H), 2.17 (t, J = 7.4 Hz, 2H), 1.97 (d, J = 6.4 Hz,2H), 1.90-1.82 (m, 2H), 1.48 (br, 4H).

¹³C-NMR of compound 2 (100 MHz, CDCl₃) δ 174.0, 169.9, 138.3, 135.2, 128.9 (2C), 128.1 (2C), 53.0, 52.8, 38.6, 35.6, 34.6, 29.6, 28.4, 27.3, 21.8, 20.8.

MS (ESI) m/z of compound 2 C₁₈H₂₉N₂O₃, calculated value: 321.2; measured value: 321.2 ([M+H]⁺).

Subsequently, reaction and purification were performed in a similar manner to Example 1 using compound 2, and IS-DO2A-ALB2 ((S)-2,2'-4-(2-((1-carboxy-5-(4-(4-tolyl)butanamide)pentyl)amino)-2-oxoethyl)-10-(2-oxo-2-((4-(2-sulfamoylimidazo[2,1-b][1,3,4]thiadiazol-6-yl)phenyl)amino)ethyl)-1,4,7,10-tetraazacyclododecane-1,7-diyl) diacetic acid) was synthesized. An obtained amount was 1.3 mg, and an yield was 0.6%.

HRMS (ESI) m/z of IS-DO2A-ALB2 C₄₃H₆₀N₁₁O₁₁S₂, calculated value: 970.3910; measured value: 970.3911 ([M+H]⁺).

### (2) Synthesis of radioactive labeled compound

Reaction and purification were performed in a similar manner to Example 1 using IS-DO2A-ALB2, and [¹¹¹In]IS-DO2A-ALB2 ([¹¹¹In] indium(III) (S)-2,2'-(4-(2-((1-carboxy-5-(4-(4-tolyl)butanamide)pentyl)amino)-2-oxoethyl)-10-(2-oxo-2-((4-(2-sulfamoylimidazo[2,1-b][1,3,4]thiadiazol-6-yl)phenyl)amino)ethyl)-1,4,7,10-tetraazacyclododecane-1,7-diyl) diacetic acid) as a radioactive labeled compound in which a radioactive metal ion was coordinated to the compound was obtained. A radiochemical yield was 44%, and a radiochemical purity was higher than 99%.

### <Example 3>

### (1) Synthesis of compound

Methyl N⁶-(4-(4-bromophenyl)butanoyl)-L-lysinate (compound 3) was synthesized by the following method.

Reaction similar to that in Example 1 was performed except that 4-(4-bromophenyl) butyric acid (97 mg, 0.40 mmol) was used in place of 4-(4-iodophenyl) butyric acid in Example 1 to obtain 154 mg (yield: 100%) of compound 3 as a pale yellow oily substance.

¹H-NMR of compound 3 (400 MHz, CDCl₃) δ 8.58 (s, 1H), 7.36 (d, J = 8.0 Hz, 2H), 7.02 (d, J = 8.0 Hz, 2H), 6.58 (t, J = 5.6 Hz, 1H), 4.02 (t, J = 6.4 Hz, 1H), 3.74 (s, 3H), 3.17 (d, J = 5.6 Hz, 2H), 2.54 (t, J = 7.6 Hz, 2H), 2.16 (t, J = 7.6 Hz, 2H), 2.00 (s, 2H), 1.89-1.84 (m, 2H), 1.48 (br, 4H).

¹³C-NMR of compound 3 (100 MHz, CDCl₃) δ 173.9, 170.0, 140.5, 131.2 (2C), 130.1 (2C), 119.5, 53.0, 52.9, 38.7, 35.4, 34.4, 29.6, 28.4, 27.0, 21.7.

MS (ESI) m/z of compound 3 C₁₇H₂₆N₂O₃, calculated value: 385.1; measured value: 385.1 ([M+H]⁺).

Subsequently, reaction and purification were performed in a similar manner to Example 1 using compound 3, and IS-DO2A-ALB3 ((S)-2,2'-(4-(2-((1-carboxy-5-(4-(4-bromophenyl)butanamide)pentyl)amino)-2-oxoethyl)-10-(2-oxo-2-((4-(2-sulfamoylimidazo[2,1-b][1,3,4]thiadiazol-6-yl)phenyl)amino)ethyl)-1,4,7,10-tetraazacyclododecane-1,7-diyl) diacetic acid) was synthesized. An obtained amount was 3 mg, and an yield was 1.5%.

HRMS (ESI) m/z of IS-DO2A-ALB3 C₄₂H₅₆BrN₁₁NaO₁₁S₂, calculated value: 1056.2678; measured value: 1056.2665 ([M+Na]⁺).

### (2) Synthesis of radioactive labeled compound

Reaction and purification were performed in a similar manner to Example 1 using IS-DO2A-ALB3, and [¹¹¹In]IS-DO2A-ALB3 ([¹¹¹In] indium(III) (S)-2,2'-(4-(2-((1-carboxy-5-(4-(4-bromophenyl)butanamide)pentyl)amino)-2-oxoethyl)-10-(2-oxo-2-((4-(2-sulfamoylimidazo[2,1-b][1,3,4]thiadiazol-6-yl)phenyl)amino)ethyl)-1,4,7,10-tetraazacyclododecane-1,7-diyl) diacetic acid) as a radioactive labeled compound in which a radioactive metal ion was coordinated to the compound was obtained. A radiochemical yield was 32%, and a radiochemical purity was higher than 99%.

### <Example 4>

### (1) Synthesis of compound

Methyl N⁶-(4-4-phenylbutanoyl)-L-lysinate (compound 4) was synthesized by the following method.

A reaction similar to that in Example 1 was performed except that 4-phenyl butyric acid (66 mg, 0.40 mmol) was used in place of 4-(4-iodophenyl) butyric acid in Example 1 to obtain 109 mg (yield: 89%) of compound 4 as a pale yellow oily substance.

¹H-NMR of compound 4 (400 MHz, CDCl₃) δ 8.53 (s, 2H), 7.25-7.11 (m, 5H), 6.74 (t, 1H), 4.00 (t, 1H), 3.69 (s, 1H), 3.14 (d, 2H), 2.57 (t, 2H), 2.15 (t, 2H), 1.94 (d, 2H), 1.97 (d, 2H), 1.90-1.82 (m, 2H), 1.45 (br, 4H).

¹³C-NMR of compound 4 (100 MHz, CDCl₃) δ 173.9, 170.0, 141.5, 128.3 (2C), 128.2 (2C), 125.8, 53.0, 52.8, 38.6, 35.6, 35.1, 29.6, 28.4, 27.2, 21.8.

MS (ESI) m/z of compound 4 C₁₇H₂₇N₂O₃, calculated value: 307.2; measured value: 307.2 ([M+H]⁺).

Subsequently, reaction and purification were performed in a similar manner to Example 1 using compound 4, and IS-DO2A-ALB4 ((S)-2,2'-(4-(2-((1-carboxy-5-(4-phenyl butanamide)pentyl)amino)-2-oxoethyl)-10-(2-oxo-2-((4-(2-sulfamoylimidazo[2,1-b][1,3,4]thiadiazol-6-yl)phenyl)amino)ethyl)-1,4,7,10-tetraazacyclododecane-1,7-diyl) diacetic acid) was synthesized. An obtained amount was 3 mg, and an yield was 1.7%.

HRMS (ESI) m/z of IS-DO2A-ALB4 C₄₂H₅₈N₁₁O₁₁S₂, calculated value: 956.3753; measured value: 956.3760 ([M+H]⁺).

### (2) Synthesis of radioactive labeled compound

Reaction and purification were performed in a similar manner to Example 1 using IS-DO2A-ALB4, and [¹¹¹In]IS-DO2A-ALB4 ([¹¹¹In] indium(III) (S)-2,2'-(-(2-((1-carboxy-5-(4-phenyl butanamide)pentyl)amino)-2-oxoethyl)-10-(2-oxo-2-((4-(2-sulfamoylimidazo[2,1-b][1,3,4]thiadiazol-6-yl)phenyl)amino)ethyl)-1,4,7,10-tetraazacyclododecane-1,7-diyl) diacetic acid) as a radioactive labeled compound in which a radioactive metal ion was coordinated to the compound was obtained. A radiochemical yield was 44%, and a radiochemical purity was higher than 99%.

### <Comparative Example 1>

[¹¹¹In]DO3A-IS1 was synthesized by the following method. An outline of a reaction path in the present Comparative Example is illustrated as a reaction path (IV).

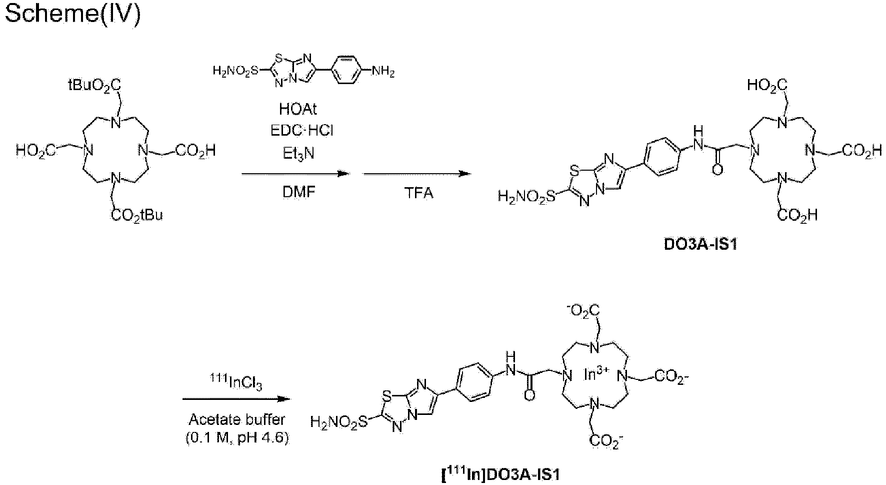

### (1) Synthesis of compound

DO3A-IS1 was synthesized by the following method. Specifically, 2,2'-(4,10-bis(2-(tert-butoxy)-2-oxoethyl)-1,4,7,10-tetraazacyclododecane-1,7-diyl) diacetic acid (103 mg, 0.20 mmol) was dissolved in anhydrous DMF (4 mL), and subsequently 6-(4-aminophenyl)imidazo[2,1-b][1,3,4]thiadiazole-2-sulfonamide (59 mg, 0.20 mmol), EDC•HCl (38 mg, 0.20 mmol), HOAt (27 mg, 0.20 mmol), and triethylamine (27 µL, 0.20 mmol) were added thereto to obtain a mixed liquid. This mixed liquid was stirred at room temperature under an argon atmosphere for 48 hours.

After completion of the reaction, trifluoroacetic acid was slowly added to a residue obtained by freeze-drying the mixed liquid in an ice bath, and the mixture was stirred at room temperature for seven hours. Thereafter, the reaction liquid was concentrated and purified by reverse phase HPLC under the following conditions to obtain a desired compound (D03A-IS1). An obtained amount was 52 mg (yield: 19%).

HPLC purification conditions (first time): Cosmosil 5C₁₈-AR-II column (20 × 250 mm), mobile phase: MeCN/H₂O/TFA [gradient from 10/90/0.1 (vol%, 5 min) to 25/75/0.1 (vol%, 75 min)], flow rate: 5 mL/min.

HPLC purification conditions (second time): Cosmosil 5C₁₈-AR-II column (10 × 250 mm), mobile phase: MeCN/H₂O/TFA [15/85/0.1 (vol%), flow rate: 2.8 mL/min.

NMR apparatus used: JNM-ECS -400 (manufactured by JEOL Ltd.)

¹H-NMR of DO3A-IS1 (400 MHz, DMSO-d₆) δ 10.59 (s, 1H), 8.81 (s, 1H), 8.75 (s, 2H), 7.86 (d, 2H, J = 21.5 Hz), 7.65 (d, 2H, J = 21.5 Hz), 3.9-4.1 (br, 16H), 3.3-3.1 (br, 8H).

HRMS (ESI) m/z of DO3A-IS1 C₂₆H₃₆N₉NaO₉S₂, calculated value: 682.2072; measured value: 682.2107 ([M+H]⁺).

### (2) Synthesis of radioactive labeled compound

[¹¹¹In]DO3A-IS1 was synthesized by the following method. Specifically, a sodium acetate buffer (0.1 M, pH 4.6, 200 µL) and [¹¹¹In] indium chloride (100 µL) as a radioactive metal source were allowed to stand in a protein low adsorption tube (1.5 mL) at room temperature for 10 minutes. Thereafter, a DMSO solution (20 mM, 10 µL) of D03A-IS1 was added to the buffer, mixed, and reacted at 90°C for 30 minutes. Thereafter, the reaction liquid was purified by reverse phase HPLC under the following conditions to obtain [¹¹¹In]DO3A-IS1 as a radioactive labeled compound in which a radioactive metal ion was coordinated to the compound. A radiochemical yield was 76%, and a radiochemical purity was higher than 99%.

HPLC purification conditions: Cosmosil 5C₁₈-AR-II column (4.6 × 150 mm), mobile phase: MeCN/H₂O/TFA [gradient from 10/90/0.1 (vol%, 0 min) to 40/60/0.1 (vol%, 30 min)], flow rate: 0.6 mL/min.

### <Evaluation 1: Distribution coefficient>

The radioactive labeled compound (740 kBq) of any one of Examples and Comparative Examples was added to a mixed liquid of PBS (3 mL, pH 7.4 (20°C)) and 1-octanol (3 mL), and the mixture was vortexed for two minutes and then centrifuged at 4000 × g for five minutes. Thereafter, 1 mL of the 1-octanol layer and 500 µL of the PBS layer were collected, and the amount of radioactivity of each of the layers was measured with a gamma counter (n = 5).

As a calculation formula of a distribution coefficient, "logio [(amount of radioactivity of 1-octanol layer)/(amount of radioactivity of PBS layer × 2)]" was used. Results are illustrated in Table 1 below as mean ± standard deviation (mean ± SD).

**[Table 1]**

| | Radioactive labeled compound | Distribution coefficient (mean ± SD) |
|---|---|---|
| Comparative Example 1 | [¹¹¹In]DO3A-IS1 | -3.72±0.05 |
| Example 1 | [¹¹¹In]IS-DO2A-ALB1 | -1.70±0.01 |
| Example 2 | [¹¹¹In]IS-DO2A-ALB2 | -2.16±0.04 |
| Example 3 | [¹¹¹In]IS-DO2A-ALB3 | -1.90±0.01 |
| Example 4 | [¹¹¹In]IS-DO2A-ALB4 | -2.36±0.01 |

### <Evaluation 2: cell binding inhibition assay>

In this evaluation, binding properties of [¹¹¹In]IS-DO2A-ALB1 to [¹¹¹In]IS-DO2A-ALB4 and [¹¹¹In]DO3A-IS1 with respect to CA-IX were evaluated.

Specifically, HT-29 cells (CA-IX highly expressing cells, purchased from Sumitomo Dainippon Pharma Co., Ltd.) were seeded together with a medium at 4.0 × 10⁵ cells/well in a 12 well plate, and cultured at 37°C for 24 hours in an atmosphere of 5% carbon dioxide.

Subsequently, the medium was removed, and a medium containing any one of the radioactive labeled compounds of Examples 1 to 4 and Comparative Example 1 (37 kBq/mL) was added to each well in an amount of 1 mL such that the acetazolamide concentration was 50000 to 0.00512 nM as an inhibitor, and the sample was cultured at 37°C for two hours in an atmosphere of 5% carbon dioxide. In addition, a sample containing the radioactive labeled compound and not containing acetazolamide was also cultured under similar conditions. The experiment was performed with each concentration group at n = 6.

Subsequently, the medium was removed, the residue was washed with PBS (1 mL), and cells were lysed with a 1 N sodium hydroxide aqueous solution (200 µL × 2) to obtain a cell lysate. Then, the amount of radioactivity in the cell lysate and that in the medium containing the radioactive labeled compound were each measured with a gamma counter (type name: Wallac 2470 Wizard manufactured by PerkinElmer, Massachusetts, U.S.A.). Separately from this, the total protein mass (mg protein) in the cell lysate was quantified by a BCA method. A value (%ID/mg protein) obtained by dividing the percentage (%ID) of the amount of radioactivity of a sample to the amount of added radioactivity by the total protein mass was calculated for each sample, and a 50% inhibitory concentration (IC₅₀) was calculated by GraphPad Prism based on the percentage of the amount of radioactivity (%ID/mg protein) to the total protein mass in a sample having each acetazolamide concentration when the amount of radioactivity (%ID/mg protein) to the total protein mass in a sample containing no acetazolamide was taken as 100%. Results are illustrated in Table 2 below as mean ± standard deviation (mean ± SD).

**[Table 2]**

| | Radioactive labeled compound | IC₅₀ (nM, mean ± SD) |
|---|---|---|
| Comparative Example 1 | [¹¹¹In]DO3A-IS1 | 126.7±19.6 |
| Example 1 | [¹¹¹In]IS-DO2A-ALB1 | 571.4±2.6 |
| Example 2 | [¹¹¹In]IS-DO2A-ALB2 | 165.1±60.6 |
| Example 3 | [¹¹¹In]IS-DO2A-ALB3 | 288.0±45.7 |
| Example 4 | [¹¹¹In]IS-DO2A-ALB4 | 105.4±8.0 |

In this assay, since the concentration range of the inhibitor was changed without changing the concentration range of each of the radioactive labeled compounds of Examples 1 to 4 and Comparative Example 1, it is indicated that as a calculated value of ICso is larger, a higher concentration of the inhibitor is required in order to inhibit binding between the radioactive labeled compound and CA-IX. That is, the larger the calculated value of ICso, the higher affinity between the radioactive labeled compound and CA-IX.

As illustrated in Table 1, the radioactive labeled compounds of Examples 1 to 4 have larger calculated values of ICso than that of Comparative Example 1. Therefore, it is found that the radioactive labeled compounds of Examples 1 to 4 have high affinity for CA-IX as a target molecule.

### <Evaluation 3: in vivo radioactivity distribution assay in HT-29 tumor-bearing mice>

Under 2% isoflurane anesthesia, a suspension of HT-29 cells (5 × 10⁶ cells/mouse) expressing CA-IX was subcutaneously injected into the left shoulder of each of 5-week-old male BALB/c nude mice (purchased from SHIMIZU Laboratory Supplies CO., LTD.). The composition of the suspension was a mixture of HT-29 cells/DMEM medium (manufactured by Thermo Fisher Scientific Inc.) and a matrix product (Geltrex (registered trademark) (manufactured by Thermo Fisher) at a volume ratio of 1 : 1.

Thereafter, the subcutaneously injected mice were raised for 14 days in a raising room that switches day and night every 12 hours with normal food and tap water to obtain HT-29 tumor-bearing mice.

Then, a saline solution (259 kBq, 100 µL, containing 3 mg of ascorbic acid) of each of the radioactive labeled compounds of Examples 1 to 3 or a saline solution (259 kBq, 100 µL, containing 1 mg of ascorbic acid) of the radioactive labeled compound of Comparative Example 1 was injected into each of the mice via the tail vein thereof. The mice (n = 5 in each group) were euthanized 1, 4, 8, 24, 48, 96, or 192 hours after the injection, blood, organs, and tumor sites were collected, and the masses thereof were measured. The amount of radioactivity was measured with a gamma counter.

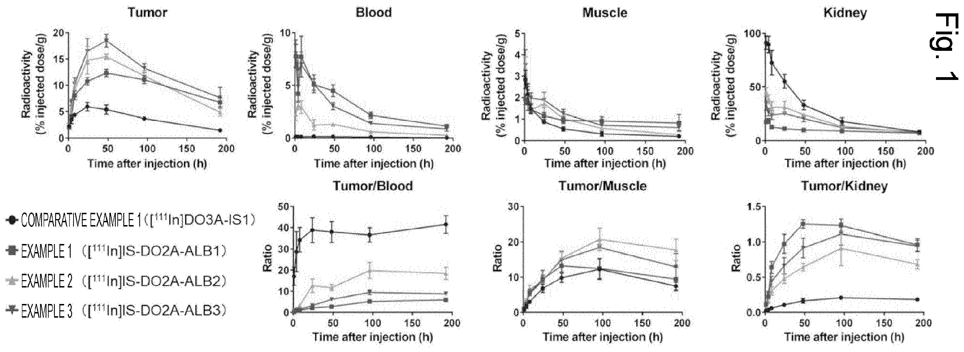

The degree of accumulation of the radioactive labeled compound is indicated as a value (%injected dose/g) obtained by dividing the percentage (%ID) of the amount of radioactivity with respect to the amount of injected radioactivity (injected dose) by the mass of blood or the mass of an organ (g). The higher a value of %ID/g, the higher the abundance of the radioactive labeled compound, indicating that the amount of the compound accumulated in the target organ is large. Results are illustrated in Fig. 1.

As illustrated in Fig. 1, it is found that the radioactive labeled compounds of Examples 1 to 3 are present in the blood for a long time, and a blood half-life is long. In addition, the amount of each of the radioactive labeled compounds of Examples 1 to 3 accumulated in the kidney is small, but the amount of each of the radioactive labeled compounds of Examples 1 to 3 accumulated in the tumor expressing CA-IX is large. Therefore, it is found that each of the radioactive labeled compounds of Examples 1 to 3 is likely to be specifically accumulated in the tumor particularly due to binding of CA-IX as a target molecule.

On the other hand, it is found that the amount of the radioactive labeled compound of Comparative Example 1 accumulated in the kidney is very large as compared with the results of Examples, and as a result, the amount of the radioactive labeled compound of Comparative Example 1 accumulated in the tumor is poor.

Therefore, the compound of the present invention and a radioactive labeled compound using the compound can achieve both improvement of accumulation in a target tissue and reduction of accumulation in a non-target tissue, particularly reduction of accumulation in the kidney.

### <Evaluation 4: in vivo radioactivity distribution assay in MDA-MB-231 tumor-bearing mice>

In a similar manner to Evaluation 3, a suspension of MDA-MB-231 cells (1 × 10⁷ cells/mouse) having low expression of CA-IX was subcutaneously injected into the left shoulder of each of 5-week-old male BALB/c nude mice to obtain MDA-MB-231 tumor-bearing mice.

Then, a saline solution (40 kBq, 100 µL, containing 3 mg of ascorbic acid) of the radioactive labeled compound of Example 1 was injected into each of the mice via the tail vein thereof. Mice (n = 5 in each group) were euthanized 24 hours after the injection, blood, organs, and tumor sites were collected, and the masses thereof were measured. The amount of radioactivity was measured with a gamma counter.

The degree of accumulation of the radioactive labeled compound is indicated as a value (%ID/g) obtained by dividing the percentage (%ID) of the amount of radioactivity with respect to the amount of injected radioactivity (injected dose) by the mass of blood or the mass of an organ (g). The higher a value of %ID/g, the higher the abundance of the radioactive labeled compound, indicating that the amount of the compound accumulated in the target organ is large. Results are illustrated in Table 3 as mean ± standard deviation (n = 5).

**[Table 3]**

| Radioactivity accumulated amount (24 hours after injection) [%ID/g] | Blood | 15.50±1.17 |
|---|---|---|
| | Spleen | 2.04±0.45 |
| | Pancreas | 2.27±0.30 |
| | Stomach [%ID] | 4.44±0.48 |
| | Intestinal | 4.65±0.48 |
| | Kidney | 11.42±0.65 |
| | Liver | 2.64±0.47 |
| | Heart | 3.12±0.25 |
| | Lung | 7.28±0.85 |
| | Brain | 0.34±0.03 |
| | Tumor (MDA-MB-231) | 5.05±0.59 |
| | Muscle | 1.21±0.22 |
| Tumor/blood radioactivity ratio | | 0.94±0.17 |
| Tumor/muscle radioactivity ratio | | 4.22±0.47 |
| Tumor/kidney radioactivity ratio | | 0.44±0.05 |

As illustrated in Table 3, accumulation of the radioactive labeled compound of Example 1 in the MDA-MB-231 tumor was lower than accumulation thereof in the HT-29 tumor (10.73%ID/g) in Evaluation 3. Therefore, it is found that the amount of the radioactive labeled compound accumulated in the CA-IX-highly expressing tumor is large.

### <Evaluation 5: SPECT/CT in HT-29 tumor-bearing mice>

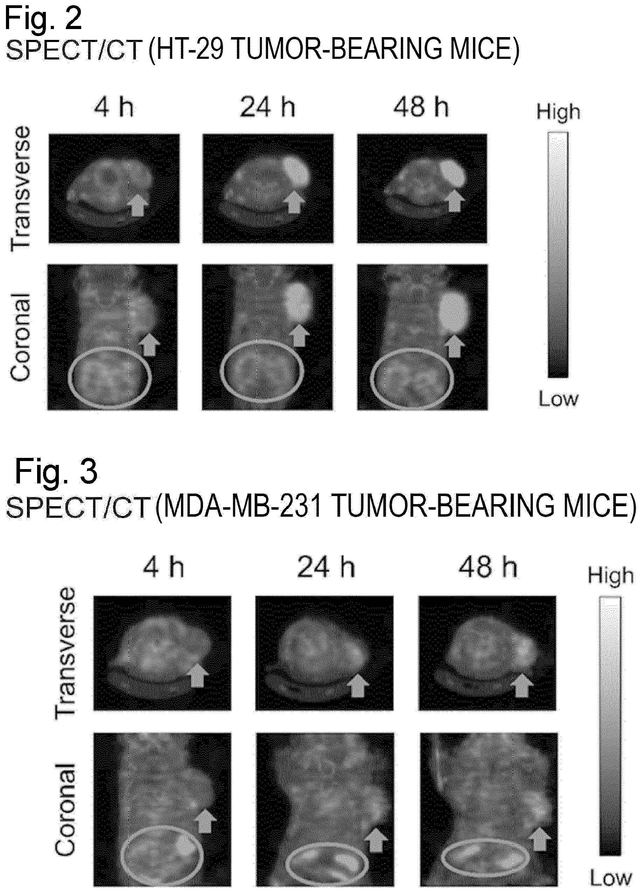

HT-29 tumor-bearing mice were obtained in a similar manner to that in Evaluation 3, and then a saline solution (7.6 to 8.0 MBq, 150 µL, containing 3 mg ascorbic acid) of the radioactive labeled compound of Example 1 was injected into each of the mice via the tail vein thereof. SPECT/CT was performed with an FX3300 pre-clinical imaging system manufactured by Gamma Medica-Ideas 4, 24, and 48 hours after the injection. SPECT/CT was performed by imaging tumor-bearing mice under 2% isoflurane anesthesia using a pinhole collimator with a diameter of 1.0 mm at a rotation radius of 35 mm, a scan time of 70 seconds, and the number of times of imaging of 32. After SPECT, CT (tube voltage: 60 kV, tube current: 350 µA) was further performed under the same anesthesia. Image reconstruction by a three-dimensional ordered subset expectation maximization method (8 subsets, 5 iterations) was performed on projection data of SPECT. Results are illustrated in Fig. 2. In Fig. 2, a portion indicated by an arrow is a position where the HT-29 tumor is present, and a portion indicated by a circle is a position where the kidney is present.

### <Evaluation 6: SPECT/CT in MDA-MB-231 tumor-bearing mice>

MDA-MB-231 tumor-bearing mice were obtained in a similar manner to that in Evaluation 4, and then SPECT/CT was performed under similar conditions to those in Evaluation 5. Results are illustrated in Fig. 3. In Fig. 3, a portion indicated by an arrow is a position where the MDA-MB-231 tumor is present, and a portion indicated by a circle is a position where the gastrointestinal tract is present.

As illustrated in Figs. 2 and 3, it is found that a radioactivity signal of the HT-29 tumor highly expressing CA-IX (see Fig. 2) is higher than a radioactivity signal of the MDA-MB-231 tumor (see Fig. 3). Therefore, it is found that the radioactive labeled compound of the present invention can clearly delineate a tumor highly expressing CA-IX.

### Examples 5-1 to 5-3 and Comparative Examples 2-1 to 2-3

In the present Examples, two compounds (PSMA-DA1 and PSMA-DB) targeting PSMA as a target molecule were synthesized. Subsequently, a radioactive labeled compound in which an ¹¹¹In ion, a ⁹⁰Y ion, or a ²²⁵Ac ion was coordinated as a radioactive metal to each of the compounds was obtained. Details are described below.

PSMA-DA1 used in Examples 5-1 to 5-3 contains a chelating moiety, a target molecule binding moiety, and an albumin binding moiety in the structure thereof. PSMA-DB used in Comparative Examples 2-1 to 2-3 contains a chelating moiety and a target molecule binding moiety, but does not contain an albumin binding moiety in the structure thereof.

### <Examples 5-1 to 5-3>

An outline of a reaction path in Examples 5-1 to 5-3 is illustrated below as reaction paths (V-1) and (V-2).

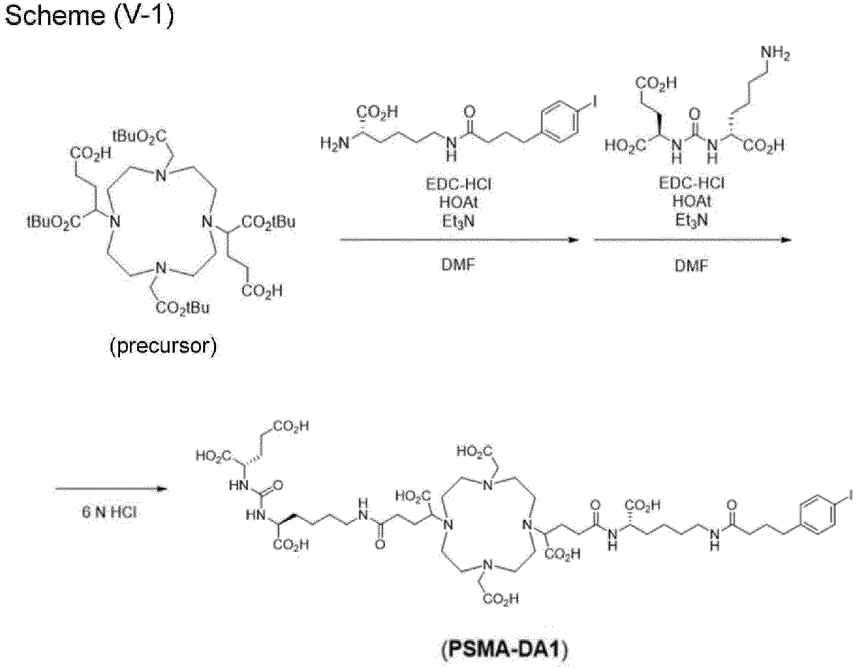

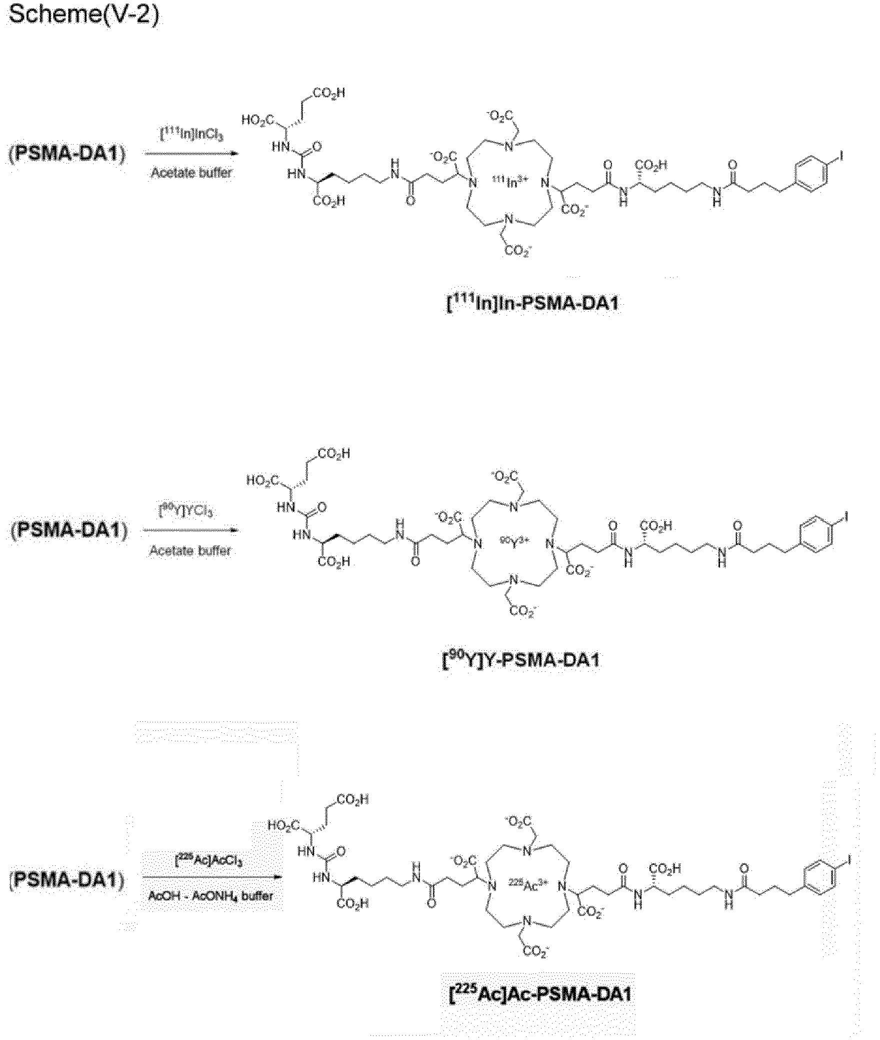

### (1) Synthesis of PSMA-DA1

According to the method of Chem Commun. 2008, 28, 3248-3250, a precursor compound synthesized from 1,4,7,10-tetraazacyclododecane in three steps was obtained. This precursor compound (20 mg, 0.026 mmol) was dissolved in N,N-dimethylformamide (DMF) (2 mL), and methyl-N⁶-(4-(4-iodophenyl)butanoyl)-L-lysinate (11 mg, 0.0254 mmol), 1-ethyl-3-(3-dimethylaminopropyl)-carbodiimide (EDC) hydrochloride (7.0 mg, 0.037 mmol), 1-hydroxy-7-azabenzotriazole (HOAt) (5.0 mg, 0.037 mmol), and triethylamine (5 µL, 0.036 mmol) were added thereto. The mixture was stirred at room temperature for 24 hours. Thereafter, (S)-di-tert-butyl 2-(3-((S)-6-amino-1-tert-butoxy-1-oxohexan-2-yl)ureido) pentanedioate (13 mg, 0.0267 mmol)•EDC hydrochloride (7.0 mg, 0.037 mmol), HOAt (5.0 mg, 0.037 mmol), and triethylamine (5 µL, 0.036 mmol) were added thereto, and the mixture was stirred at room temperature for 72 hours. The solvent was removed. Thereafter, 6 N hydrochloric acid (3 mL) was added to the residue, and the mixture was stirred at 40°C for 24 hours and then purified by reverse phase HPLC under the following conditions to obtain a desired compound (PSMA-DA1). An obtained amount and MS were as follows.

Purification conditions: Cosmosil 5C₁₈-AR-II column (10 × 250 mm), mobile phase: MeCN/H₂O/trifluoroacetic acid (TFA) [10/90/0.1 (0 min) to 100/0/0.1 (90 min)], flow rate: 4 mL/min.

Obtained amount: 1.0 mg (3%).

MS(ESI)m/z1250.5[M+H]⁺.

### (2) ¹¹¹In label (Example 5-1)

An [¹¹¹In]InCl₃ solution (3.7 MBq, 100 µL) and a DMSO solution of PSMA-DA1 (1 mM, 10 µL) were added to an acetate buffer (0.1 M, pH 5.5, 200 µL), and the mixture was allowed to stand at 90°C for 30 minutes. Thereafter, the reaction liquid was purified by reverse phase HPLC under the following conditions to obtain a desired radioactive labeled compound ([¹¹¹In]In-PSMA-DA1).

As a result, a radiochemical yield was 61 to 90%, and a radiochemical purity was 95% or more.

Purification conditions with ¹¹¹In label: Cosmosil 5C₁₈-PAQ column (4.6 × 250 mm), mobile phase: MeCN/H₂O/TFA [20/80/0.1 (0 min) to 50/50/0.1 (30 min)] or 5/95/0.1 (0 to 10 min), 5/95/0.1 (10 min) to 35/65/0.1 (40 min)], flow rate: 1 mL/min.

Note that a compound in which PSMA-DA1 is coordinated to non-radioactive In can be manufactured, for example, by the following method.

PSMA-DA1 (1 mg) and indium (III) chloride nonhydrate (2 mg) were dissolved in dimethyl sulfoxide (DMSO) (100 µL), and a 2-(N-morpholino) ethanesulfonic acid buffer (0.1 M, pH 5.6, 900 µL) was added thereto. The reaction liquid was stirred at 60°C for 12 hours, and then the solution was purified by reverse phase HPLC using the following method to obtain a compound having the following MS.

Purification conditions: Cosmosil 5C₁₈-AR-II column (10 × 250 mm), mobile phase: MeCN/H₂O/TFA [10/90/0.1 (0 min) to 100/0/0.1 (90 min)], flow rate: 4 mL/min.

MS(ESI):m/z1362.4[M+H]⁺.

### (3) ⁹⁰Y label (Example 5-2)

A [⁹⁰Y]YCl₃ solution (65-118 MBq, 10 µL) and a DMSO solution of PSMA-DA1 (1 mM, 10 µL) were added to an acetate buffer (0.1 M, pH 5.5, 200 µL), and the mixture was allowed to stand at 90°C for 30 minutes. Thereafter, the reaction liquid was purified by reverse phase HPLC under the following conditions to obtain a desired radioactive labeled compound ([⁹⁰Y]Y-PSMA-DA1).

As a result, a radiochemical yield was 49 to 79%, and a radiochemical purity was 95% or more.

Purification conditions with ⁹⁰Y label: Cosmosil 5C₁₈-PAQ column (4.6 × 250 mm), mobile phase: MeCN/H₂O/TFA [20/80/0.1 (0 min) to 50/50/0.1 (30 min)] or 5/95/0.1 (0 to 10 min), 5/95/0.1 (10 min) to 35/65/0.1 (40 min)], flow rate: 1 mL/min.

### (4) ²²⁵Ac label (Example 5-3)

A 0.1 M acetic acid-ammonium acetate buffer (pH 5.5, 170 µL) and a DMSO solution of PSMA-DA1 (2.0 M, 10 µL) were added to a 0.2 M hydrochloric acid solution (1.5 MBq, 10 µL) of [²²⁵Ac]AcCl₃, and the mixture was allowed to stand at 70°C for one hour. H₂O (800 µL) was added to the reaction liquid, and the mixture was caused to pass through an Oasis HLB Light column. H₂O (10 mL) was caused to pass through the column, and then 70% EtOH (0.5 mL) was caused to pass through the column to obtain a desired radioactive labeled compound ([²²⁵Ac]Ac-PSMA-DA1).

As a result, a radiochemical yield was 48 to 49%, and a radiochemical purity was 83 to 87%.

### <Comparative Examples 2-1 to 2-3>

An outline of a reaction path in Comparative Examples 2-1 to 2-3 is illustrated below as reaction paths (VI-1) and (VI-2).

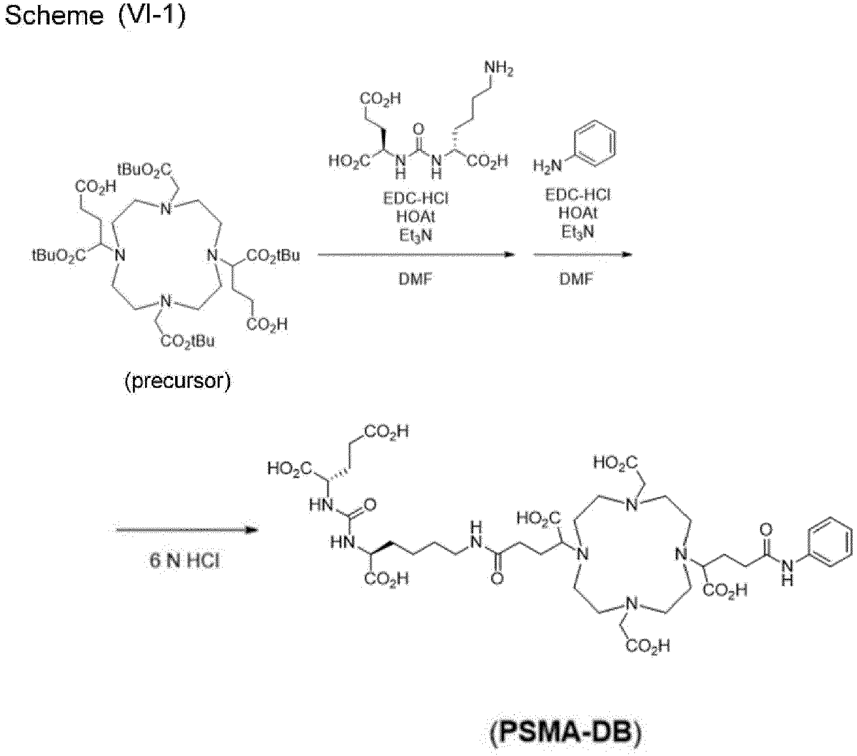

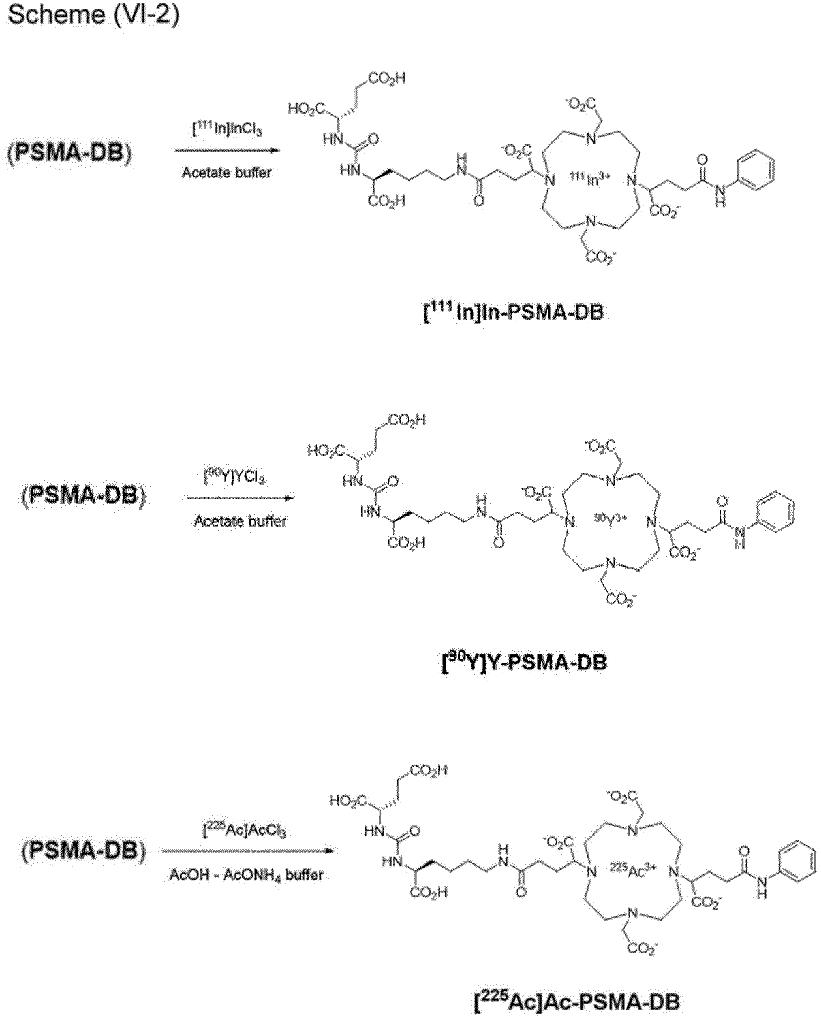

### (1) Synthesis of PSMA-DB

A precursor compound (35 mg, 0.045 mmol) synthesized in a similar manner to Example 5 was dissolved in DMF (2 mL), and (S)-di-tert-butyl-2-(3-((S)-6-amino-1-tert-butoxy-1-oxohexan-2-yl)ureido) pentanedioate (22 mg, 0.045 mmol), EDC hydrochloride (10 mg, 0.052 mmol), HOAt (7.0 mg, 0.051 mmol), and triethylamine (7 µL, 0.050 mmol) were added thereto. The mixture was stirred at room temperature for 24 hours. Thereafter, aniline (5 µL, 0.055 mmol), EDC hydrochloride (10 mg, 0.052 mmol), HOAt (7.0 mg, 0.051 mmol), and triethylamine (7 µL, 0.050 mmol) were added thereto, and the mixture was stirred at room temperature for 24 hours. The solvent was removed. Thereafter, TFA (1.8 mL), triisopropylsilane (100 µL), and H₂O (100 µL) were added to the residue, and the mixture was stirred at room temperature for 24 hours. The solvent was removed. Thereafter, the residue was purified by reverse phase HPLC under the following conditions to obtain a desired compound (PSMA-DB). An obtained amount and MS were as follows.

Purification conditions: Cosmosil 5C₁₈-AR-II column (10 × 250 mm), mobile phase: MeCN/H₂O/TFA [5/95/0.1 (0 to 10 min), 5/95/0.1 (10 min) to 35/65/0.1 (40 min)], flow rate: 4 mL/min.

Obtained amount: 5.0 mg (12%).

MS(ESI)m/z925.3[M+H]⁺.

### (2) ¹¹¹In label (Comparative Example 2-1)

A desired radioactive labeled compound ([¹¹¹In]In-PSMA-DB) was obtained in a similar manner to Example 5-1 except that PSMA-DB was used in place of PSMA-DA1.

As a result, a radiochemical yield was 61 to 90%, and a radiochemical purity was 95% or more.

Note that a compound in which PSMA-DB is coordinated to non-radioactive In can be manufactured, for example, by the following method.

To a H₂O/MeCN/TFA (49.95/49.95/0.1, 300 µL) solution of PSMA-DB (1 equivalent), indium (III) chloride nonhydrate (10 equivalent) was added. The mixture was stirred at room temperature for 18 hours, and then the solution was purified by reverse phase HPLC.

Purification conditions: Cosmosil 5C₁₈-AR-II column (4.6 × 150 mm), mobile phase: MeCN/H₂O/TFA [5/95/0.1 (0 to 10 min), 5/95/0.1 (10 min) to 35/65/0.1 (40 min)], flow rate: 1 mL/min.

### (3) ⁹⁰Y label (Comparative Example 2-2)

A desired radioactive labeled compound ([⁹⁰Y]Y-PSMA-DB) was obtained in a similar manner to Example 5-2 except that PSMA-DB was used in place of PSMA-DA1. As a result, a radiochemical yield was 49 to 79%, and a radiochemical purity was 95% or more.

### (4) ²²⁵Ac label (Comparative Example 2-3)

A desired radioactive labeled compound ([²²⁵Ac]Ac-PSMA-DB) was obtained in a similar manner to Example 5-3 except that PSMA-DB was used in place of PSMA-DA1.

As a result, a radiochemical yield was 48 to 49%, and a radiochemical purity was 83 to 87%.

### <Evaluation of stability in plasma>

A saline (20 µL) solution of [¹¹¹In]In-PSMA-DA1 (370 kBq) or [⁹⁰Y]Y-PSMA-DA1 (3.7 MBq) was added to mouse plasma (200 µL), and the mixture was allowed to stand at 37°C for 24 hours (n = 3). Thereafter, MeCN (200 µL) was added thereto, and the mixture was centrifuged at 10,000 × g for five minutes. The supernatant was filtered, and the filtrate was analyzed by reverse phase HPLC under the following conditions.

(Analysis conditions: Cosmosil 5C₁₈-PAQ column (4.6 × 250 mm), mobile phase: MeCN/H₂O/TFA [20/80/0.1 (0 min) to 50/50/0.1 (30 min)], flow rate: 1 mL/min.

As a result, 95% or more of each of the labeled compounds was stably present in mouse plasma even after being allowed to stand at 37°C for 24 hours.

### <Evaluation of binding using cultured cells>

LNCaP cells (PSMA positive, human prostate cancer) and PC-3 cells (PSMA negative, human prostate cancer) were used. These cells were purchased from American Type Culture Collection and DS Biomedical, respectively. The LNCaP cells and the PC-3 cells were each cultured in RPMI 1640 containing antibiotics (penicillin and streptomycin) and 10% inactivated fetal bovine serum, manufactured by Nacalai Tesque, Inc. at 37°C under 5% CO₂.

The LNCaP cells and the PC-3 cells were each seeded in a 12 well plate at 4.0 × 10⁵ cells/well, and allowed to stand at 37°C under 5% CO₂ for 48 hours.

The culture medium was removed, and an assay medium (0.5% FBS-containing RPMI 1640 medium) solution (1 mL) containing [¹¹¹In]In-PSMA-DA1 or [¹¹¹In]In-PSMA-DB (37 kBq) was added to the residue. Thereafter, the plate was allowed to stand at 37°C under 5% CO₂ for one hour.

In an inhibition assay, the culture medium was removed, and then an assay medium (1 mL) solution containing [¹¹¹In]In-PSMA-DA1 or [¹¹¹In]In-PSMA-DB (37 kBq) and 2-(phosphonomethyl) pentanedioic acid (2-PMPA) (final concentration: 100 µM) was added to the residue. Thereafter, the plate was allowed to stand at 37°C under 5% CO₂ for one hour.

The assay medium was removed, and then each well was washed with an assay medium (1 mL) containing neither a radioactive labeled compound nor 2-PMPA, and the cells were lysed with a 1 N sodium hydroxide aqueous solution (200 µL × 2).

The radioactivity of each of the assay medium and the cell lysate was measured with a gamma counter. Separately from this, the total protein concentration in the cell lysate was calculated using a BCA Protein Assay Kit manufactured by Thermo Fisher Scientific. A value (%ID/mg protein) obtained by dividing the percentage (%ID) of the amount of radioactivity of a sample to the amount of added radioactivity by the total protein mass was calculated for each sample.

Data was expressed as mean ± standard deviation. A significant difference test was performed using Student's t-test, and a result of p < 0.05 was defined to have a significant difference.

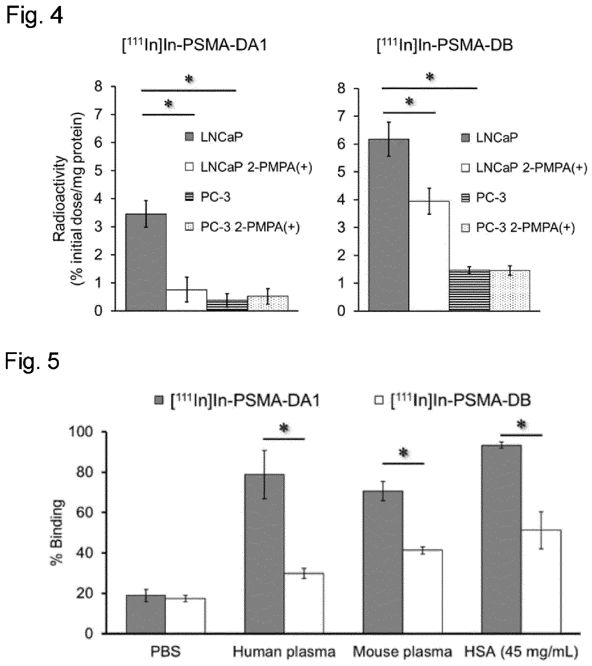

Results of evaluation of binding to cultured cells are illustrated in Fig. 4. The higher a value, the higher the abundance of the radioactive labeled compound, indicating that the amount of the compound accumulated is large.

[¹¹¹In]In-PSMA-DA1 and [¹¹¹In]In-PSMA-DB exhibited a higher binding property to the LNCaP cells than the PC-3 cells, and the binding was significantly reduced by addition of an excess amount of PSMA inhibitor (2-PMPA). These results indicate that [¹¹¹In]In-PSMA-DA1 and [¹¹¹In]In-PSMA-DB specifically bind to PSMA highly expressing cells.

### <Evaluation of binding to albumin>

A PBS solution (37 kBq, 50 µL) of [¹¹¹In]In-PSMA-DA1 or [¹¹¹In]In-PSMA-DB was added to each of 200 µL of PBS, 200 µL of a PBS solution (45 mg/mL) of mouse plasma, 200 µL of a PBS solution (45 mg/mL) of human plasma, and 200 µL of a PBS solution (45 mg/mL) of human serum albumin (HSA), and the mixture was allowed to stand at 37°C for 10 minutes. Thereafter, the reaction liquid was added to a spin column (Sephadex G-100 manufactured by Cytiva), and centrifuged at 1500 × g at 4°C for two minutes. After the separation, the radioactivity of each of the column and the eluate was measured with a gamma counter.

Data was expressed as mean ± standard deviation. A significant difference test was performed using Student's t-test, and a result of p < 0.05 was defined to have a significant difference.

Results of evaluation of binding to albumin are illustrated in Fig. 5. The higher a value, the higher binding property to albumin.

When a compound to be evaluated binds to albumin to form a complex, the complex passes through the column due to an increase in molecular size, but when the compound does not bind to albumin, the compound is retained in a gel in the column.

When [¹¹¹In]In-PSMA-DA1 and [¹¹¹In]In-PSMA-DB were allowed to stand in PBS and then applied to the column, no significant radioactivity was observed in the eluate. Meanwhile, when [¹¹¹In]In-PSMA-DA1 and [¹¹¹In]In-PSMA-DB were allowed to stand in each of a mouse plasma solution, a human plasma solution, and an HSA solution, the radioactivity in the eluate of [¹¹¹In]In-PSMA-DA1 was significantly higher than that of [¹¹¹In]In-PSMA-DB, indicating that [¹¹¹In]In-PSMA-DA1 binds to plasma albumin.

### <Evaluation of in vivo radioactivity distribution using LNCaP or PC-3 tumor transplanted mice>

Animal experiments were performed in accordance with the guidelines of Kyoto University Animal Experiment Committee. Male CB17/IcrJcl-Prkdc^{scid} mice were purchased from CLEA Japan, Inc. The animals were raised under 12 h/12 h day/night cycle conditions and freely fed with food and water. LNCaP cells (1.0 × 10⁷ cells/mouse) or PC-3 cells (1.0 × 10⁷ cells/mouse) were suspended in a mixed liquid (1 : 1,150 µL) of PBS and Matrigel manufactured by Corning Life Sciences Inc., and the suspension was subcutaneously transplanted to the right shoulder of each of the mice under isoflurane anesthesia. Thereafter, the mice were raised for 40 to 60 days.

A saline solution (185 kBq, 100 µL) of [¹¹¹In]In-PSMA-DA1 or [¹¹¹In]In-PSMA-DB was injected into each of LNCaP or PC-3 tumor transplanted mice via the tail vein thereof (n = 3 in each group). The mice were euthanized 1, 4, 24, 48, 96, and 192 hours after the injection. Thereafter, blood and organs were collected, and the mass and radioactivity of each of the organs were measured.

A result thereof is indicated as a value (%ID/g) obtained by dividing the percentage (%ID) of the amount of radioactivity with respect to the amount of injected radioactivity (injected dose) by the mass of blood or the mass of an organ (g). The higher a value of %ID/g, the higher the abundance of the radioactive labeled compound, indicating that the amount of the compound accumulated in the target organ is large.

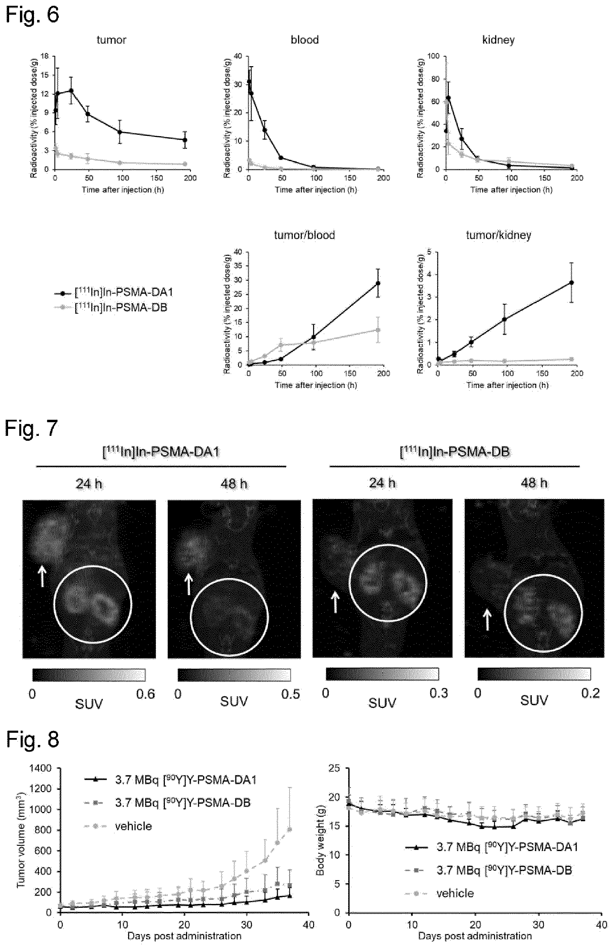

Results (mean ± standard deviation, n = 3 in each group) in the LNCaP tumor transplanted mice and [¹¹¹In]In-PSMA-DA1 are illustrated in Table 4 below and Fig. 6.

Results (mean ± standard deviation, n = 3 in each group) in the LNCaP tumor transplanted mice and [¹¹¹In]In-PSMA-DB are illustrated in Table 5 below and Fig. 6.

[¹¹¹In]In-PSMA-DA1 exhibited high accumulation in the LNCaP tumor (9.41 to 12.6%ID/g 1 to 24 hours after the injection). In addition, a retention property in blood was exhibited (14.0%ID/g 24 hours after the injection), and a tumor/kidney ratio exceeded 1 48 hours after the injection. Meanwhile, in [¹¹¹In]In-PSMA-DB, tumor accumulation lower than that of [¹¹¹In]In-PSMA-DA1 was observed, and a lower tumor/kidney ratio was exhibited at any time point.

These results clarify that [¹¹¹In]In-PSMA-DA1 has a high accumulation property in the PSMA-highly expressing tumor, and has better in vivo distribution than [¹¹¹In]In-PSMA-DB.

**[Table 4]**

| | 1 hour | 4 hours | 24 hours | 48 hours | 96 hours | 192 hours |
|---|---|---|---|---|---|---|
| Blood | 31.1±3.99 | 26.9±9.56 | 14.0±3.27 | 4.18±0.56 | 0.76±0.58 | 0.16±0.03 |
| Spleen | 8.56±2.90 | 9.52±4.28 | 6.01±2.33 | 4.28±0.79 | 2.42±1.54 | 2.69±0.83 |
| Pancreas | 3.34±0.40 | 2.48±1.03 | 2.12±0.49 | 1.06±0.12 | 0.64±0.24 | 0.46±0.08 |
| Stomach [%ID] | 0.52±0.02 | 0.51±0.05 | 0.40±0.09 | 0.25±0.05 | 0.08±0.03 | 0.05±0.01 |
| Intestinal | 2.63±0.42 | 3.17±0.73 | 1.53±0.44 | 0.96±0.25 | 0.75±0.44 | 0.12±0.03 |
| Kidney | 34.3±7.79 | 63.5±13.9 | 27.1±9.25 | 9.04±2.70 | 3.51±2.54 | 1.33±0.42 |
| Liver | 4.95±0.27 | 4.20±1.51 | 2.64±0.38 | 1.59±0.14 | 1.10±0.27 | 0.69±0.12 |
| Heart | 7.93±2.10 | 7.10±1.82 | 4.26±0.89 | 1.66±0.23 | 0.75±0.33 | 0.41±0.08 |
| Lung | 16.2±3.86 | 17.9±6.62 | 10.5±2.58 | 4.17±0.26 | 1.55±0.86 | 0.75±0.22 |
| Brain | 0.56±0.06 | 0.52±0.15 | 0.36±0.11 | 0.16±0.01 | 0.07±0.03 | 0.05±0.01 |
| Muscle | 2.02±0.17 | 2.15±1.09 | 1.38±0.39 | 0.54±0.10 | 0.23±0.09 | 0.15±0.05 |
| LNCaP tumor | 9.41±2.30 | 12.1±4.04 | 12.6±2.15 | 8.82±1.26 | 5.98±1.87 | 4.69±1.32 |

**[Table 5]**

| | 1 hour | 4 hours | 24 hours | 48 hours | 96 hours | 192 hours |
|---|---|---|---|---|---|---|
| Blood | 3.33±0.21 | 2.00±0.29 | 0.68±0.17 | 0.27±0.16 | 0.14±0.04 | 0.07±0.03 |
| Spleen | 3.05±0.53 | 2.39±0.05 | 3.37±0.75 | 2.63±0.83 | 2.82±0.87 | 3.46±0.32 |
| Pancreas | 0.74±0.25 | 0.54±0.22 | 0.71±0.10 | 0.71±0.17 | 0.85±0.39 | 1.04±0.18 |
| Stomach [%ID] | 0.10±0.04 | 0.13±0.03 | 0.16±0.05 | 0.07±0.02 | 0.10±0.03 | 0.05±0.00 |
| Intestinal | 0.59±0.14 | 1.05±0.10 | 1.00±0.44 | 0.79±0.21 | 0.41±0.17 | 0.13±0.02 |
| Kidney | 59.2±35.2 | 22.7±9.69 | 13.2±2.41 | 8.58±2.02 | 7.04±3.28 | 3.47±1.24 |
| Liver | 0.91±0.20 | 0.95±0.05 | 1.55±0.32 | 1.28±0.16 | 1.59±0.57 | 1.20±0.09 |
| Heart | 0.87±0.33 | 0.60±0.08 | 0.43±0.07 | 0.32±0.05 | 0.40±0.12 | 0.33±0.01 |
| Lung | 2.24±0.60 | 1.56±0.07 | 0.97±0.15 | 0.66±0.24 | 0.70±0.15 | 0.67±0.05 |
| Brain | 0.06±0.01 | 0.05±0.00 | 0.07±0.02 | 0.03±0.02 | 0.09±0.05 | 0.04±0.00 |
| Muscle | 0.23±0.68 | 0.21±0.04 | 0.21±0.07 | 0.15±0.06 | 0.14±0.08 | 0.14±0.09 |
| LNCaP tumor | 3.36±0.04 | 2.50±0.50 | 2.09±0.39 | 1.69±0.77 | 1.05±0.03 | 0.83±0.11 |

Results (mean ± standard deviation, n = 3 in each group) in the PC-3 tumor transplanted mice and [¹¹¹In]In-PSMA-DA1 are illustrated in Table 6 below.

Results (mean ± standard deviation, n = 3 in each group) in the PC-3 tumor transplanted mice and [¹¹¹In]In-PSMA-DB are illustrated in Table 7 below.

Accumulation of each of [¹¹¹In]In-PSMA-DA1 and [¹¹¹In]In-PSMA-DB in the PC-3 tumor is lower than accumulation thereof in the LNCaP tumor at the same time point, indicating that these radioactive labeled compounds are selectively accumulated in the PSMA positive tumor.

**[Table 6]**

| | 24 hours |
|---|---|
| Blood | 5.73±0.67 |
| Spleen | 2.52±0.12 |
| Pancreas | 1.37±0.04 |
| Stomach [%ID] | 0.27±0.08 |
| Intestinal | 1.37±0.22 |
| Kidney | 9.56±1.55 |
| Liver | 1.60±0.12 |
| Heart | 1.90±0.22 |
| Lung | 4.74±0.67 |
| Brain | 0.17±0.04 |
| Muscle | 0.59±0.02 |
| PC-3 tumor | 2.69±0.30 |

**[Table 7]**

| | 1 hour |
|---|---|
| Blood | 1.92±0.27 |
| Spleen | 2.57±0.39 |
| Pancreas | 0.44±0.06 |
| Stomach [%ID] | 0.12±0.04 |
| Intestinal | 0.48±0.04 |
| Kidney | 49.5±12.6 |
| Liver | 0.62±0.03 |
| Heart | 0.49±0.05 |
| Lung | 1.36±0.23 |
| Brain | 0.06±0.00 |
| Muscle | 0.21±0.08 |
| PC-3 tumor | 0.61±0.06 |

### <SPECT/CT using LNCaP tumor transplanted mice>

A saline solution (1.9 to 3.0 MBq, 150 µL) of [¹¹¹In]In-PSMA-DA1 or [¹¹¹In]In-PSMA-DB was injected into each of the LNCaP tumor transplanted mice generated by the above-described method via the tail vein thereof. SPECT/CT was performed with an FX3300 pre-clinical imaging system manufactured by Gamma Medica-Ideas 24 and 48 hours after the injection. Imaging was performed under isoflurane anesthesia using a pinhole collimator with a diameter of 1.0 mm at a rotation radius of 35 mm, a scan time of 70 seconds, and the number of times of imaging of 32. After SPECT, CT (tube voltage: 60 kV, tube current: 350 µA) was performed. Image reconstruction by a three-dimensional ordered subset expectation maximization method (8 subsets, 5 iterations) was performed on projection data of SPECT.

Results of SPECT/CT are illustrated in Fig. 7. In Fig. 7, a portion indicated by an arrow is a position where the tumor is present, and a portion indicated by a circle is a position where the kidney is present. The higher the SUV, the amount of radioactivity accumulated is large.

In SPECT/CT imaging using [¹¹¹In]In-PSMA-DA1, significant radioactivity accumulation was observed in the LNCaP tumor (arrow in Fig. 7) 24 and 48 hours after the injection. High radioactivity accumulation was also observed in the kidney (circle in Fig. 7), but a radioactivity signal of the kidney was almost the same as that of the tumor 48 hours after the injection. Meanwhile, in SPECT/CT imaging using [¹¹¹In]In-PSMA-DB, radioactivity accumulation was also observed in the LNCaP tumor (arrow in Fig. 7), but the accumulation was lower than that in the kidney (circle in Fig. 7).

These results indicate that [¹¹¹In]In-PSMA-DA1 can clearly delineate the PSMA-highly expressing tumor by SPECT, and is superior to [¹¹¹In]In-PSMA-DB.

### <Evaluation of suppression of tumor growth by ⁹⁰Y-labeled compound>

A saline solution (100 µL) of [⁹⁰Y]Y-PSMA-DA1 (3.7 MBq) or [⁹⁰Y]Y-PSMA-DB (3.7 MBq) obtained by the above-described method was injected into each of the LNCaP tumor transplanted mice via the tail vein thereof (n = 7). As a control group, 100 µL of saline was injected into each of the LNCaP tumor transplanted mice via the tail vein thereof (n = 7).

After the injection of the ⁹⁰Y-labeled compound, a tumor volume and a body weight were measured three times per week. The tumor volume was calculated based on a calculation formula "(tumor volume) = [(long side) × (short side)²/2]".

The tumor volumes on the start day of injection of the ⁹⁰Y-labeled compound were 63.1 ± 8.7, 66.1 ± 30.7, and 66.7 ± 25.7 mm³ in the [⁹⁰Y]Y-PSMA-DA1 injection group, the [⁹⁰Y]Y-PSMA-DB injection group, and the saline injection group, respectively.

### Results of the present evaluation are illustrated in Fig. 8.

When [⁹⁰Y]Y-PSMA-DA1 and [⁹⁰Y]Y-PSMA-DB were injected into the LNCaP tumor transplanted mice, a significant difference in tumor volume was observed 9 days and 33 days or more after the injection, respectively, as compared with the saline injection group. A tendency to suppress tumor growth was observed for [⁹⁰Y]Y-PSMA-DA1 as compared with [⁹⁰Y]Y-PSMA-DB. The body weight of each of the mice was slightly reduced by injection of [⁹⁰Y]Y-PSMA-DA1, but thereafter, the body weight was recovered to a value equivalent to that in the saline injection group.

### <Evaluation of suppression of tumor growth by ²²⁵Ac-labeled compound>

[²²⁵Ac]Ac-PSMA-DA1 (20 kBq) or [²²⁵Ac]Ac-PSMA-DB (20 kBq) obtained by the above-described method was dissolved in a 5% ethanol-containing acetate buffer (158 mM, pH 6.5, 100 µL), and the solution was injected into each of the LNCaP tumor transplanted mice via the tail vein thereof (n = 6 or 5). As a control group, 100 µL of 5% ethanol-containing acetate buffer (158 mM, pH 6.5) was injected into each of the LNCaP tumor transplanted mice via the tail vein thereof (n = 4).

After the injection of the ²²⁵Ac-labeled compound, a tumor volume and a body weight were measured three times per week. The tumor volumes on the start day of injection of the ²²⁵Ac-labeled compound were 75.3 ± 26.0, 80.6 ± 20.8, and 91.1 ± 15.9 mm³ in the [²²⁵Ac]Ac-PSMA-DA1 injection group, the [²²⁵Ac]Ac-PSMA-DB injection group, and the 5% ethanol-containing acetate buffer injection group, respectively.

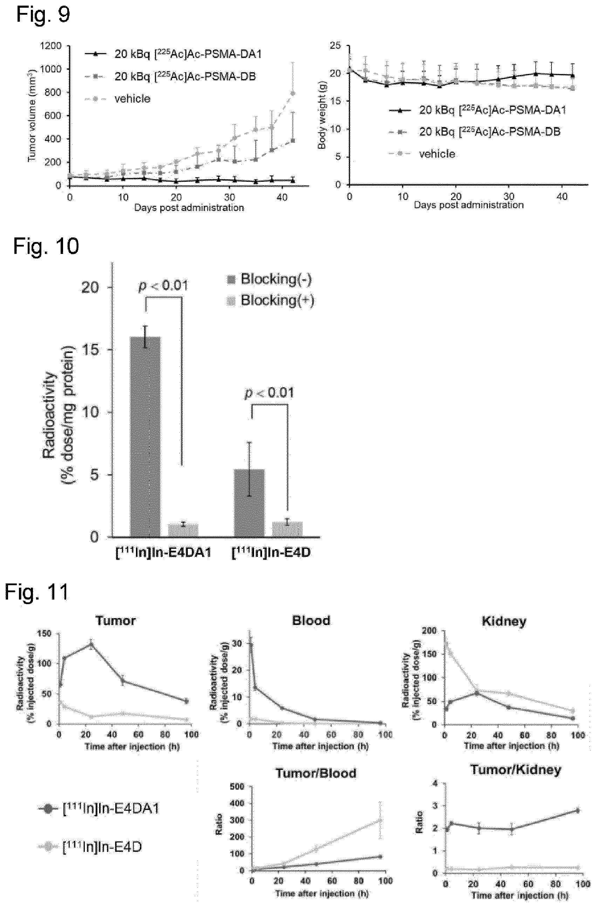

### Results of the present evaluation are illustrated in Fig. 9.

When [²²⁵Ac]Ac-PSMA-DA1 and [²²⁵Ac]Ac-PSMA-DB were injected into the LNCaP tumor transplanted mice, tumor growth was significantly suppressed as compared with the saline injection group. In particular, in the [²²⁵Ac]Ac-PSMA-DA1 injection group, the tumor hardly grew, and continuous growth suppression was observed up to six weeks after the injection.

A decrease in body weight, which is considered to be affected by the injection of [²²⁵Ac]Ac-PSMA-DA1, was transient. A decrease in body weight, which is considered to be affected by the injection of [²²⁵Ac]Ac-PSMA-DB, was temporarily accelerated, but there was no sign of recovery thereafter.

### Example 6 and Comparative Example 3

In the present Examples, two compounds (E4DA1 and E4D) each targeting the GLP-1 receptor as a target molecule were synthesized. Subsequently, a radioactive labeled compound in which an ¹¹¹In ion was coordinated as a radioactive metal to each of these compounds was obtained. An outline of a reaction path is illustrated below as reaction paths (VII-1) and (VII-2).

E4DA1 used in Example 6 contains a chelating moiety, a target molecule binding moiety, and an albumin binding moiety in the structure thereof. E4D used in Comparative Example 3 contains a chelating moiety and a target molecule binding moiety, but does not contain an albumin binding moiety in the structure thereof.

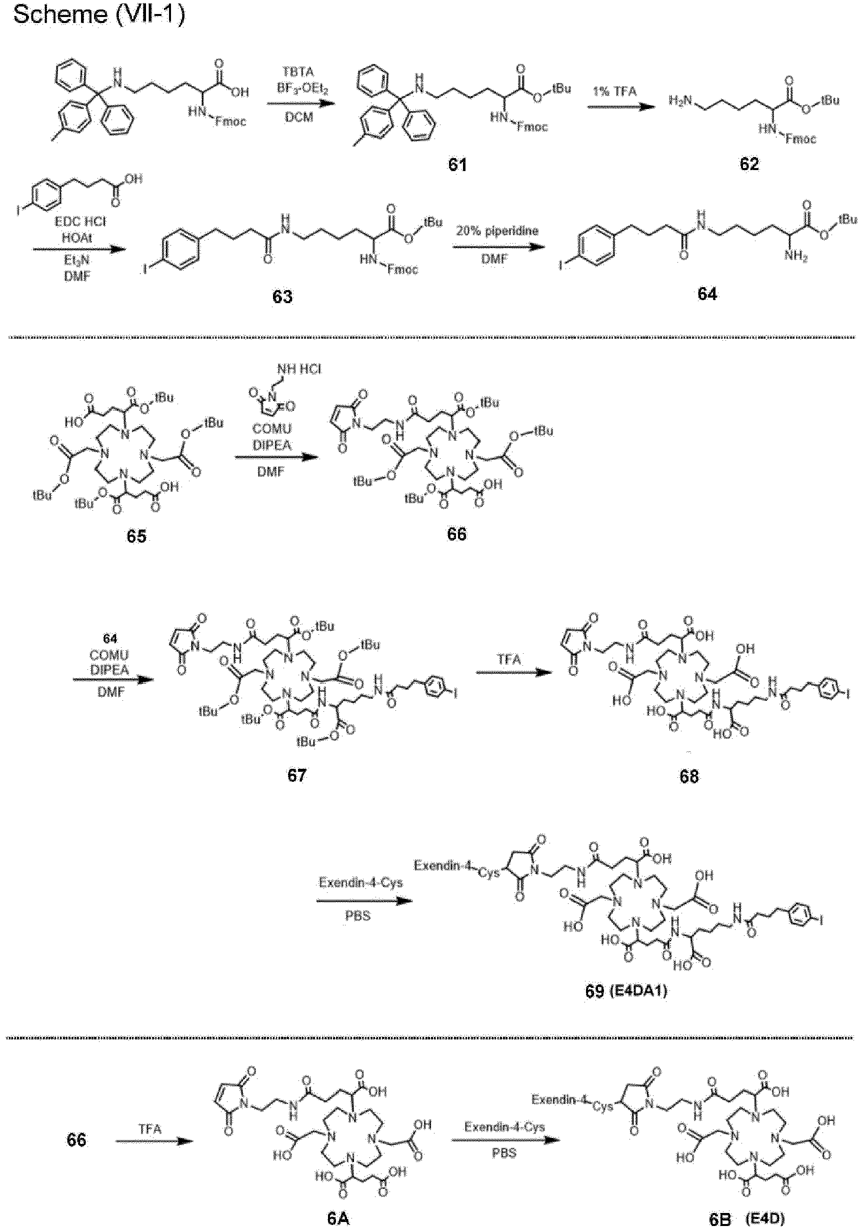

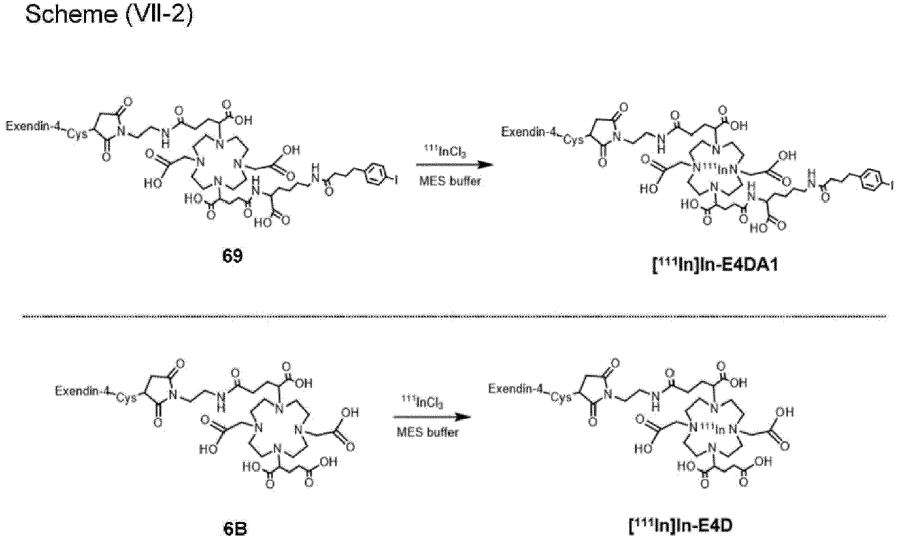

### <Synthesis of compound 61>

Fmoc-Lys (Mtt)-OH (200 mg, 0.32 mmol) was dissolved in dichloromethane (5 mL), and tert-butyl trichloroacetimidate (140 mg, 0.64 mmol) was slowly added thereto. BF₃•OEt₂ (10 µL) was further added thereto, and the mixture was stirred at room temperature for 24 hours. Thereafter, the solvent was distilled off under reduced pressure. Hexane was added to the residue, and the mixture was filtered. Thereafter, H₂O was added to the filtrate, and the mixture was separated. The organic layer was distilled off under reduced pressure. Thereafter, the residue was purified by medium-pressure column chromatography (hexane/ethyl acetate). An obtained amount, NMR spectrum, and MS were as follows.

Obtained amount: 47.3 mg (22%).

¹H-NMR: (400MHz, CDCl₃)δ7.90(d, 2H), 7.55(d, 2H), 7.30(t, 17H), 7.09(s, 2H), 4.70(d, 2H), 4.46(t, 2H), 4.16(s, 1H), 2.53(t, 2H), 2.19(s, 3H), 1.88(t, 2H), 1.42(s, 9H), 1.39(t, 2H), 1.25(t, 2H). ¹³C-NMR: (100MHz, CDCl₃)δ171.5, 155.9, 145.0(2C), 143.6(2C), 142.6(2C), 142.0, 135.9, 129.2(6C), 127.0(4C), 126.7(2C), 126.2(2C), 125.2(2C), 120.5(2C), 82.1, 78.3, 67.3, 58.6, 47.0, 44.3, 30.8(2C), 28.7(3C), 25.0, 21.3.

MS(ESI): m/z681.4[M+H]⁺.

### <Synthesis of compound 62>

Compound 61 (100 mg, 0.147 mmol) was added to a mixed solution of trifluoroacetic acid (TFA)/triisopropylsilane/dichloromethane (1/5/94, 5 mL), and the mixture was stirred at room temperature. 48 hours later, the solvent was distilled off under reduced pressure. Thereafter, the residue was purified by medium-pressure column chromatography (chloroform/methanol). An obtained amount, NMR spectrum, and MS were as follows.

Obtained amount: 50.3 mg (81%).

¹H-NMR: (400MHz, CDCl₃)δ7.90(d, 2H), 7.55(d, 2H), 7.38(t, 3H), 4.51(t, 1H), 4.46(t, 2H), 2.69(t, 2H), 1.88(t, 2H), 1.53(t, 2H), 1.50(d, 2H), 1.42(s, 9H), 1.25(t, 2H). ¹³C-NMR: (100MHz, CDCl₃)δ171.5, 155.9, 143.6(2C), 142.6(2C), 126.7(2C), 126.2(2C), 125.2(2C), 120.5(2C), 82.1, 67.3, 42.0, 30.8, 29.0, 28.7(3C), 22.7.

MS(ESI): m/z425.3[M+H]⁺.

### <Synthesis of compound 63>

4-(4-Iodophenyl) butyric acid (42.5 mg, 0.1 mmol) was dissolved in N,N-dimethylformamide (DMF), 1-ethyl-3-(3-dimethylaminopropyl)-carbodiimide (EDC) hydrochloride (38.3 mg, 0.2 mmol) and 1-hydroxy-7-azabenzotriazole (HOAt) (27.2 mg, 0.2 mmol) were added thereto, and the mixture was stirred at 0 °C. 15 minutes later, compound 62 (29.0 mg, 0.1 mmol) dissolved in DMF (5 mL) was added thereto, and the mixture was stirred at 0°C. 10 hours later, DMF was distilled off under reduced pressure. Thereafter, the residue was purified by medium-pressure column chromatography (chloroform/methanol). An obtained amount, NMR spectrum, and MS were as follows.

Obtained amount: 41.7 mg (60%).

¹H-NMR: (400MHz, CDCl₃)δ7.90(d, 2H), 7.70(s, 1H), 7.57(d, 2H), 7.55(d, 2H), 7.38(t, 3H), 7.28(t, 2H), 6.97(d, 2H), 4.70(d, 2H), 4.51(t, 1H), 4.46(t, 2H), 3.00(t, 2H), 2.63(t, 2H), 2.34(t, 2H), 1.88(t, 2H), 1.70(t, 2H), 1.53(t, 2H), 1.42(s, 9H), 1.25(t, 2H). ¹³C-NMR: (100MHz, CDCl₃)δ172.6, 171.5, 155.9, 143.6(2C), 142.6(2C), 140.9, 137.7(2C), 129.7(2C), 126.7(2C), 126.2(2C), 125.2(2C), 120.5(2C), 91.6, 82.1, 67.3, 58.6, 47.0, 42.0, 39.2, 36.1, 34.7, 30.8, 29.7, 28.7(3C), 27.4, 22.7.

MS(ESI): m/z697.3[M+H]⁺.

### <Synthesis of compound 64>

Compound 63 (34.8 mg, 0.050 mmol) was dissolved in DMF (5 mL), 20% piperidine (500 µL) was added thereto, and the mixture was stirred at room temperature. Two hours later, the mixture was separated with ethyl acetate/hexane = 1 : 5 (50 mL) and H₂O (50 mL). Thereafter, the organic layer was distilled off under reduced pressure. Thereafter, the residue was purified by medium-pressure column chromatography (chloroform/methanol). An obtained amount, NMR spectrum, and MS were as follows.

Obtained amount: 17.6 mg (74%).

¹H-NMR: (400MHz, CDCl₃)δ8, 76(s, 2H), 7.70(s, 1H), 7.57(d, 2H), 6.97(d, 2H), 3.36(t, 1H), 3.00(t, 2H), 2.63(t, 2H), 2.34(t, 2H), 1.88(t, 2H), 1.70(t, 2H), 1.53(t, 2H), 1.42(s, 9H), 1.25(t, 2H). ¹³C-NMR: (100MHz, CDCl₃)δ174.0, 172.6, 140.9, 137.7(2C), 129.7(2C), 91.6, 82.1, 52.9, 39.2, 36.1, 34.7, 31.3, 29.7, 28.7(3C), 27.4, 22.7.

MS(ESI): m/z475.2[M+H]⁺.

### <Synthesis of compound 65>

Compound 65 was synthesized from 1,4,7,10-tetraazacyclododecane in three steps in a similar manner to Example 5 (Chem Commun. 2008, 28, 3248-3250).

### <Synthesis of compound 66>

Compound 65 (50 mg, 0.065 mmol) and N-(2-aminoethyl)maleimide hydrochloride (11.4 mg, 0.065 mmol) were added to DMF (10 mL), and the mixture was stirred at room temperature. 10 minutes later, N-[1-(cyano-2-ethoxy-2-oxoethylideneaminooxy) dimethylamino (morpholino)] uronium hexafluorophosphate (COMU) (137.0 mg, 0.32 mmol) and N,N-diisopropylethylamine (DIPEA) (4.80 µL, 0.32 mmol) were added thereto, and the mixture was stirred at room temperature. 36 hours later, the mixture was purified by reverse phase HPLC under the following conditions. An obtained amount and MS were as follows.

Purification conditions: Cosmosil 5C₁₈-AR-II column (10 × 250 mm), mobile phase: MeCN/H₂O/TFA [10/90/0.1 (0 min) to 60/40/0.1 (30 min)], flow rate: 5 mL/min.

Obtained amount: 20.8 mg (36%).

MS(ESI): m/z895.4[M+H]⁺.

### <Synthesis of compound 67>

Compound 66 (20.0 mg, 0.022 mmol) and compound 68 (10.6 mg, 0.022 mmol) described later were added to DMF (5 mL), and the mixture was stirred at room temperature for 10 minutes. Thereafter, COMU (47.1 mg, 0.11 mmol) and DIPEA (1.65 µL, 0.11 mmol) were added thereto. The mixture was stirred at room temperature for four hours, and then purified by reverse phase HPLC under the following conditions. An obtained amount and MS were as follows.

Purification conditions: Cosmosil 5C₁₈-AR-II column (10 × 250 mm), mobile phase: MeCN/H₂O/TFA [10/90/0.1 (0 min) to 60/40/0.1 (30 min)], flow rate: 5 mL/min.

Obtained amount: 5.8 mg (20%).

MS(ESI): m/z1351.4[M+H]⁺.

### <Synthesis of compound 68>

TFA (2 mL) was added to compound 67 (5.75 mg, 4.2 µmol). The mixture was stirred at room temperature for one hour, and then purified by reverse phase HPLC under the following conditions. An obtained amount and MS were as follows.

Purification conditions: Cosmosil 5C₁₈-AR-II column (10 × 250 mm), mobile phase: MeCN/H₂O/TFA [10/90/0.1 (0 min) to 60/40/0.1 (30 min)], flow rate: 5 mL/min.

Obtained amount: 4.3 mg (95%).

MS(ESI): m/z1071.1[M+H]⁺.

### <Synthesis of compound 69 (E4DA1)>

Compound 68 (4.27 mg, 3.99 µmol) and Exendin-4-Cys (17.1 mg, 3.99 µmol) were dissolved in phosphate buffered saline (PBS) (pH 7.5, 2 mL), and the solution was stirred at 3°C. 24 hours later, the mixture was purified by reverse phase HPLC under the following conditions [gradient of H₂O (0.1%TFA)/MeCN (0.1%TFA) = 90/10 to 40/60, 30 min]. An obtained amount and MS were as follows.

Purification conditions: Cosmosil 5C₁₈-AR-II column (10 × 250 mm), mobile phase: MeCN/H₂O/TFA [10/90/0.1 (0 min) to 60/40/0.1 (30 min)], flow rate: 5 mL/min.

Obtained amount: 3.8 mg (18%).

MS(ESI): m/z1787.5[M+3H]³⁺,1072.8[M+5H]⁵⁺.

### <Synthesis of compound 6A>

TFA (2 mL) was added to compound 66 (20 mg, 0.022 mmol), and the mixture was stirred at room temperature for two hours. Thereafter, the mixture was purified by reverse phase HPLC under the following conditions. An obtained amount and MS were as follows.

Purification conditions: Cosmosil 5C₁₈-AR-II column (10 × 250 mm), mobile phase: MeCN/H₂O/TFA [10/90/0.1 (0 min) to 60/40/0.1 (30 min)], flow rate: 5 mL/min.

Obtained amount: 13.2 mg (90%). MS(ESI)m/z671.2[M+H]⁺.

### <Synthesis of compound 6B (E4D)>

Compound 6A (2.4 mg, 3.58 µmol) and Exendin-4-Cys (15.3 mg, 3.58 µmol) were dissolved in PBS (pH 7.5, 2 mL), and the solution was stirred at 3°C. 24 hours later, the solution was purified by reverse phase HPLC under the following conditions. An obtained amount and MS were as follows.

Purification conditions: Cosmosil 5C₁₈-AR-II column (10 × 250 mm), mobile phase: MeCN/H₂O/TFA [10/90/0.1 (0 min) to 60/40/0.1 (30 min)], flow rate: 5 mL/min.

Obtained amount: 1.8 mg (10%).

MS(ESI): m/z827.4[M+6H]⁶⁺.

### <¹¹¹In label (Example 6 and Comparative Example 3)>

An [¹¹¹In]InCl₃ solution (74 kBq, 20 µL) was mixed with a 2-(N-morpholino) ethanesulfonic acid (MES) buffer (0.1 M, pH 5.6, 200 µL) containing 0.1% Tween 80, and the mixture was allowed to stand at room temperature for 10 minutes. A dimethyl sulfoxide (DMSO) solution (10 µM, 30 µL) of the precursor compound (compound 69 or compound 6B) was added to the reaction solution, and the mixture was allowed to stand at 90°C for 30 minutes. Thereafter, the reaction liquid was purified by reverse phase HPLC under the following conditions to obtain a desired radioactive labeled compound ([¹¹¹In]In-E4DA1 or [¹¹¹In]In-E4D).

Purification conditions: Cosmosil 5C₁₈-AR-II column (4.6 × 150 mm), mobile phase: MeCN/H₂O/TFA [10/90/0.1 (0 min) to 60/40/0.1 (40 min)], flow rate: 1 mL/min.

As a result, a radiochemical yield of [¹¹¹In]In-E4DA1 was 11.6%, and a radiochemical purity was 95% or more. In addition, a radiochemical yield of [¹¹¹In]In-E4D was 4.6%, and a radiochemical purity was 95% or more.

### <Evaluation of binding using cultured cells>

INS-1 cells (rat insulinoma) were used. The INS-1 cells were cultured in RPMI 1640 containing Na pyruvate (1 mM), β-mercaptoethanol (50 µM), HEPES, sodium hydrogen carbonate (1.5 g/L), antibiotics (penicillin and streptomycin), and 10% inactivated fetal bovine serum, manufactured by Nacalai Tesque, Inc. at 37°C under 5% CO₂.

The INS-1 cells (4.0 × 10⁵ cells/well) were seeded in a 12 well plate, and allowed to stand at 37°C under 5% CO₂ for 48 hours. The culture medium was removed. Thereafter, as an assay medium, an RPMI 1640 solution (1 mL) containing [¹¹¹In]In-E4DA1 (9.8 kBq) or an RPMI 1640 solution (1 mL) containing [¹¹¹In]In-E4D (9.8 kBq) was added to the residue.

In an inhibition assay, after the removal of the culture medium, as an assay medium, an RPMI 1640 solution (1 mL) containing [¹¹¹In]In-E4DA1 (9.8 kBq) and Exendin-4 Cys (final concentration 10 µM) or an RPMI 1640 solution (1 mL) containing [¹¹¹In]In-E4D (9.8 kBq) and Exendin-4 Cys (final concentration 10 µM) was added to the residue. Thereafter, the plate was allowed to stand at 37°C under 5% CO₂ for two hours.

The assay medium was removed. Thereafter, each well was washed with PBS (1 mL), and the cells were lysed with a 1 N sodium hydroxide aqueous solution (200 µL × 2).

The radioactivity of the cell lysate was measured with a gamma counter. Separately from this, the total protein concentration in the cell lysate was calculated by a BCA method in a similar manner to the above-described method.

A value (%ID/mg protein) obtained by dividing the percentage (%ID) of the amount of radioactivity of a sample to the amount of added radioactivity by the total protein mass was calculated for each sample.

Data was expressed as mean ± standard deviation. A significant difference test was performed using Student's t-test, and a result of p < 0.05 was defined to have a significant difference.

Results of evaluation of binding to cultured cells are illustrated in Fig. 10. The higher a value, the higher the abundance of the radioactive labeled compound, indicating that the amount of the compound accumulated is large.

[¹¹¹In]In-E4DA1 exhibited a binding property to the INS-1 cells (16% dose/mg protein), and the binding was significantly decreased by addition of an excessive amount of Exendin-4-Cys (1.0% dose/mg protein). Similarly, a binding property of [¹¹¹In]In-E4D to INS-1 cells (5.4% dose/mg protein) and a decrease in the binding amount by addition of Exendin-4-Cys (1.2% dose/mg protein) were observed.

These results indicate that [¹¹¹In]In-E4DA1 specifically binds to the GLP-1 receptor highly expressing cells, and the binding property to the GLP-1 receptor highly expressing cells was improved by introduction of the albumin binding moiety.

### <Evaluation of binding to albumin>

APBS solution of [¹¹¹In]In-E4DA1 (37 kBq, 50 µL) or a PBS solution of [¹¹¹In]In-E4D (37 kBq, 50 µL) was added to each of PBS and a human albumin (HSA) solution (45 mg/mL), and the mixture was allowed to stand at 37°C for 10 minutes.

Thereafter, the reaction liquid was added to a spin column (Sephadex G-100 manufactured by Cytiva), and centrifuged at 1500 × g at 4°C for two minutes. After the centrifugation, the radioactivity of each of the column and the eluate was measured with a gamma counter.

Data was expressed as mean ± standard deviation. A significant difference test was performed using Student's t-test, and a result of p < 0.05 was defined to have a significant difference.

### Results of evaluation of binding to albumin are illustrated in Table 8.

When [¹¹¹In]In-E4DA1 was allowed to stand in PBS or the HSA solution and then applied to the column, the radioactivity ratio (65.6%) of the eluate in the HSA solution was significantly higher than the radioactivity ratio (5.9%) of the eluate in PBS. In addition, the radioactivity ratio of the eluate of [¹¹¹In]In-E4DA1 in the HSA solution was significantly higher than that of [¹¹¹In]In-E4D (20.2%). These results indicate that [¹¹¹In]In-E4DA1 has a binding property to albumin, and the binding property to albumin is retained even when an albumin binding moiety is introduced together with a structure containing a peptide having a large molecular weight, such as Exendin-4.

**[Table 8]**

| % Albumin binding | PBS | HSA |
|---|---|---|
| Example 6 ([¹¹¹In]In-E4DA1) | 5.9±0.4 | 65.6±1.6 |
| Comparative Example 3 ([¹¹¹In]In-E4D) | 6.6±2.2 | 20.2±3.0 |

### <Evaluation of in vivo radioactivity distribution using INS-1 tumor transplanted mice>

Animal experiments were performed in accordance with the guidelines of Kyoto University Animal Experiment Committee. Male BALB/c-nu/nu nude mice were purchased from Japan SLC, Inc. The animals were raised under 12 h/12 h day/night cycle conditions and freely fed with food and water. INS-1 cells (5.0 × 10⁶ cells/mouse) were suspended in a mixed liquid (1 : 1,100 µL) of PBS and Matrigel manufactured by Corning Life Sciences Inc., and the suspension was subcutaneously transplanted to the right shoulder of each of the BALB/c-nu/nu nude mice under isoflurane anesthesia. The tumor transplanted mice were used when a tumor diameter reached 10 mm.

A saline solution (37 kBq, 100 µL) of [¹¹¹In]In-E4DA1 or a saline solution (30 kBq, 100 µL) of [¹¹¹In]In-E4D was injected into each of the INS-1 tumor transplanted mice via the tail vein thereof (n = 4). The mice were euthanized 1, 4, 24, 48, and 96 hours after the injection. Thereafter, blood and organs were collected, and the mass and radioactivity of each of the organs were measured.

A result thereof is indicated as a value (%ID/g) obtained by dividing the percentage (%ID) of the amount of radioactivity with respect to the amount of injected radioactivity (injected dose) by the mass of blood or the mass of an organ (g). The higher a value of %ID/g, the higher the abundance of the radioactive labeled compound, indicating that the amount of the compound accumulated in the target organ is large.

Results (mean ± standard deviation, n = 4 in each group) in the INS-1 tumor transplanted mice and [¹¹¹In]In-E4DA1 are illustrated in Table 9 below and Fig. 11.

Results (mean ± standard deviation, n = 4 in each group) in the INS-1 tumor transplanted mice and [¹¹¹In]In-E4D are illustrated in Table 10 below and Fig. 11.
[¹¹¹In]In-E4DA1 exhibited high accumulation in the INS-1 tumor (66.1 to 132%ID/g 1 to 24 hours after the injection). In addition, a retention property in blood was exhibited (6.01%ID/g 24 hours after the injection), and a tumor/kidney ratio exhibited a high value (1.96 to 2.80 1 to 96 hours after the injection). A similar experiment was performed for [¹¹¹In]In-E4D, and comparison was made for radioactivity accumulation in the tumor, blood, and kidney, a tumor/blood accumulation ratio, and a tumor/kidney accumulation ratio. At any time point, as compared with [¹¹¹In]In-E4D, low accumulation in the kidney was observed, and high accumulation in the tumor was exhibited in [¹¹¹In]In-E4DA1. These results clarify that [¹¹¹In]In-E4DA1 has a high accumulation property in the GLP-1 receptor-highly expressing tumor, and has better in vivo distribution than [¹¹¹In]In-E4D.

**[Table 9]**

| | 1 hour | 4 hours | 24 hours | 48 hours | 96 hours |
|---|---|---|---|---|---|
| Blood | 24.5±2.86 | 13.8±1.24 | 6.01±0.46 | 1.80±0.10 | 0.47±0.05 |
| Spleen | 4.77±0.25 | 3.71±0.51 | 3.24±0.16 | 2.61±0.16 | 1.81±0.13 |
| Pancreas | 11.83±3.40 | 22.9±2.80 | 25.0±2.63 | 18.1±1.48 | 10.4±1.03 |
| Stomach [%ID] | 0.88±0.16 | 0.74±0.08 | 0.78±0.21 | 0.42±0.04 | 0.22±0.05 |
| Intestinal | 3.34±0.47 | 2.77±0.33 | 2.33±0.42 | 1.49±0.13 | 0.70±0.10 |
| Kidney | 33.99±3.20 | 49.3±2.61 | 67.7±6.22 | 37.4±3.21 | 13.9±1.83 |
| Liver | 7.04±0.62 | 5.10±0.41 | 4.31±0.21 | 3.46±0.17 | 2.51±0.15 |
| Heart | 6.67±0.69 | 4.48±0.37 | 2.40±0.41 | 1.32±0.15 | 1.41±0.67 |
| Lung | 24.8±3.06 | 16.2±0.78 | 12.2±1.74 | 5.74±0.92 | 3.08±0.55 |
| Brain | 0.86±0.17 | 1.38±0.93 | 0.64±0.19 | 0.23±0.02 | 0.52±0.23 |
| Muscle | 1.62±0.24 | 1.60±0.44 | 0.61±0.17 | 0.42±0.10 | 0.25±0.06 |
| INS-1 tumor | 66.1±4.71 | 109±2.51 | 132±8.49 | 71.9±7.96 | 38.3±4.11 |

**[Table 10]**

| | 1 hour | 4 hours | 24 hours | 48 hours | 96 hours |
|---|---|---|---|---|---|
| Blood | 1.97±0.10 | 1.86±0.28 | 0.30±0.02 | 0.15±0.03 | 0.03±0.01 |
| Spleen | 0.76±0.08 | 2.29±0.40 | 0.93±0.07 | 0.81±0.07 | 0.58±0.04 |
| Pancreas | 11.3±1.28 | 12.4±1.12 | 6.40±0.75 | 7.30±0.23 | 3.35±0.12 |
| Stomach [%ID] | 0.44±0.09 | 0.68±0.13 | 0.21±0.02 | 0.22±0.02 | 0.12±0.01 |
| Intestinal | 0.74±0.03 | 1.00±0.24 | 0.63±0.05 | 0.69±0.10 | 0.32±0.03 |
| Kidney | 173±12.6 | 152±8,67 | 73.4±12.8 | 66.6±5.23 | 30.2±6.17 |
| Liver | 1.25±0.03 | 1.63±0.13 | 1.07±0.02 | 1.81±0.69 | 0.91±0.08 |
| Heart | 0.78±0.03 | 3.47±1.99 | 0.44±0.10 | 0.35±0.06 | 0.20±0.07 |
| Lung | 6,54±0.61 | 5.07±0.44 | 2.35±0.31 | 1.49±0.15 | 0.81±0.16 |
| Brain | 0.08±0.02 | 0.55±0.03 | 0.07±0.01 | 0.06±0.01 | 0.04±0.01 |
| Muscle | 0.14±0.03 | 2.48±0.80 | 0.15±0.06 | 0.16±0.04 | 0.07±0.01 |
| INS-1 tumor | 36.0±1.53 | 28.9±0.98 | 12.2±1.35 | 17.5±0.68 | 7.30±1.19 |

### <SPECT/CT using INS-1 tumor transplanted mice>

A saline solution (0.30 MBq, 100 µL) of [¹¹¹In]In-E4DA1 or a saline solution (1.2 MBq, 100 µL) of [¹¹¹In]In-E4D was injected into each of the INS-1 tumor transplanted mice generated by the above-described method via the tail vein thereof. SPECT/CT was performed with an FX3300 pre-clinical imaging system manufactured by Gamma Medica-Ideas six hours after the injection. Imaging was performed under isoflurane anesthesia using a pinhole collimator with a diameter of 1.0 mm at a rotation radius of 35 mm, a scan time of 70 seconds, and the number of times of imaging of 32. After SPECT, CT (tube voltage: 60 kV, tube current: 350 µA) was performed. Image reconstruction by a three-dimensional ordered subset expectation maximization method (8 subsets, 5 iterations) was performed on projection data of SPECT.

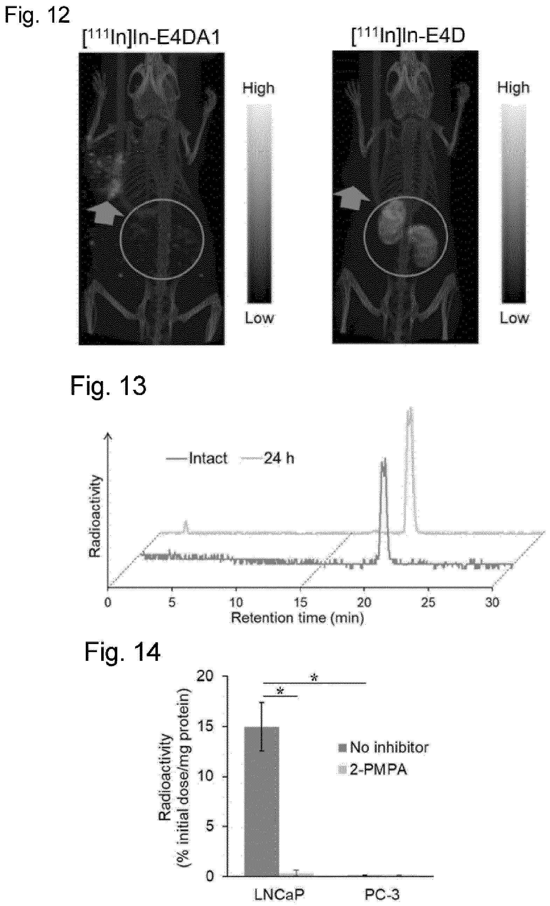

Results of SPECT/CT are illustrated in Fig. 12. In Fig. 12, a portion indicated by an arrow is a position where the tumor is present, and a portion indicated by a circle is a position where the kidney is present. The higher the SUV, the amount of radioactivity accumulated is large.

In SPECT/CT imaging using [¹¹¹In]In-E4DA1, high radioactivity accumulation was observed in the tumor, but a radioactivity signal in the kidney was hardly observed. Meanwhile, in [¹¹¹In]In-E4D, higher radioactivity accumulation was observed in the kidney than in the tumor. These results indicate that inclusion of an albumin binding moiety improves the in vivo distribution, and [¹¹¹In]In-E4DA1 can clearly delineate the GLP-1 receptor-highly expressing tumor.

### Example 7

In the present Example, a compound (PtDA) was synthesized which contained (((S)-5-amino-1-carboxypentyl)carbamoyl)-L-glutamic acid (a structure in which a is 2 and b is 4 in the above formula (C3)) targeting PSMA as a target molecule in the structure thereof as a target molecule binding moiety, and used the click reaction between an azide group and dibenzylcyclooctyne (DBCO) for binding between a chelating moiety and the target molecule binding moiety. Subsequently, a radioactive labeled compound in which an ¹¹¹In ion was coordinated as a radioactive metal to each of these compounds was obtained. An outline of a reaction path is illustrated below as reaction paths (VIII-1) to (VIII-4).

The compound used in Example 7 contains a chelating moiety, a target molecule binding moiety, and an albumin binding moiety in the structure thereof.

In addition, there are two reaction paths (see reaction path (VIII-4)) for coordination to an ¹¹¹In ion, and the same radioactive labeled compound is obtained in either path.

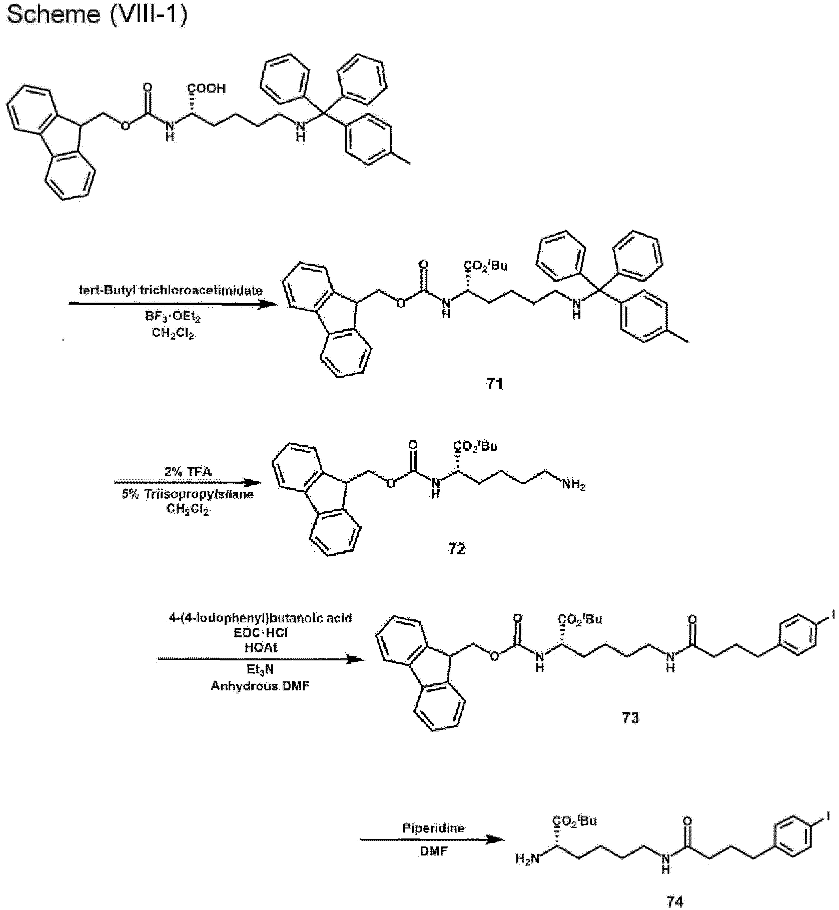

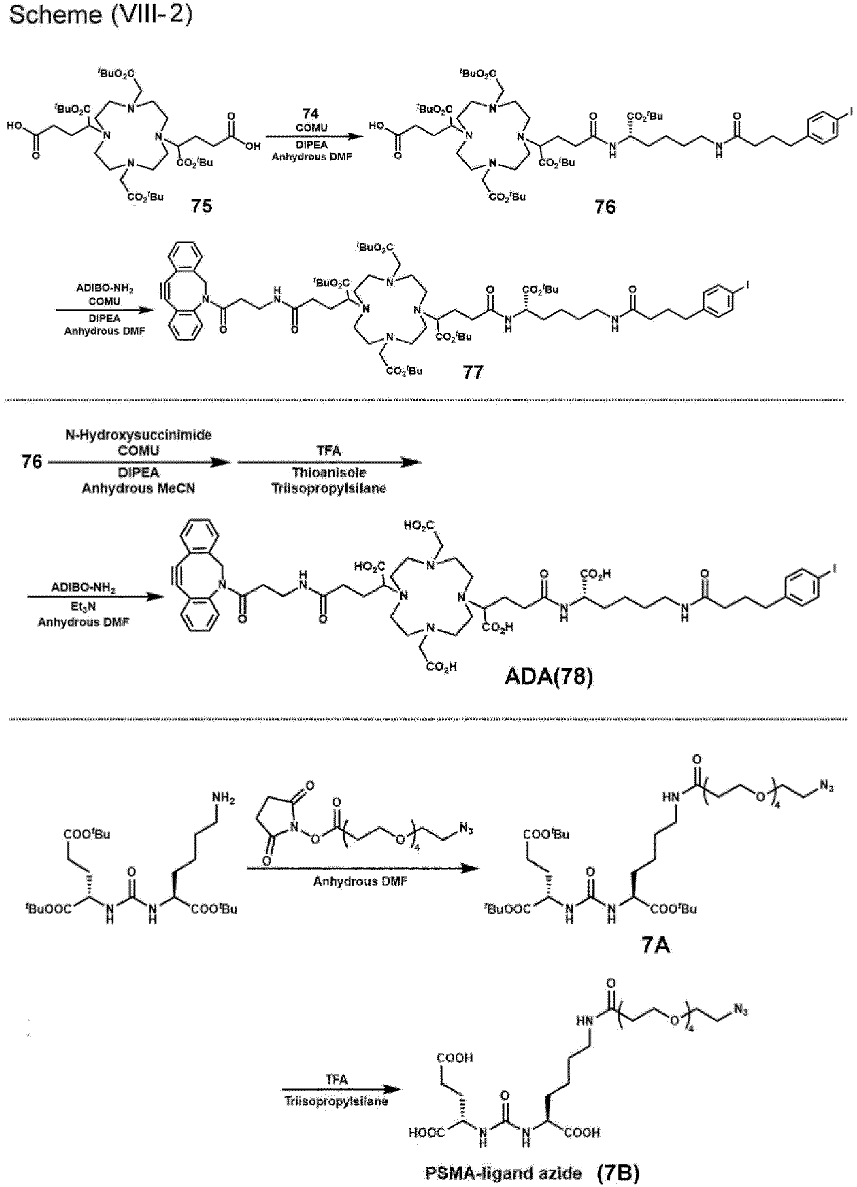

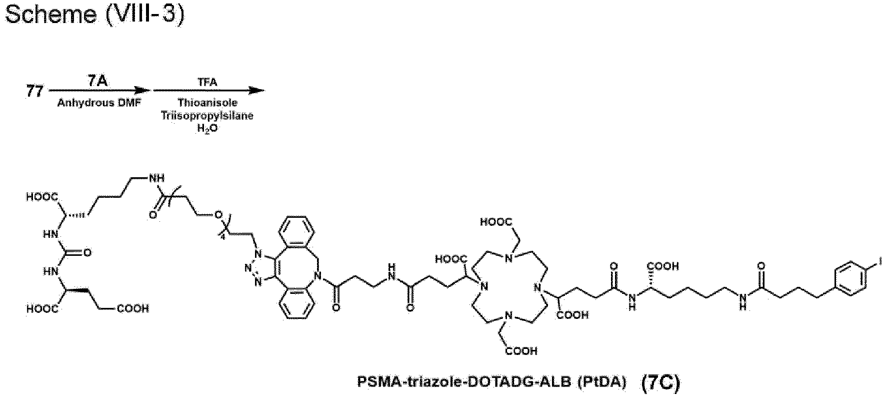

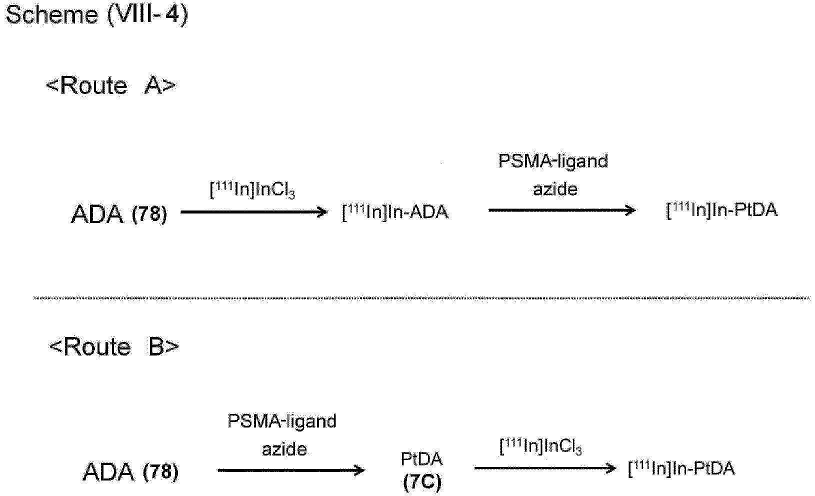

### <Synthesis of compound 71>

N²-[(9H-fluoren-9-ylmethoxy)carbonyl]-N⁶-[(4-methylphenyl)diphenylmethyl]-L-lysine (1000 mg, 1.6 mmol) was dissolved in dichloromethane (10 mL), and tert-butyl trichloroacetimidate (699.5 mg, 3.2 mmol) and BF₃•OEt₂ (25 µL) were added thereto. The reaction solution was stirred at room temperature for 42 hours. The solvent was removed. Thereafter, the residue was washed with H₂O (100 mL), and extracted with ethyl acetate/hexane (1/5, 100 mL × 2). The organic layer was dried over sodium sulfide and filtered. The filtrate was distilled off under reduced pressure, and then the residue was purified by medium-pressure column chromatography (ethyl acetate/hexane). An obtained amount, NMR spectrum, and MS were as follows.

Obtained amount: 569 mg (52%).

¹H-NMR(400MHz, CDCl₃)δ7.69(d, J=7.3Hz, 2H), 7.56(d, J=7.8Hz, 2H), 7.45(d, J=7.8Hz, 4H), 7.33(d, J=8.2Hz, 4H), 7.23(m, 6H), 7.13(m, 2H), 7.04(d, J=7.8Hz, 2H), 5.39(d, J=8.2Hz, 1H), 4.35(d, J=6.9Hz, 2H), 4.25(m, 1H), 4.18(t, J=6.9Hz, 1H), 2.26(s, 3H), 2.12(m, 2H), 1.77(m, 1H), 1.59(m, 1H), 1.49(m, 4H), 1.44(s, 9H). ¹³C-NMR(100MHz, CDCl₃)δ171.6, 155.7, 146.3(2C), 143.7(2C), 143.2, 141.1(2C), 135.4, 128.4-128.3(8C), 127.6-127.5(6C), 126.9(2C), 126.0(2C), 125.0(2C), 119.8(2C), 81.7, 70.5, 66.7, 60.2, 47.1, 43.2, 32.7, 30.4, 27.8(3C), 22.8, 20.7.

HRMS(ESI): m/z681.3677[M+H]⁺.

### <Synthesis of compound 72>

Compound 71 (569 mg, 0.84 mmol) was dissolved in a mixed liquid of trifluoroacetic acid (TFA) (100 µL), triisopropylsilane (250 µL), and dichloromethane (4.65 mL), and the solution was stirred at room temperature for six hours. The solvent was removed. Thereafter, the residue was purified by medium-pressure column chromatography (methanol/chloroform). An obtained amount, NMR spectrum, and MS were as follows.

Obtained amount: 355 mg (100%).

¹H-NMR(400MHz, CDCl₃)δ7.71(d, J=7.6Hz, 2H), 7.56(d, J=7.1Hz, 2H), 7.35(t, J=7.6Hz, 2H), 7.27(t, J=7.6Hz, 2H), 5.72(d, J=8.0Hz, 1H), 4.33(d, J=7.1Hz, 2H), 4.16(t, J=6.6Hz, 2H), 3.38(s, 2H), 1.77-1.56(m, 4H), 1.43(s, 9H), 1.27-1.13(m, 2H). ¹³C-NMR(100MHz, CDCl₃)δ171.5, 156.2, 143.6(2C), 141.1(2C), 127.6(2C), 127.0(2C), 125.0(2C), 119.8(2C), 82.3, 66.9, 54.1, 50.0, 46.9, 39.5, 31.1, 27.7(3C), 26.8, 22.1.

HRMS(ESI): m/z425.2437[M+H]⁺.

### <Synthesis of compound 73>

4-(4-Iodophenyl) butanoic acid (364 mg, 1.25 mmol) was dissolved in N,N-dimethylformamide (DMF) (3 mL), and 1-ethyl-3-(3-dimethylaminopropyl)-carbodiimide (EDC) hydrochloride (320 mg, 1.7 mmol) and 1-hydroxy-7-azabenzotriazole (HOAt) (228 mg, 1.7 mmol) were added thereto. The mixture was stirred at 0°C for 15 minutes. Thereafter, compound 72 (355 mg, 0.84 mmol) and triethylamine (169 mg, 1.7 mmol) were added thereto, and the mixture was stirred at 0°C. 15 hours later, the reaction solution was washed with H₂O (100 mL), and extracted with ethyl acetate/hexane (1/5, 100 mL × 2). The organic layer was dried over sodium sulfide and filtered. The filtrate was distilled off under reduced pressure, and then the residue was purified by medium-pressure column chromatography (ethyl acetate/hexane). An obtained amount, NMR spectrum, and MS were as follows.

Obtained amount: 226 mg (39%).

¹H-NMR(400MHz, CDCl₃)δ7.73(d, J=7.3Hz, 2H), 7.60-7.50(m, 6H), 7.37(t, J=7.3Hz, 2H), 7.28(t, J=7.3Hz, 2H), 6.83(d, J=8.2Hz, 2H), 4.38-4.27(m, 2H), 4.25-4.16(m, 2H), 3.21-3.14(m, 2H), 2.48(m, 2H), 2.08(t, J=7.3Hz, 2H), 1.91-1.84(m, 2H), 1.73-1.57(m, 2H), 1.47-1.29(m, 13H).¹³C-NMR(100MHz, CDCl₃)δ172.8, 171.5, 156.0, 143.5(2C), 141.0-140.9(3C), 137.1(2C), 130.3(2C), 127.5(2C), 126.9(2C), 124.9(2C), 119.8(2C), 90.8, 82.0, 66.8, 53.9, 46.9, 38.9, 35.4, 34.4, 32.1, 28.6, 27.9(3C), 26.7, 22.2.

HRMS(ESI): m/z697.2130[M+H]⁺.

### <Synthesis of compound 74>

To a DMF (4 mL) solution of compound 73, piperidine (1 mL) was added. The mixture was stirred at room temperature for two hours. Thereafter, the solution was washed with H₂O (100 mL), and extracted with ethyl acetate/hexane (1/5, 100 mL × 2). The organic layer was dried over sodium sulfide and filtered. The filtrate was distilled off under reduced pressure. Thereafter, the residue was purified by medium-pressure column chromatography (methanol/chloroform). An obtained amount, NMR spectrum, and MS were as follows.

Obtained amount: 133 mg (87%).

¹H-NMR(400MHz, CDCl₃)δ7.58(d, J=8.2Hz, 2H), 6.92(d, J=8.2Hz, 2H), 3.31-3.27(m, 1H), 3.23(m, 2H), 2.58(m, 2H), 2.13(m, 2H), 1.96-1.88(m, 2H), 1.74-1.65(m, 2H), 1.56-1.48(m, 2H), 1.44(s, 9H), 1.43-1.40(m, 2H).¹³C-NMR(100MHz, CDCl₃)δ175.2, 172.3, 141.1, 137.3(2C), 130.5(2C), 90.9, 80.9, 54.7, 39.1, 35.6, 34.6, 34.3, 29.2, 28.0(3C), 26.8, 22.9.

HRMS(ESI):m/z475.1453[M+H]⁺.

### <Synthesis of compound 75>

Compound 75 was synthesized from 1,4,7,10-tetraazacyclododecane in three steps in a similar manner to Example described above (Chem Commun. 2008, 28, 3248-3250).

### <Synthesis of compound 76>

N-[1-(cyano-2-ethoxy-2-oxoethylideneaminooxy)dimethylamino(morpholino)]uronium hexafluorophosphate (COMU) (176 mg, 0.41 mmol) was added to a DMF (2 mL) solution of compound 75 (317 mg, 0.41 mmol), and the mixture was stirred at 0°C for 15 minutes. N,N-diisopropylethylamine (DIPEA) (53 mg, 0.41 mmol) was added to the reaction liquid, and the mixture was further stirred at 0°C for 15 minutes. Thereafter, compound 74 (163 mg, 0.34 mmol) was added thereto. The mixture was stirred at room temperature for 12 hours, and then the solution was purified by reverse phase HPLC under the following conditions. An obtained amount and MS were as follows.

Purification conditions: Cosmosil 5C₁₈-AR-II column (20 × 250 mm), mobile phase: MeCN/H₂O/TFA [30/70/0.1 (0 min) to 90/10/0.1 (40 min)], flow rate: 5 mL/min.

Obtained amount: 229 mg (55%).

HRMS(ESI)m/z1229.6182[M+H]⁺.

### <Synthesis of compound 77>

COMU (147 mg, 0.34 mmol) was added to a DMF (0.6 mL) solution of compound 76 (106 mg, 0.086 mmol), and the mixture was stirred at 0°C for 15 minutes. DIPEA (89 mg, 0.69 mmol) was added to the reaction liquid, and the mixture was further stirred at 0°C for 15 minutes. Thereafter, dibenzocyclooctyne amine (ADIBO-NH2) (36 mg, 0.22 mmol) was added thereto. The mixture was stirred at room temperature for 12 hours, and then the solution was purified by reverse phase HPLC under the following conditions. An obtained amount and MS were as follows.

Purification conditions: Cosmosil 5C₁₈-AR-II column (20 × 250 mm), mobile phase: MeCN/H₂O/TFA [20/80/0.1 (0 min) to 90/10/0.1 (35 min)], flow rate: 5 mL/min.

Obtained amount: 51 mg (40%).

HRMS(ESI): m/z1487.7351[M+H]⁺.

### <Synthesis of compound 78 (ADA)>

COMU (70 mg, 0.16 mmol) was added to a MeCN (0.4 mL) solution of compound 76 (50 mg, 0.041 mmol), and the mixture was stirred at 0°C for 15 minutes. DIPEA (89 mg, 0.69 mmol) was added to the reaction liquid, and the mixture was further stirred at 0°C for 15 minutes. Thereafter, N-hydroxysuccinimide (19 mg, 0.16 mmol) was added thereto. The mixture was stirred at room temperature for 24 hours. Thereafter, the solution was washed with H₂O (100 mL), and extracted with ethyl acetate/hexane (1/5, 100 mL × 2). The organic layer was dried over sodium sulfide and filtered. The filtrate was distilled off under reduced pressure, and then half of the residue was dissolved in a mixed liquid of TFA/thioanisole/triisopropylsilane (95/3/2, 2 mL). The solution was stirred at room temperature for 11 hours. The solvent was removed. Thereafter, the residue was dissolved in DMF (0.4 mL) and triethylamine (10 µL), and ADIBO-NH₂ (6.2 mg, 0.023 mmol) was added thereto. The reaction liquid was stirred at room temperature for 24 hours, and then the solution was purified by reverse phase HPLC under the following conditions. An obtained amount and MS were as follows.

Purification conditions: Cosmosil 5C₁₈-AR-II column (4.6 × 150 mm), mobile phase: MeCN/H₂O/TFA [10/90/0.1 (0 min) to 90/10/0.1 (40 min)], flow rate: 1 mL/min.

Obtained amount: 6.7 mg (27%).

HRMS(ESI): m/z1207.4213[M+H]⁺.

### <Synthesis of compound 7A>

(S)-Di-tert-butyl 2-(3-((S)-6-amino-1-tert-butoxy-1-oxohexan-2-yl)ureido)pentanedioate (31 mg, 0.064 mmol) was dissolved in DMF (0.5 mL), and 2,5-dioxopyrrolidin-1-yl 1-azido-3,6,9,12-tetraoxapentadecan-15-oatel (25 mg, 0.064 mmol) was added thereto. The mixture was stirred at room temperature for 12 hours, and then the solution was purified by reverse phase HPLC under the following conditions. An obtained amount, NMR spectrum, and MS were as follows.

Purification conditions: Cosmosil 5C₁₈-AR-II column (10 × 250 mm), mobile phase: MeCN/H₂O/TFA [30/70/0.1 (0 min) to 90/10/0.1 (30 min)], flow rate: 4 mL/min.

Obtained amount: 35 mg (72%).

¹H-NMR(400MHz, CDCl₃)δ4.26-4.23(m, 2H), 3.75(t, J=5.2Hz, 2H), 3.69-3.61(m, 14H), 3.41(t, J=5.2Hz, 2H), 3.38-3.16(m, 2H), 2.59(t, J=5.2Hz, 2H), 2.34(dt, J=2.3, 7.5Hz, 2H), 2.11-1.60(m, 4H), 1.54(t, J=7.0Hz, 2H), 1.48-1.40(m, 27H), 1.39-1.26(m, 2H). ¹³C-NMR(100MHz, CDCl₃)δ174.3(2C), 172.8(2C), 158.4, 82.7, 82.4, 81.2, 70.4(2C), 70.3, 70.1, 70.0(2C), 69.9, 66.8, 53.7, 53.3, 50.5, 39.2, 35.8, 31.5, 31.3, 28.2, 27.9(3C), 27.8(7C), 21.9.

HRMS(ESI): m/z761.4656[M+H]⁺.

### <Synthesis of compound 7B>

Compound 7A (23 mg, 0.030 mmol) was dissolved in TFA (950 µL) and triisopropylsilane (50 µL), and the solution was stirred at room temperature for four hours. The solvent was removed, and then the residue was purified by reverse phase HPLC under the following conditions. An obtained amount, NMR spectrum, and MS were as follows.

Purification conditions: Cosmosil 5C₁₈-AR-II column (10 × 250 mm), mobile phase: MeCN/H₂O/TFA [10/90/0.1 (0 min) to 40/60/0.1 (30 min)], flow rate: 4 mL/min.

Obtained amount: 11 mg (63%).

¹H-NMR(400MHz, CDCl₃)δ7.52(s, 1H), 6.37(s, 2H), 4.39(s, 1H), 4.31(s, 1H), 3.66(m, 16H), 3.40(s, 2H), 2.53-1.23(m, 14H).

HRMS(ESI): 593.2779[M+H]⁺.

### <Synthesis of compound 7C (PtDA)>

Compound 77 (20 mg, 0.013 mmol) was dissolved in DMF (0.6 mL), and compound 7A (35 mg, 0.046 mmol) was added thereto. The mixture was stirred at room temperature for 12 hours, and then the solvent was removed. TFA (1.9 mL), thioanisole (60 µL), triisopropylsilane (20 µL), and H₂O (20 µL) were added to the residue, and the mixture was stirred at room temperature for 10 hours. The solvent was removed, and then the residue was purified by reverse phase HPLC under the following conditions. An obtained amount and MS were as follows.

Purification conditions: Cosmosil 5C₁₈-AR-II column (4.6 × 150 mm), mobile phase: MeCN/H₂O/TFA [10/90/0.1 (0 min) to 90/10/0.1 (40 min)], flow rate: 1 mL/min.

Obtained amount: 1.0 mg (4.2%).

HRMS(ESI): m/z900.3488[M+2H]²⁺.

### <Synthesis of [¹¹¹In]In-ADA by route A>

An [¹¹¹In]InCl₃ solution (9.2 MBq, 100 µL) and a dimethyl sulfoxide (DMSO) solution of compound 78 (0.60 mM, 7 µL) were added to a 2-(N-morpholino)ethanesulfonic acid (MES) buffer (0.1 M, pH 5.7, 100 µL), and the mixture was allowed to stand at 90°C for five minutes. Thereafter, the reaction solution was purified by reverse phase HPLC under the following conditions.

Purification conditions: Cosmosil 5C₁₈-AR-II column (4.6 × 150 mm), mobile phase: MeCN/H₂O/TFA [10/90/0.1 (0 min) to 70/30/0.1 (30 min)], flow rate: 1 mL/min.

A radiochemical conversion ratio (ratio of radioactivity of [¹¹¹In]In-ADA to radioactivity of [¹¹¹In]InCl₃) and a radiochemical yield are illustrated in Table 11 below.

### <Synthesis of [¹¹¹In]In-PtDA by route A>

[¹¹¹In]In-ADA (0.80 MBq) was added to a phosphate buffered saline (PBS)/DMSO mixed liquid (9/1, 200 µL) or DMSO (200 µL), and compound 7B (0.2 mg) was further added thereto. The mixture was allowed to stand at 37°C for 10 or 30 minutes. Thereafter, the reaction solution was purified by reverse phase HPLC under the following conditions.

Purification conditions: Cosmosil 5C₁₈-AR-II column (4.6 × 150 mm), mobile phase: MeCN/H₂O/TFA [10/90/0.1 (0 min) to 70/30/0.1 (30 min)], flow rate: 1 mL/min.

A radiochemical conversion ratio (ratio of radioactivity of [¹¹¹In]In-PtDA to radioactivity of [¹¹¹In]In-ADA) and a radiochemical yield are illustrated in Table 11 below.

### <Synthesis of [¹¹¹In]In-PtDA by route B>

An [¹¹¹In]InCl₃ solution (2.1 MBq, 100 µL) and a DMSO solution (0.56 mM, 2 µL) of compound 7C were added to a MES buffer (0.1 M, pH 5.7, 150 µL), and the mixture was allowed to stand at 90°C for five minutes. Thereafter, the reaction solution was purified by reverse phase HPLC under the following conditions.

Purification conditions: Cosmosil 5C₁₈-AR-II column (4.6 × 150 mm), mobile phase: MeCN/H₂O/TFA [10/90/0.1 (0 min) to 70/30/0.1 (30 min)], flow rate: 1 mL/min.

A radiochemical conversion ratio (ratio of radioactivity of [¹¹¹In]In-PtDA to radioactivity of [¹¹¹In]InCl₃) and a radiochemical yield are illustrated in Table 11 below.

**[Table 11]**

| | Compound | Solvent | Reaction conditions | Radiochemical conversion ratio (%) | Radiochemical yield (%) |
|---|---|---|---|---|---|
| Route A | [¹¹¹In]In-ADA | MES buffer | 90°C, 5 min | >95 | 54.9 ± 9.3 |
| | [¹¹¹In]In-PtDA | PBS | 37°C, 10 min | >95 | 55.3 ± 1.6 |
| | | DMSO | 37°C, 30 min | >95 | 66.7 ± 7.2 |
| Route B | [¹¹¹In]In-PtDA | MES buffer | 90°C, 5 min | >95 | 47.4 ± 14.7 |

Note that In-ADA and In-PtDA to which non-radioactive In is coordinated can be manufactured by the following method.

### <Synthesis of non-radioactive In-ADA>

Compound 78 (1 equivalent) was dissolved in an acetate buffer (1.0 M, pH 5.0, 100 µL), and indium (III) chloride nonhydrate (10 equivalent) was added thereto. The reaction liquid was stirred at 90°C for five minutes, and then the solution was purified by reverse phase HPLC.

Purification conditions: Cosmosil 5C₁₈-AR-II column (4.6 × 150 mm), mobile phase: MeCN/H₂O/TFA [10/90/30.1 (0 min) to 90/10/0.1 (40 min)], flow rate: 1 mL/min.

MS(ESI):m/z1319.3[M+H]⁺.

### <Synthesis of non-radioactive In-PtDA>

To a H₂O/MeCN/TFA (49.95/49.95/0.1, 300 µL) solution of compound 7C (0.5 mg, 1.25 mmol), indium (III) chloride nonhydrate (0.62 mg, 2.8 µmol) was added. The mixture was stirred at room temperature for 18 hours, and then the solution was purified by reverse phase HPLC.

Purification conditions: Cosmosil 5C₁₈-AR-II column (4.6 × 150 mm), mobile phase: MeCN/H₂O/TFA [10/90/0.1 (0 min) to 90/10/0.1 (40 min)], flow rate: 1 mL/min.

Obtained amount: 0.05 mg (9.4%).

HRMS(ESI): m/z956.2893[M+2H]²⁺.

### <Evaluation of distribution coefficient

[¹¹¹In]In-PtDA (111 kBq) was added to a mixed liquid of PBS (pH 7.4, 3 mL) and 1-octanol (3 mL). The mixture was dispersed by vortexing for two minutes, and then centrifuged at 4000 × g for five minutes. 1 mL of the 1-octanol layer and 1 mL of the PBS layer were collected, and the radioactivity of each of the layers was measured with a gamma counter (n = 3).

As a calculation formula, "(distribution coefficient) = Log₁₀ [(radioactivity of 1-octanol layer [kBq])/(radioactivity of PBS layer [kBq])]" was used.

As a result, LogP of [¹¹¹In]In-PtDA was "-3.08 ± 0.02".

### <Evaluation of stability in plasma>

A saline (20 µL) solution of [¹¹¹In]In-PtDA (259 kBq) was added to mouse plasma (200 µL), and the mixture was allowed to stand at 37°C for 24 hours (n = 3). MeCN (400 µL) was added thereto, and the mixture was centrifuged at 10,000 × g for five minutes. The supernatant was filtered, and the filtrate was analyzed by reverse phase HPLC under the following conditions.

Analysis conditions: Cosmosil 5C₁₈-AR-II column (4.6 × 150 mm), mobile phase: MeCN/H₂O/TFA [10/90/0.1 (0 min) to 70/30/0.1 (30 min)], flow rate: 1 mL/min.

As a result, as illustrated in Fig. 13, 95% or more of [¹¹¹In]In-PtDA was stably present in mouse plasma even after being allowed at 37°C for 24 hours.

### <Evaluation of binding using cultured cells>

In a similar manner to Example 5-1 described above, LNCaP cells and PC-3 cells were cultured, and a cell binding assay was performed. Evaluation was performed by a similar experimental procedure and statistical method to Example 5-1 except that a 0.5% FBS-containing RPMI 1640 medium (1 mL) containing [¹¹¹In]In-PtDA (37 kBq) was used as an assay medium.

Results are illustrated in Fig. 14.

[¹¹¹In]In-PtDA exhibited a higher binding property to the LNCaP cells (15%ID/mg protein) than the PC-3 cells (0.15%ID/mg protein), and the binding was significantly reduced by addition of an excess amount of PSMA inhibitor (2-PMPA) (0.36%ID/mg protein). These results indicate that [¹¹¹In]In-PtDA specifically binds to PSMA-positive cells.

### <Evaluation of binding to albumin>

In a similar manner to Example 5-1 described above, PBS, mouse plasma, human plasma, and human albumin were used, and evaluation of binding thereof to albumin was performed. Evaluation was performed by a similar experimental procedure and statistical method to Example 5-1 except that a PBS solution of [¹¹¹In]In-PtDA (37 kBq, 50 µL) was used.

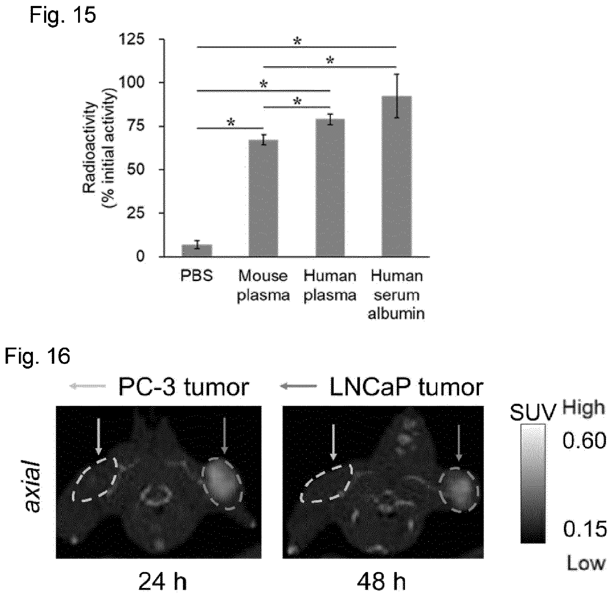

Results are illustrated in Fig. 15.

When [¹¹¹In]In-PtDA was allowed to stand in PBS and then applied to the column, radioactivity was not much detected in the eluate (6.9%). Meanwhile, when [¹¹¹In]In-PtDA was allowed to stand in a mouse plasma solution, a human plasma solution, and a human albumin solution, high radioactivity was observed in the eluates (67.2, 79.0 and 92.4% respectively), indicating that [¹¹¹In]In-PtDA binds to plasma albumin.

### <Evaluation of in vivo radioactivity distribution using LNCaP tumor transplanted mice>

A saline solution (241 kBq/100 µL) of [¹¹¹In]In-PtDA was injected into each of LNCaP tumor transplanted mice generated by a similar method to Example 5-1 via the tail vein thereof (n = 3), and the mice were euthanized 1, 24, and 48 hours after the injection. Thereafter, blood and organs were collected, and the mass and radioactivity of each of the organs were measured.

A result thereof is indicated as a value (%ID/g) obtained by dividing the percentage (%ID) of the amount of radioactivity with respect to the amount of injected radioactivity (injected dose) by the mass of blood or the mass of an organ (g). The higher a value of %ID/g, the higher the abundance of the radioactive labeled compound, indicating that the amount of the compound accumulated in the target organ is large.

Results (mean ± standard deviation, n = 3 in each group) in the LNCaP tumor transplanted mice and [¹¹¹In]In-PtDA are illustrated in Table 12 below.

[¹¹¹In]In-PtDA exhibited high accumulation in the LNCaP tumor (16.0 and 18.7%ID/g 24 and 48 hours after the injection, respectively) and exhibited a retention property in blood (6.33 to 19.8%ID/g 1 to 48 hours after the injection). In addition, accumulation in the kidney one hour after the injection was 37.2%ID/g, which is significantly lower than [¹¹¹In]In-PSMA-I&T (kidney: 191%ID/g) and [⁶⁸Ga]Ga-PSMA-11 (kidney: 139%ID/g) (for example, EJNMMI Res, 2012, 2, 23, JNucl Med, 2017, 58, 235-242), which are known radioactive labeled compounds targeting PSMA as a target molecule.

**[Table 12]**

| | 1 hour | 24 hours | 48 hours |
|---|---|---|---|
| Blood | 19.8±2.74 | 12.5±1.47 | 6.33±1.53 |
| Spleen | 15.3±4.89 | 9.69±0.92 | 10.5±5.62 |
| Pancreas | 2.31±0.14 | 2,03±0.48 | 1.12±0.39 |
| Stomach [%ID] | 0.79±0.19 | 0.46±0.23 | 0.29±0.20 |
| Intestinal | 2.66±0.28 | 1.52±0.41 | 0.74±0.28 |
| Kidney | 37.2±6.85 | 68.0±2.82 | 55.9±6.39 |
| Liver | 3.51±0.38 | 3.36±0.77 | 2.28±0.75 |
| Heart | 5.49±0.77 | 3.95±0.87 | 1.98±0.41 |
| Lung | 11.9±1.57 | 8.63±1.31 | 6.16±1.56 |
| Brain | 0.36±0.05 | 0.30±0.05 | 0.20±0.07 |
| Muscle | 1.95±0.30 | 1.28±0.34 | 0.81±0.40 |
| LNCaP tumor | 2.18±0.17 | 16.0±9.49 | 18.7±5.21 |

### <SPECT/CT evaluation using tumor transplanted mice>

LNCaP cells (1.0 × 10⁷ cells/mouse) were suspended in a mixed liquid (1 : 1,150 µL) of PBS and Matrigel, and the suspension was subcutaneously transplanted to the right shoulder of each of male CB17/IcrJcl-Prkdc^{scid} mice raised by a similar method to Example 5-1 under isoflurane anesthesia. In addition, a suspension of PC-3 cells (1.0 × 10⁷ cells/mouse) in a mixed liquid of PBS and Matrigel (1 : 1,150 µL) was subcutaneously transplanted to the left shoulder of each of the same mice under isoflurane anesthesia. Thereafter, the mice were raised for 40 to 60 days. In this way, tumor transplanted mice to each of which the LNCaP cells and the PC-3 cells were simultaneously transplanted were obtained.

Subsequently, a saline solution (2.7 MBq, 100 µL) of [¹¹¹In]In-PtDA was injected into each of the tumor transplanted mice via the tail vein thereof. SPECT/CT was performed with an FX3300 pre-clinical imaging system manufactured by Gamma Medica-Ideas Inc. 24 and 48 hours after the injection. Imaging was performed under isoflurane anesthesia using a pinhole collimator with a diameter of 1.0 mm at a rotation radius of 35 mm, a projection time of 70 seconds, and the number of times of projection of 32. After SPECT, CT (tube voltage: 60 kV, tube current: 350 µA) was performed. Image reconstruction by a three-dimensional ordered subset expectation maximization method (8 subsets, 5 iterations) was performed on projection data of SPECT.

Results of SPECT/CT are illustrated in Fig. 16.

In SPECT/CT imaging using [¹¹¹In]In-PtDA, high radioactivity accumulation was observed in the LNCaP tumor, but a radioactivity signal was hardly observed from the PC-3 tumor 24 and 48 hours after the injection. This result indicates that [¹¹¹In]In-PtDA can clearly delineate the PSMA positive tumor.

### Example 8

In the present Example, a compound (RtDA) containing a cRGD peptide as a target molecule binding moiety in the structure thereof and using the click reaction between an azide group and dibenzylcyclooctyne (DBCO) for binding between a chelating moiety and the target molecule binding moiety was synthesized. Subsequently, a radioactive labeled compound in which an ¹¹¹In ion was coordinated as a radioactive metal to each of these compounds was obtained. An outline of a reaction path is illustrated below as reaction paths (IX-1) and (IX-2).

The compound used in Example 8 contains a chelating moiety, a target molecule binding moiety, and an albumin binding moiety in the structure thereof.

In addition, there are two reaction paths (see reaction path (IX-2)) for coordination to an ¹¹¹In ion, and the same radioactive labeled compound is obtained in either path. Note that the synthesis method of compound 77 and compound 78 (ADA) is similar to that in Example 7.

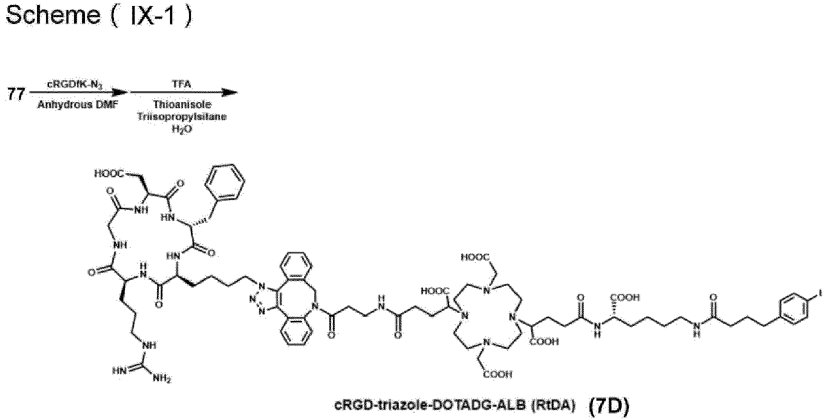

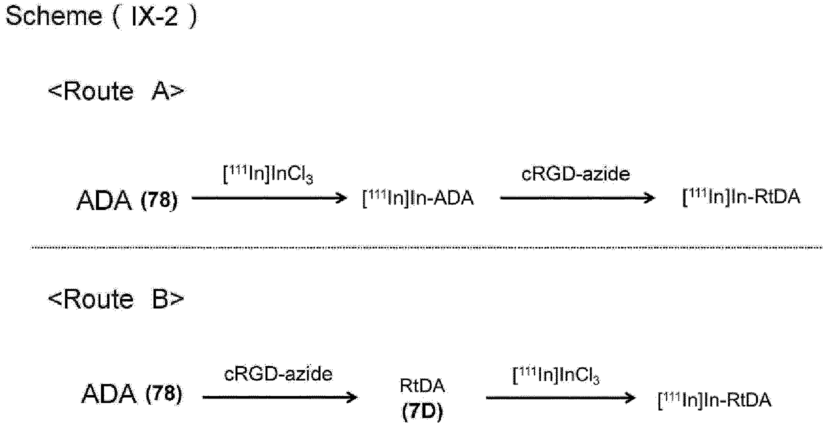

### <Synthesis of compound 7D (RtDA)>

Compound 77 (3.9 mg, 2.6 µmol) was dissolved in DMF (0.4 mL), and cyclo [Arg-Gly-Asp-D-Phe-Lys (azide)] (1.7 mg, 2.6 µmol) was added thereto. The mixture was stirred at room temperature for 12 hours, and then the solvent was removed. TFA (1.9 mL), thioanisole (60 µL), triisopropylsilane (20 µL), and H₂O (20 µL) were added to the residue, and the mixture was stirred at room temperature for 10 hours. The solvent was removed, and then the residue was purified by reverse phase HPLC under the following conditions. An obtained amount and MS were as follows.

Purification conditions: Cosmosil 5C₁₈-AR-II column (4.6 × 150 mm), mobile phase: MeCN/H₂O/TFA [10/90/0.1 (0 min) to 90/10/0.1 (40 min)], flow rate: 1 mL/min.

Obtained amount: 2.8 mg (58%).

HRMS(ESI)m/z918.8662[M+2H]²⁺.

### <Synthesis of [¹¹¹In]In-RtDA by route A>

[¹¹¹In]In-ADA (1.5 MBq) was added to a PBS/DMSO mixed liquid (9/1, 200 µL), and cyclo[Arg-Gly-Asp-D-Phe-Lys(azide)] (0.2 mg) was further added thereto. The mixture was allowed to stand at 37°C for 10 minutes. Thereafter, the reaction solution was purified by reverse phase HPLC under the following conditions.

Purification conditions: Cosmosil 5C₁₈-AR-II column (4.6 × 150 mm), mobile phase: MeCN/H₂O/TFA [10/90/0.1 (0 min) to 70/30/0.1 (30 min)], flow rate: 1 mL/min.

A radiochemical conversion ratio (ratio of radioactivity of [¹¹¹In]In-RtDA to radioactivity of [¹¹¹In]In-ADA) and a radiochemical yield are illustrated in Table 13 below.

### <Synthesis of [¹¹¹In]In-RtDA by route B>

An [¹¹¹In]InCl₃ solution (2.1 MBq, 80 µL) and a DMSO solution (0.27 mM, 2 µL) of compound 7D were added to a MES buffer (0.1 M, pH 5.7, 150 µL), and the mixture was allowed to stand at 90°C for five minutes. Thereafter, the reaction solution was purified by reverse phase HPLC.

Purification conditions: Cosmosil 5C₁₈-AR-II column (4.6 × 150 mm), mobile phase: MeCN/H₂O/TFA [10/90/0.1 (0 min) to 70/30/0.1 (30 min)], flow rate: 1 mL/min.

A radiochemical conversion ratio (ratio of radioactivity of [¹¹¹In]In-RtDA to radioactivity of [¹¹¹In]InCl₃) and a radiochemical yield are illustrated in Table 13 below.

**[Table 13]**

| | Compound | Solvent | Reaction conditions | Radiochemical conversion ratio (%) | Radiochemical yield (%) |
|---|---|---|---|---|---|
| Route A | [¹¹¹In]In-RtDA | PBS | 37°C, 10 min | >95 | 63.1 ± 20.3 |
| Route B | [¹¹¹In]In-RtDA | MES buffer | 90°C, 5 min | >95 | 52.9 ± 9.4 |

Note that In-RtDA to which non-radioactive In is coordinated can be manufactured by the following method.

### <Synthesis of non-radioactive In-RtDA>

To a H₂O/MeCN/TFA (49.95/49.95/0.1, 300 µL) solution of compound 7D (0.8 mg, 0.40 µmol), indium (III) chloride nonhydrate (1.0 mg, 4.0 µmol) was added. The mixture was stirred at room temperature for 18 hours, and then the solution was purified by reverse phase HPLC.

Purification conditions: Cosmosil 5C₁₈-AR-II column (4.6 × 150 mm), mobile phase: MeCN/H₂O/TFA [10/90/0.1 (0 min) to 90/10/0.1 (40 min)], flow rate: 1 mL/min.

Obtained amount: 0.06 mg (7.7%).

HRMS(ESI): m/z974.7980[M+2H]²⁺.

### Industrial Applicability

The present invention can achieve both improvement of accumulation in a target tissue and reduction of accumulation in a non-target tissue, particularly reduction of accumulation in the kidney.

## Claims

1. A compound represented by the following formula (1):
C-A-B (1)
wherein, A represents a chelating moiety capable of being coordinated to a radioactive metal ion, B represents an atomic group containing an albumin binding moiety, and C represents an atomic group containing a target molecule binding moiety,
B binds to a site of A, and
C binds to A at a site different from the site where B binds to A.

2. The compound according to claim 1, wherein
in the formula (1), A has a cyclic structure, the cyclic structure has two or more nitrogen atoms, and the nitrogen atoms are connected to each other across two or more adjacent carbon atoms, or A has a chain structure, the chain structure has two or more nitrogen atoms, and the nitrogen atoms are connected to each other across two or more adjacent carbon atoms,
A has a nitrogen binding atomic group directly binding to any one of the nitrogen atoms constituting the cyclic structure or the chain structure,
the nitrogen binding atomic group contains one or more of a carboxy group, a phosphate group, an amide group, a benzene ring, and a pyridine ring, and
when B binds to the nitrogen binding atomic group, C binds to the nitrogen binding atomic group other than the atomic group to which B binds.

3. The compound according to claim 1 or 2, wherein in the formula (1), A has a structure derived from a compound represented by the following formula (A1): 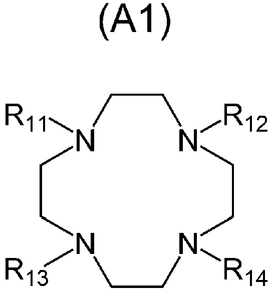 wherein, R₁₁, R₁₂, R₁₃, and R₁₄ each independently represent any one of groups consisting of -(CH₂)ₚCOOH, -(CH₂)ₚC₅H₅N, -(CH₂)ₚPO₃H₂, -(CH₂)ₚCONH₂, - (CHCOOH)(CH₂)ₚCOOH, and p represents an integer of 0 or more and 3 or less.

4. The compound according to any one of claims 1 to 3, wherein
in the formula (1), the albumin binding moiety has a structure derived from one or more of γ-glutamic acid, a phenylbutyric acid with or without a substituent, lipid, hematin, bilirubin, clofibric acid, clofibrate, carotenoid, a compound having a steroid skeleton, a compound having an ibuprofen skeleton, a linear or branched and saturated or unsaturated hydrocarbon having 13 or more and 20 or less carbon atoms, a cyanine dye, a dye having a sulfonate group, a diazo dye, a pentamethine cyanine dye, blue dextran, bromocresol green, Evans blue, and derivatives thereof or is an antibody or a peptide capable of binding to albumin.

5. The compound according to any one of claims 1 to 4, wherein in the formula (1), the albumin binding moiety has a structure represented by the following formula (B1) or (B2): 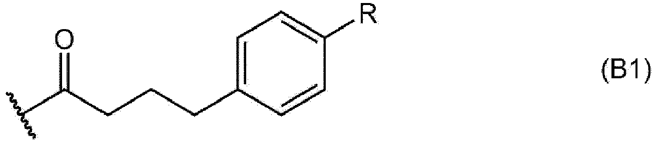 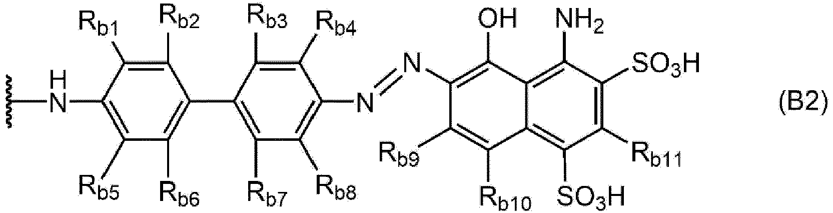
in the formula (B1), R represents a hydrogen atom, a halogen atom, or an alkyl group having 1 or more and 5 or less carbon atoms, and a portion indicated by a wavy line is a binding moiety to another structure, and
in the formula (B2), R_{b1} to R_{b11} each independently represent a hydrogen atom, a halogen atom, a hydroxy group, a cyano group, an alkyl group having 1 or more and 6 or less carbon atoms with or without a substituent, or an alkoxy group having 1 or more and 6 or less carbon atoms with or without a substituent, and a portion indicated by a wavy line is a binding moiety to another structure.

6. The compound according to any one of claims 1 to 5, wherein in the formula (1), the target molecule binding moiety has a structure to bind to a target molecule expressed in a cancer tissue.

7. The compound according to any one of claims 1 to 6, represented by the following formula (2): 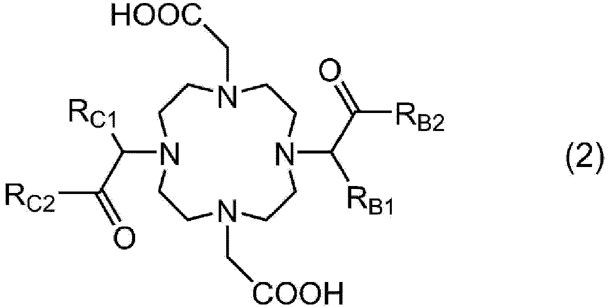
wherein, R_{B1} represents an atomic group containing an albumin binding moiety, Rci represents an atomic group containing a target molecule binding moiety, and R_{B2} and R_{C2} both represent a hydroxy group, or
R_{B2} represents an atomic group containing an albumin binding moiety, R_{C2} represents an atomic group containing a target molecule binding moiety, and R_{B1} and Rci both represent a hydrogen atom or each independently represent a carboxyalkyl group having 1 to 5 carbon atoms.

8. The compound according to any one of claims 1 to 7, which is used as a labeling precursor.

9. A radioactive labeled compound in which the compound according to any one of claims 1 to 8 is coordinated to an ion of a radioactive metal.

10. The radioactive labeled compound according to claim 9, wherein the radioactive metal is ⁴⁴Sc, ⁵¹Cr, ⁵⁷Co, ⁵⁸Co, ⁶⁰Co, ⁵⁹Fe, ⁶⁷Ga, ⁶⁸Ga, ⁶⁴Cu, ⁶⁷Cu, ⁸⁹Sr, ⁸⁹Zr, ⁹⁰Y, ^{99m}Tc, ¹⁰³Ru, ¹¹¹In, ¹⁵³Sm, ¹⁶⁵Dy, ¹⁶⁶Ho, ¹⁷⁷Lu, ¹⁸⁶Re, ¹⁸⁸Re, ¹⁹⁸Au, ²⁰¹Tl, ¹⁹⁷Hg, ²⁰³Hg, ²¹²Bi, ²¹³Bi, ²¹²Pb, ²²⁷Th, or ²²⁵Ac.

11. The radioactive labeled compound according to claim 9 or 10, wherein the radioactive metal is an α ray-emitting nuclide.

12. The radioactive labeled compound according to claim 9 or 10, wherein the radioactive metal is a β ray-emitting nuclide.
